(19) Europäisches Patentamt
European Patent Office
Office européen des brevets

(11) EP 1 397 500 B1

(12) **EUROPEAN PATENT SPECIFICATION**

(45) Date of publication and mention of the grant of the patent:
**17.09.2014 Bulletin 2014/38**

(21) Application number: **02748810.5**

(22) Date of filing: **21.06.2002**

(51) Int Cl.:
***C12N 15/87*** *(2006.01)*

(86) International application number:
**PCT/EP2002/006897**

(87) International publication number:
**WO 2003/000907 (03.01.2003 Gazette 2003/01)**

(54) **IMPROVED TRANSFECTION OF EUKARYOTIC CELLS WITH LINEAR POLYNUCLEOTIDES BY ELECTROPORATION**

VERBESSERTE TRANSFEKTION EUKARYOTISCHER ZELLEN MIT LINEAREN POLYNUKLEOTIDEN MITTELS ELEKTROPORATION

TRANSFECTION AMELIOREE DE CELLULES EUCARYOTES AVEC POLYNUCLEOTIDES LINEAIRES PAR ELECTROPORATION

(84) Designated Contracting States:
**AT BE CH CY DE DK ES FI FR GB GR IE IT LI LU MC NL PT SE TR**

(30) Priority: **21.06.2001 EP 01115076**

(43) Date of publication of application:
**17.03.2004 Bulletin 2004/12**

(60) Divisional application:
**10185221.8 / 2 308 987**

(73) Proprietors:
- **Schuler, Gerold**
  **91080 Spardorf (DE)**
- **Universiteit Antwerpen**
  **2000 Antwerpen (BE)**

(72) Inventors:
- **SCHULER, Gerold**
  **91080 Spardorf (DE)**
- **BERNEMAN, Zwi N.**
  **2018 Antwerpen (BE)**
- **VAN TENDELOO, Viggo F.I.**
  **2500 Lier (BE)**
- **PONSAERTS, Peter**
  **2100 Deume (BE)**
- **STROBEL, Isolde**
  **91052 Erlangen (DE)**

(74) Representative: **Von Kreisler Selting Werner - Partnerschaft**
**von Patentanwälten und Rechtsanwälten mbB**
**Deichmannhaus am Dom**
**Bahnhofsvorplatz 1**
**50667 Köln (DE)**

(56) References cited:
**EP-A- 0 414 551 US-A- 5 554 528**
**US-A- 5 766 902**

- **VAN TENDELOO VIGGO F I ET AL: "Highly efficient gene delivery by mRNA electroporation in human hematopoietic cells: Superiority to lipofection and passive pulsing of mRNA and to electroporation of plasmid cDNA for tumor antigen loading of dendritic cells." BLOOD, vol. 98, no. 1, 1 July 2001 (2001-07-01), pages 49-56, XP001037914 ISSN: 0006-4971**
- **STROBEL I ET AL: "Human dendritic cells transfected with either RNA or DNA encoding influenza matrix protein M1 differ in their ability to stimulate cytotoxic T lymphocytes." GENE THERAPY, vol. 7, no. 23, December 2000 (2000-12), pages 2028-2035, XP001034189 ISSN: 0969-7128 cited in the application**
- **NAIR SMITA K ET AL: "Induction of primary carcinoembryonic antigen (CEA)-specific cytotoxic T lymphocytes in vitro using human dendritic cells transfected with RNA." NATURE BIOTECHNOLOGY, vol. 16, no. 4, April 1998 (1998-04), pages 364-369, XP001026122 ISSN: 1087-0156 cited in the application**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **ZHANG WEIPING ET AL: "Enhanced therapeutic efficacy of tumor RNA-pulsed dendritic cells after genetic modification with lymphotactin." HUMAN GENE THERAPY, vol. 10, no. 7, 1 May 1999 (1999-05-01), pages 1151-1161, XP001037915 ISSN: 1043-0342 cited in the application**
- **MORSE MICHAEL A ET AL: "Optimization of the sequence of antigen loading and CD40-ligand-induced maturation of dendritic cells." CANCER RESEARCH, vol. 58, no. 14, 15 July 1998 (1998-07-15), pages 2965-2968, XP001037215 ISSN: 0008-5472 cited in the application**
- **CELLA MARINA ET AL: "Origin, maturation and antigen presenting function of dendritic cells." CURRENT OPINION IN IMMUNOLOGY, vol. 9, no. 1, 1997, pages 10-16, XP002166451 ISSN: 0952-7915**


Remarks:

The file contains technical information submitted after the application was filed and not included in this specification

## Description

The present invention provides an improved method for gene delivery in hematopoietic cells and stem cells not including human embryonic stem cells by electroporation, preferably in human hematopoietic cells, in particular dendritic cells. The method of the invention is superior to lipofection and passive pulsing of mRNA and to electroporation of plasmid cDNA for gene delivery, including tumor antigen loading of dendritic cells.

## Background of the Invention

Dendritic cells (DC) are bone-marrow-derived leukocytes that function as professional antigen-capturing and -presenting cells for the initiation of a primary immune response *in vitro* and *in vivo* (Banchereau, J., Steinman, R.M., Nature, 392:245-252 (1998)). Given their central role in cell-mediated immunity *in vivo,* they represent highly attractive targets for molecular immunotherapy of acquired diseases, such as AIDS and cancer. Recent advances in the *ex vivo* generation of DC and the ability to modulate DC functions provide a rationale to design DC-based tumor vaccines (Avigan, D., Blood Rev., 13:51-64 (1999)). The outcome of such tumor vaccines will highly depend on the efficacy of the applied antigen-loading method for optimal stimulation of cytotoxic T lymphocytes (CTL)-mediated anti-tumor immune responses (Tarte, K., Klein, B., Leukemia, 13:653-663 (1999)). Although several reports have documented viral transfer of cDNA encoding tumor-associated antigens to load DC for induction of TAA-specific cytotoxic T lymphocytes (CTL) (Dietz, A.B., Vuk, P.S., Blood, 91:392-398 (1998); Brossart, P. et al., J. Immunol., 158:3270-3276 (1997); Specht, J.M. et al., J. Exp. Med., 186:1213-1221 (1997)) nonviral gene delivery systems for DC-based vaccines would provide a more attractive approach with clinical perspectives since safety issues and immunogenicity of the vector are reduced to a minimum. Furthermore, it is generally known that nonviral DNA transfection methods are inefficient, particularly in nondividing cells, as there is only very limited DNA trafficking to the nucleus where transcription occurs (Luo, D., Saltzman, W.M., Nat. Biotechnol., 18:33-37 (2000)). Therefore, several groups demonstrated the feasibility of mRNA transfection as a valid alternative for nonviral gene delivery, since this strategy avoids the need for entry into the nucleus as well as the complex issues of transcriptional regulation associated with DNA vectors (Lu, D. et al., Cancer Gene Ther., 1:245-252 (1994); Kariko, K. et al., Biochim. Biophys. Acta, 1369:320-334 (1998); Sawai, K. et al., Mol. Genet. Metab., 64:44-51 (1998)). The RNA approach has several advantages that render it attractive in developing DC-based tumor vaccines. First, DC can be transfected to comparable levels as compared to transduction by recombinant viruses, such as poxviruses Kim, C. J. et al., J. Immunother., 20:276-286 (1997)) or adenoviruses (Dietz, A. B., Vuk, P.S., Blood, 91:392-398 (1998)), while circumventing the drawbacks of viral vectors (Jenne, L. et al., Gene Ther., 7:1575-1583 (2000); Jonuleit, H. et al., Gene Ther., 7:249-254 (2000)). Second, DC can be charged with the full antigenic spectrum using total mRNA instead of IVT mRNA as a source of tumor antigens without prior identification of tumor-associated antigens (Zhang, W. et al., Hum. Gene Ther., 10:1151-1161 (1999)). Moreover, RNA has a short cellular half-life and lacks the potential to integrate into the host genome, thereby obviating safety concerns, e.g. insertional mutagenesis, in the context of clinical gene therapy trials (Lu, D. et al., Cancer Gene Ther., 1:245-252 (1994); Ying, H. et al., Nat. med., 5:823-827 (1999)). On the other hand this short cellular half-life may be disadvantageous since it may result in a relatively short protein expression.

However, the problem of inefficient gene transfer and low level of expression by nonviral transfection remains (Arthur, J.F. et al., Cancer Gene Ther., 4:17-25 (1997).

Previously, we reported high-level transgene expression in proliferating $CD34^+$ progenitor-derived DC (34-DC) and Langerhans cells (34-LC) using electroporation-mediated gene delivery (Van Tendeloo, V.F.I, et al., Gene Ther., 5:700-707 (1998)). In contrast, nondividing monocyte-derived DC (Mo-DC), which represent a highly accessible and widely used source of *in vitro* cultured DC, were relatively refractory to cDNA transfection techniques, either by electroporation or by lipofection.

Recently, it was shown that human DC could be transfected with RNA and were capable of inducing primary antigen-specific CTL (Nair, S. K. et al., Nat. Biotechnol., 16:364-369 (1998)). However, there are very few data on efficiency of mRNA transfer in Mo-DC using passive pulsing, lipofection or electroporation, if at all. Furthermore, the feasibility of mRNA transfection in 34-DC or 34-LC has not yet been established, let alone that the method has been adapted to Mo-DC. Electroporation methods for the integration of cyclic polynucleic acids into "normal" cells (such as tumor cells) are generally use the following reaction conditions (Van Tendeloo V.F.I et al., Gene Ther. 5:700-707 (1998); Van Tendeloo, V.F.I, et al., Gene Ther. 7:1431-1437 (2000); Van Bockstaele, D., Berneman, Z.N., Cytometry 41:31-35 (2000); Lurquin, P.F., Mol. Biotechnol. 7:5-35 (1997); Matthews, K.E. et al., Mol. Biotechnol., vol 48, Chapter 22, Ed.Nickoloff); Spencer, S.C., Biochem. and Biotechnol. 42:75-82 (1993)):

- a cell concentration in the range of 1 to $10x10^6$ cell/ml
- a voltage in the range of 200-350 V
- a capacitance of greater than 300 $\mu$F

- a pulse length in the range of 15 to 40 μs

However such "conventional" electroporation methods give only very poor RNA transfection yields if applied to primary cells (such as Mo-DC) and/or if linear polynucleotides are electroporated as it was e.g. shown in Strobel, I. et al., Gene Therapy 7:2028-2035 (2000) where we reported on the electroporation of monocyte-derived dendritic cells and tested various "conventional" parameter settings: i) the cell density was tested in the range from $2x10^6$ - $4x10^7$ cells/ml while capacitance and voltage were kept constant at 300 μF and 250V, respectively, showing that an increased cell density resulted in a decreased mortality; ii) the impact of the voltage was investigated in the range from 250-350 V, while capacitance and cell density were kept constant at 300 μF and $4x10^7$cells/ml, respectively, demonstrating that an increasing voltage resulted in a higher mortality; iii) the capacitance was evaluated in the range from 300-1500 μF while cell density and voltage were kept constant at $4x10^7$ cells/ml and 250 V, respectively. Increasing capacitance yielded in an increased mortality. Although pulse times below 22 ms increased the cell viability, only a very low heterologous gene expression was detectable. In contrast pulse times above 28 ms increased transient gene expression but resulted in a high cell loss. In conclusion, the optimal electroporation conditions for immature monocyte-derived DC were found in this previously published work as follows: i.) cell density of $4x10^7$ cells/ml; ii.) voltage of 250 V; iii.) capacitance of 300 - 500 μF and iv.) pulse times between 22-28 ms. Using these optimised electroporation conditions up to 11 % of DC were $GFP^+$ after 48 h, when GFP RNA was transfected. A similar transfection efficacy was obtained using GFP DNA.

Recently we also reported that effective electroporation of *in vitro* transcribed mRNA into monocyte-derived dendritic cells is possible, but did not mention how this electroporation can be achieved (Poster at the 6th Symposium on dendritic cells, Port Douglas, Australia, May 26-June 1, 2000 and at the Keystone Symposia, Taos, NM, USA., March 12-18, 2001).

US patent no. 5,766,902 discloses an electroporation method for nucleic acid molecules, wherein the nucleic acid molecules are applied in or together with a ligand which binds to the target cell. Said complex may comprise an endosomal disruption agent.

US patent No. 5,554,528 decribes the use of plasmids (i.e., cyclic DNA constructs) containing a toxin gene under the control of HIV elements for stable transformation of cell lines in order to block HIV replication when cells are infected. Said patent mentions DNA transfection by electroporation (column 15, example 2) using electrical settings (250 μF; 220 to 290 V; 100 μl volumes, BioRad cuvettes and Gene Pulser®) which are not typical for plasmid electroporation, it does, however, not mention RNA transfection, let alone RNA electropration. Furthermore, only "normal" mammalian cell lines are electroporated, primary cells are not contemplated.

**Summary of the invention**

It was now surprisingly found that with a particular electroporation setting hematopoietic cells and stem cells not including human embryonic stem cells, such as monocyte derived dendritic cells can effectively be transfected with naked linear RNA. In particular, it was found out that the mRNA transfection efficiency was improved using an optimized mRNA-based electroporation. Thus, the present invention describes a method for high-efficiency non-viral transfection of Mo-DC as well as other types of dendritic cells (including $CD34^+$ derived Langerhans cells and interstitial type DC) by mRNA electroporation correlated with effective loading of tumor antigens into different types of human DC. The efficiency of the method of the present invention was compared with other transfection methods, such as lipofection and passive pulsing of mRNA as well as cDNA electroporation, and found to be highly superior. Furthermore, the effect of DC maturation on loading efficiency was investigated. An electroporation-based mRNA transfection protocol was developed which is suitable for highly efficient antigen loading in Mo-DC, as well as in 34-DC and 34-LC. This technique proved to be superior to mRNA lipofection or passive mRNA pulsing in terms of loading efficiency and subsequent activation of an antigen-specific $CD8^+$ CTL clone. With such mRNA-based electroporation, the transfection efficiency in Mo-DC, 34-DC and 34-LC was at least 25, 6 and 3 times, respectively, more efficient as compared to plasmid DNA electroporation described in van Tendeloo, V.F.I, et al., Gene Ther., 5:700-707 (1998), and also superior to previously described mRNA electroporation. Also, such mRNA electroporation was superior to mRNA lipofection and passive pulsing. This increased transfection efficiency was translated in a superior biological effect *in vitro,* as confirmed by our CTL activation experiments, and could be used as a tool to investigate as to whether it results in a higher immunopotency *in vivo* (Porgador, A. et al., J. Exp. Med., 188:1075-1082 (1998)). Importantly, mRNA-transfected DC were able to efficiently process the introduced antigen and present antigenic epitopes in an MHC class I-restricted manner to a specific $CD8^+$ TIL clone (Fig. 4). Furthermore, in concordance with previous reports on the effect of maturation on the antigen-presenting capacity of DC (Cella, M. et al., Curr. Opin. Immunol., 9:10-16 (1997)), antigen loading by mRNA electroporation was preferably performed prior to DC maturation in order to achieve the most optimal antigen presentation (Fig. 5), indicating the importance of the sequence of loading and DC maturation for future DC-based vaccine design (Morse, M. A. et al., Cancer Res., 58:2965-2968 (1998)).

The present invention thus provides a method for transfection of hematopoietic cells or stem cells not including human embryonic stem cells with one or more or a mixture of naked linear RNAs, which method comprises electroporation of a suspension containing the hematopoietic cells or stem cells and the naked linear RNAs to be transfected at a capacitance of 100 to 250 μF, at a voltage from 200 to 350 V and a pulsing time from 1 to 40 ms, wherein the concentration of the cells in the suspension is 100 to $1x10^9$ cells per ml.

The above method (hereinafter also referred to as "the method of the invention") is applicable for loading human dendritic cells (DC) with antigens such as tumor antigens, which is a challenging approach for DC-based tumor vaccines. (This is quite important, since in preliminary experiments it was found that the plasmid DNA electroporation approach is not applicable for tumor antigen loading of DC, because the T cell stimulation it provokes is indistinguishable from non-specific T cell stimulation mediated by plasmid DNA, either directly or indirectly. This nonspecific stimulation was not observed when using mRNA electroporation, establishing the superiority of that technique for specific DC-based T cell stimulation.) Also, the expression of other proteins (e.g., stimulatory or tolerogenic or apoptotic molecules) in DC by gene transfer might be desired, furthermore the introduction of antisense RNA by electroporation. In other words, the present invention describes a cytoplasmic expression system based on mRNA electroporation to efficiently introduce genetic information into DC. Preliminary experiments in K562 cells using an enhanced green fluorescent protein (EGFP) reporter gene revealed that mRNA electroporation as compared to plasmid DNA electroporation showed a markedly improved transfection efficiency (89% versus 40% $EGFP^+$ cells, respectively) and induced a strikingly lower cell toxicity (15% death rate with mRNA versus 51% with plasmid DNA). Applying mRNA electroporation for nonviral transfection of different types of human DC, including monocyte-derived DC (Mo-DC), $CD34^+$ progenitor-derived DC (34-DC) and Langerhans cells (34-LC), high-level transgene expression by mRNA electroporation was obtained in more than 50% of all DC types. mRNA-electroporated DC retained their phenotype and maturational potential. Importantly, DC electroporated with mRNA encoding Melan-A strongly activated a Melan-A-specific cytotoxic T lymphocyte (CTL) clone in an HLA-restricted manner and were superior to mRNA-lipofected or -pulsed DC. Optimal stimulation of the CTL occurred when Mo-DC underwent maturation following mRNA transfection. Strikingly, a nonspecific stimulation of CTL was observed when DC were transfected with plasmid DNA. Our data clearly demonstrate that Mo-DC electroporated with mRNA efficiently present functional antigenic peptides to cytotoxic T cells. Therefore, electroporation of mRNA encoding tumor antigens is a powerful technique to charge human dendritic cells with tumor antigens and could serve applications in future DC-based tumor vaccines. Transfection of ready mature DC was less efficient when maturation stimuli such as TNFα + LPS were used. The use of a certain generation methanol for Mo-DC including an optimized maturation stimulus (see Example 4) allowed, however, also for efficient transfection such as tumor antigens of mature Mo-DC. The invention is hereinafter described in more detail by the appended figures and the examples.

**Description of the Figures**

Figure 1 shows the flow cytometric analysis of transgene expression in K562 cells following EGFP mRNA electroporation.

A: K562 cells were electroporated with EGFP mRNA at 300 V, 150 μF or with EGFP plasmid DNA at 260V, 1050 μF (dashed line) as described in the Examples. Twenty-four hours post-electroporation, flow cytometric (FCM) EGFP analysis was performed to estimate transfection efficiency of mRNA electroporation (bold line) and plasmid DNA electroporation (dashed line). An overlay histogram representative of five independent experiments is shown. Non-electroporated cells (thin line) were used to determine background fluorescence. The M1 region indicates the EGFP-positive cell fraction. The percentage of $EGFP^+$ cells was 85% (bold line) and 50% (dashed line) following mRNA or plasmid DNA electroporation, respectively.
B: Kinetics of EGFP mRNA expression in K562 cells in function of time (n=3). Note the rapid induction of high-level EGFP expression already 3 hours following electroporation.

Figure 2 shows the FCM analysis of transgene expression following EGFP mRNA transfection in different types of DC.

A: Immature Mo-DC were cultured with GM-CSF and IL-4 and transfected at day 6 of culture with control (Melan-A) or EGFP mRNA by lipofection (bottom) or electroporation (top) and analyzed by FCM one day after transfection. The dot plots show EGFP fluorescence on the x-axis and ethidium bromide staining on the y-axis. Gates were drawn based on control mRNA-lipofected or electroporated Mo-DC. Percentage of dead cells (upper left corner) and viable $EGFP^+$ cells (lower right corner) is indicated. Results are representative of 8 independent experiments.
B: Monitoring of EGFP mRNA expression and cell viability in Mo-DC following mRNA electroporation in function of time (n=2).

C: 34-DC (bottom) and 34-LC (top) were cultured as described in Materials and Methods and transfected at day 12 and 25 of culture, respectively, with control (Melan-A) or EGFP mRNA by mRNA electroporation. FCM analysis was performed 24 h after mRNA electroporation. The dot plots show EGFP fluorescence on the x-axis and ethidium bromide staining on the y-axis. Gates were drawn based on control mRNA-electroporated Mo-DC (left). Percentage of dead cells (upper left corner) and viable $EGFP^+$ cells (lower right corner) is indicated. Results are representative of 4 independent experiments.

Figure 3 shows the phenotypical analysis and maturation potential of mRNA-electroporated DC.

A: Immature Mo-DC (iMo-CD) were transfected by electroporation with mRNA encoding EGFP and stained with phycoerythrin (PE)-labeled antibodies specific for CD1a, HLA-DR and CD86 one day after electroporation (bottom). Untransfected iMo-DC (top) served as controls and isotype-matched antibodies were used to set quadrants. Results are representative of 3 experiments.
B: iMo-DC were transfected by electroporation with mRNA encoding Melan-A and directly stained with a PE-labeled CD80 antibody (bottom) or indirectly stained with a CD83 antibody (top). A representative overlay histogram is shown in which the dashed line represents the control non-electroporated iMo-DC, the thin line the electroporated iMo-DC and the bold line represents electroporated iMo-DC that were allowed to mature for an additional 24 h following mRNA electroporation in the presence of TNF-$\alpha$ and LPS.
C: 12 day-cultured 34-DC were transfected by electroporation with mRNA encoding EGFP and stained with PE-labeled antibodies specific for CD1a, HLA-DR, CD86 and CD80 one day after electroporation (bottom). Untransfected 34-DC (top) served as controls and isotype-matched antibodies were used to set quadrants. Results are representative of 3 experiments.

Figure 4 shows the mRNA-based antigen loading of Mo-DC. Immature Mo-DC were cultured with GM-CSF and IL-4 and transfected at day 6 of culture with Melan-A mRNA by electroporation (n= 11), lipofection (n=8) or passive pulsing (n=5) or with EGFP mRNA by electroporation (n=6). The SK23-MEL melanoma cell line, $HLA-A2^+$ Mo-DC pulsed with a Melan-A or irrelevant influenza peptide and HLA-A2-negative Mo-DC electroporated with Melan-A mRNA served as controls. Antigen-presenting cells (indicated on the left of the graph) were coincubated with a Melan-A specific $CD8^+$ CTL clone to determine antigen loading eficiency, as reflected by IFN-$\gamma$ production of the CTL clone. Results are shown as mean $\pm$ SD. * P<0.05; EP= electroporation; lipo= lipofection; puls= passive pulsing.
Figure 5 shows the effect of DC maturation on tumor antigen presentation of mRNA-transfected Mo-DC. IFN-$\gamma$ production by the CTL clone was measured after coculture with $HLA-A2^+$ Mo-DC electroporated with Melan-A mRNA. iMo-DC, Mo-DC transfected at the immature stage and used as such; Mo-DCa, Mo-DC transfected at the mature stage after LPS+TNF-$\alpha$ stimulation; Mo-DCb, Mo-DC transfected at the immature stage, matured by LPS+TNF-$\alpha$ and then assayed for Melan-A-specific CTL clone stimulation. Results are shown as mean $\pm$ SD (n=4). * P<0.05
Figure 6 shows the outcome of plasmid cDNA-based antigen loading of 34-LC. IFN-$\gamma$ production by the CTL clone was measured after coculture with $HLA-A2^+$ 34-LC electroporated with various plasmid DNA constructs encoding Melan-A (pcDNA1.1/Melan-A; n= 12), EGFP (pEGFP-N1; n=12), luciferase (pCMV-Luc; n= 3) or with a backbone vector (pcDNA1.1/Amp; n=6) lacking a eukaryotic cDNA sequence. Alternatively, 34-LC were electroporated with *in vitro* transcribed mRNA encoding EGFP or Melan-A (n=3). Results are shown as mean $\pm$ SD. * P<0.05.
Figure 7 shows the result of electroporation of immature monocyte-derived cells, in particular, the phenotype of dendritic cells 48 h after electroporation with GFP-RNA. The numbers in the lower right part of the quadrant indicate the EGFP-positive DC, the numbers in the upper right part show the EGFP+/CD83+ and EGFP+/CD25+ DC, respectively.
Figure 8 shows the transfection efficiency of and kinetics of EGFP expression in dendritic cells following GFP-RNA-transfection using electroporation.
Figure 9 shows the results of EGFP RNA-transfection of monocyte-derived dendritic cells by electroporation.

A: Contour plots showing the influence of voltage on cell size and granularity.
B: shows that the EGFP expression of CD83 and CD25 is influenced by the voltage.

Figure 10: EGFP RNA-transfection of mature monocyte-derived dendritic cells by electroporation.

A and H show the transfection efficiency and kinetics of EGFP expression following GFP-RNA transfection of mature dendritic cells using electroporation.
B to G confirm that the phenotype of dendritic cells is maintained after electroporation with GFP-RNA.

Figure 11: FCM analysis of transgene expression in immature and mature DC after EGFP mRNA electroporation in non-frozen controls and after thawing of cryopreserved samples. The dot plots show EGFP fluorescence on the x-axis and ethidium bromide staining on the y-axis. Analysis was performed on cells exhibiting a large forward scatter and large side scatter profile, in order to allow exclusion of contaminating autologous lymphocytes. Percentage of dead cells (upper left corner), viable EGFP+ cells (lower right corner) and viable EGFP- cells (lower left corner) is indicated based on the number of dots in the quadrant analysis. (A) Dot plots show analysis of non-frozen iMo-DC 24 hours after mRNA electroporation (left), and of mRNA-electroporated iMo-DC 6 hours after thawing (middle) and 24 hours (right) after thawing. (B) Dot plots show analysis of non-frozen mMo-DC 24 hours after mRNA electroporation (left),and of mRNA-electroporated mMo-DC 6 hours after thawing (middle) and 24 hours (right) after thawing. EP= electroporation.

Figure 12: Representative example of phenotypical analysis of non-frozen and frozen mRNA-electroporated immature and mature DC. Dot plots show FCM analysis of PE-labeled monoclonal antibodies directed against typical DC-markers including CD1a, HLA-DR, CD80 and CD86 (y-axis). As controls to set quadrants, isotype-matched antibodies and a PE-labeled monoclonal CD14 antibody was used. Analysis of DC markers was done on viable EGFP- cells in control samples and on viable EGFP+ cells in mRNA-electroporated DC as shown by the EGFP fluorescence on the x-axis. (A) iMo-DC on day 8 of culture, (B) EGFP+ iMo-DC after mRNA electroporation on day 6, followed by 48 hours of culture. (C) EGFP+ iMo-DC after mRNA electroporation on day 6, culture for 18 hours, cryopreservation, thawing and culture for 24 hours. (D) iMo-DC that have been stimulated for 48 hours with a maturation cocktail after day 6. (E) EGFP+ iMo-DC after mRNA electroporation on day 6 and stimulation for 48 hours with the maturation cocktail, (F) EGFP+ iMo-DC after mRNA electroporation on day 6 and culture for 24 hours with a maturation cocktail, cryopreservation, thawing and culture for 24 hours in presence of the maturation cocktail. In general, phenotyping was performed after 2 days of culture, with or without a frozen interval (that was not counted), following day 6 of the Mo-DC culture.

As shown by the dot plot analysis of Figure 12, iMo-DC undergo maturation at 48 hours after mRNA electroporation as demonstrated by an upregulation of HLA-DR, CD80 and CD86 (Figure 12, A & B). Thawed DC have the same upregulation of HLA-DR and CD80, but have lower levels of CD86 (Figure 12, C). This is probably caused by the fact that the frozen immature DC culture is dying 24 hours after thawing. Immature Mo-DC responded well to the maturation cocktail as seen by the upregulation of HLA-DR, CD80 and CD86 in mMo-DC as compared with the expression levels in iMo-DC (Figure 12, A & D). However, the combination of mRNA electroporation and a maturation stimulus seems to be very potent in maturing DC, as this combination results in high level of HLA-DR, CD80 and CD86 expression (Figure 12, E). Frozen mature DC that were electroporated show high level maturation marker expression after thawing (Figure 12, F).

Figure 13: Stimulatory capacity of cryopreserved mRNA-electroporated mature DC. Cryopreserved matrix protein M1 mRNA-electroporated mature DC were used as stimulators for PBMC during a 6 day coculture. Primed PBMC were then stimulated with T2 cells, pulsed with an MHC class I-restricted M1 immunodominant epitope, during a 6 hour coculture. Antigen specific T cells in the primed PBMC culture were detected as shown by positive IFN-$\gamma$ production. As controls, unpulsed T2 cells were used as stimulators and fresh PBMC as responders. Results are shown as mean $\pm$ standard error.

Upon restimulation with peptide-pulsed T2 cells, the activated T cells in the primed PBMC culture produced IFN-$\gamma$ against the immunodominant matrix protein peptide. The specificity of this activation is shown by only background IFN-$\gamma$ production of the primed PBMC culture against unpulsed T2 cells. To show that these cultured PBMC were stimulated during the 6 day culture, the same experiment was done with fresh PBMC. After coculture with either T2 cells or T2 cells pulsed with the peptide, no IFN-$\gamma$ production was detected above background level (Figure 13).

**Detailed Description of the Invention**

In the method of the invention it is preferred that an conventional electroporation apparatus is utilized which provides for an exponential decay pulse. It is moreover preferred that the electroporation is performed at a voltage from 200 to 350 V, most preferably from 250 to 300 V. It is also preferred that the capacitance is 150 to 250 $\mu$F. The pulsing time is strongly dependent from the type of the tray (cuvette) and the amount of reaction mixture (cell suspension) in the cuvette and is from 1 to 40 ms. For a 4 mm cuvette and 200 $\mu$l reaction mixture the pulsing time is from 5 to 40 ms, preferably 1 to 25 ms, and most preferably 7 to 10 ms. For a different cuvette and/or different amount of reaction mixture volumes, different voltage and pulsing times can easily be determined by the skilled artisan.

In the method of the invention the concentration of the cells in the suspension is preferably $1x10^3$ to $1x10^9$ cells per ml, more preferably $1x10^5$ to $1x10^9$ cells per ml. Even more preferred are $1x10^5$ to $5x10^7$ cells per ml, most preferably 1 to $4x10^7$ cells per ml.

The linear RNAs to be transfected are so-called "naked" RNAs, i.e. RNAs which are not complexed or stabilized by a ligand or the like. Linear RNAs to be utilized in the present invention include, but are not limited to, modified or unmodified, defined or undefined RNA and all kinds of modified variants thereof. The most preferred linear RNAs is mRNA. It is moreover preferred that the concentration of the RNAs to be transfected is $1x10^{-7}$ to $1x10^{-5}$ mmol/ml, preferably $4x10^{-6}$ to $6x10^{-6}$ mmol/ml.

All types of hematopoietic cells and stem cells, except for human embryonic stem cells, can be electroporated with the method of the invention, such as vertebrate cells including mammalian cells (such as human cells, rodent (mouse, rat) cells) and non-vertebrate cells (such as cells of fish and worms).

The hematopoietic cells and stem cells not including human embryonic stem cells are preferably mammalian/human cells including stem cells and derivatives thereof such as embryonic stem cells, hematopoietic stem cells and derivatives thereof (but not including human embryonic stem cells), and hematopoietic cells including mononuclear cells, marrow $CD34^+$ progenitor derived dendritic cells, $CD34^+$ progenitor derived Langerhans cell, monocycle-derived dendritic cells (Mo-DC), and most preferably are Mo-DC including immature Mo-DC and mature Mo-DC, but can also be applied to DC precursors or progenitors such as monocytes or CD34+ hematopoietic progenitor cells and also to embryonic stem cells. The method of the invention is also suitable to transduce primary bone marrow cells by RNA electroporation (it could be shown that mRNA electroporation of total bone marrow mononuclear cells is possible). The above mentioned precursor cells are electroporated with mRNA encoding the relevant antigen prior to (rapid) differentiation into dendritic cells. This strategy will be published in Ponsaerts et al. Journal of Immunology 2002, in press. This approach might also be of value for other types of precursor dendritic cells including CD123+ plasmacytoid dendritic cells or fresh CD11c+ blood dendritic cells that have a relative short halflife *in vitro.*

The linear RNAs used in the method of the invention may be any functional nucleotide sequence exhibiting a certain effect in the eukaryotic cell, which includes RNAs encoding proteins or peptides to be expressed in the eukaryotic cells, RNAs being functional or regulatory sequences and the like. The proteins or peptides to be expressed in the eukaryotic cells may or may not have a direct function in the eukaryotic cells, i.e. the expessed protein or peptide changes the property of the transfected cell, or is merely expressed in the cell or secreted by the cell (e.g. is a reporter gene or a gene product in acordance with embodiment (6)). The above mentioned proteins or peptides encoded by the linear RNAs include tumor antigens, microbial antigens, viral antigens, immunostimulatory or tolerogenic molecules, anti-apoptotic molecules, adhesion and homing molecules and antigen processing molecules. The above mentioned functional or regulatory sequences include, but are not limited to, differentiation-regulating genes, differentiation-associated genes and tissue specific genes. Examples of the above proteins or peptides encoded by the linear RNAs are Reportergenes such as EGFP (Enhanced green fluorescent protein; SEQ ID NOs:1 and 2); Tumor/Viral Antigens such as WT1 (Wilms tumor 1 protein; SEQ ID NOs:3 and 4), E6 (Human Papilloma Virus E6 protein; SEQ ID NOs:5 and 6), E7 (Human Papilloma Virus E7 protein; SEQ ID NO:7 and 8), env (Human Immunodefficiency Virus env protein; SEQ ID NO:9), gag (Human Immunodefficiency Virus gag protein SEQ ID NO:10), tat(WT) (Human Immunodefficiency Virus tat(WT) proteins; SEQ ID NO: 11) tat(SLT) (Human Immunodefficiency Virus tat(SLT) protein SEQ ID NO:12), Nef (Human Immunodefficiency Virus Nef protein; SEQ ID NO:13), Ref (Human Immunodefficiency Virus Ref protein; SEQ ID NO:14); Melan-A/MART1 (Melanoma antigen Melan-A; SEQ ID NOs:15 and 16); MAGEA1 (Melanoma antigen 1; SEQ ID NOs:17 and 18); MAGEA3 (Melanoma antigen 3; SEQ ID NOs:19 and 20); Cytokines such as GM-CSF (Granulocyte-macrophage colony stimulating factor; SEQ ID NOs:21 and 22), IL-2 (interleukin 2; SEQ ID NOs:23 and 24); and Genes for Stem Cells such as Nkx2.5 (CSX: cardiac-specific homeo box; SEQ ID NOs:25 and 26), Notch (Notch homolog 1; ; SEQ ID NOs:27 and 28), BAALC (brain and acute leukemia, cytoplasmic Locus; SEQ ID NOs:29 and 30), Wnt genes, GATA-4, GABA, desmine and cardiac troponine.

For the electroporation, the following parameters were most preferred: a 4 mm cuvette with 200 μl of cell suspension and we shock the cells using 300 volts and a capacitance of 150 μF (pulse time 8-10 ms). These are optimal parameters for both leukemic K562 cells and different types of DC, both progenitor- and monocyte-derived DC. In the optimization process, other parameters were also checked, e.g., by ranging the voltage and the capacitance, as well as the volume in the cuvette, resulting in shorter or longer pulse times. In summary the following parameters for efficiency and toxicity of RNA electroporation were found:

300 V - 150 μF - 200 μl - 8 ms
450 V - 150 μF - 200 μl - 8 ms

The common denominator for RNA electroporation is the low voltage (range 100 V-450 V), combined with a low capacitance (150 to below 300 μF) (which is in contrast to DNA settings, for which a high capacitance is required) and a low electroporation volume (200 μl) to increase cell concentration.

Electroporation and incubations are all performed at room temperature and cells are resuspended in serumfree buffer (e.g. IMDM, RPMI, a serum reduced buffer (e.g. Opti-MEM®) or in optimized electroporation buffer Optimix® purchased from EquiBio, UK cat n# EKIT-E1). The electroporator type is Easyject Plus® (EquiBio) which only delivers exponential decay pulses. In Examples 2-4 a Gene Pulser II® (Biorad) was used.

The significant decrease in toxicity observed with mRNA electroporation could in part be explained by the less stringent electrical settings required for introduction of the RNA (Table 1). Nevertheless, mRNA electroporation performed at stringent DNA settings resulted in a lower cell toxicity as well, suggesting that cell toxicity is not solely due to the electroporation procedure itself, but can also be related to the nature of the introduced nucleic acids. Moreover, co-introduction of bacterial contaminants (e.g. LPS) often found in plasmid preparations, could affect cell viability (Gordillo, G. M., Transpl. Immunol., 7:83-94 (1999)).

In an attempt to compare DNA and mRNA loading of DC, it was unexpectedly observed that a nonspecific stimulation of the TIL clone with plasmid DNA- but not with mRNA-electroporated 34-DC and 34-LC (Fig. 6), which could be abolished by DNase I treatment of the plasmid DNA. Although this stimulatory effect of plasmid DNA confounded data interpretation, the impact of this phenomenon on DC loading with respect to antigen-presenting capacity needs further investigation. Possible involvement of immunostimulatory sequences present in plasmid DNA (i.e. unmethylated CpG motifs) should be considered (Klinman, D. M. et al., Proc. Natl. Acad. Sci. USA, 93:2879-2883 (1996); Klinman, D. M. et al., Vaccine, 17:19-25 (1999)).

Although mRNA lipofection was overall less efficient than mRNA electroporation for loading DC, especially 34-DC and 34-LC, these data were derived from experiments with only one cationic lipid, i.e. DMRIE-C. Therefore, we cannot exclude the possibility that other lipids would accomplish comparable, or even higher, efficiencies of transfection and/or of MHC class I-restricted antigen loading of Mo-DC, 34-LC or 34-DC as compared to mRNA electroporation. Using passive mRNA pulsing, we were not able to detect any EGFP expression nor CTL activation by any type of DC examined. Therefore, these results seem somewhat in contrast to the findings of Nair et al., Nat. Biotechnol., 16:364-369 (1998) who showed in pulsing experiments that immature Mo-DC can take up mRNA without the use of a transfection agent, and subsequently prime tumor-specific CTL *in vitro.* It is possible that in the Experiment of Nair et al. passive RNA pulsing of DC lead to effective RNA transfection in a substantial portion of DC as protein expression cannot be detected.

In conclusion, it is shown that IVT mRNA-based electroporation is a highly efficient and simple nonviral method to gene-modify human Mo-DC, 34-DC and 34-LC with tumor antigens. The technique described in this study can serve applications in DC-based tumor vaccine development and in other gene transfer protocols requiring high-level short-term transgene expression in hematopoietic cells.

## Examples

<u>Materials and methods</u>

<u>Electroporation Devices:</u>

<u>Normal pulse:</u> EasyjecT PLUS D2000 model SHV (220 V; exponential decay pulses) was purchased from EquiBio Ltd. (cat # EJ-002, Action Court, Ashford Road, Ashford, Middlesex, TW15 1XB, U.K). The EasyjecT PLUS is fully microprocessor controlled via a bench top remote control unit, featuring an LCD display, membrane keypad and "Smart Card" reader/recorder. Hard copies of the parameters and initiated pulse values can be taken using the EasyjecT printer (included in the EasysyjecT PLUS). This information is invaluable in confirming your experimental procedure and giving results assured information. The EasyjecT PLUS includes a multitude of safety and operating detection features to enable safe operation without compromising the experimental procedure. Features includes: -Pre-Arc detection, open and short circuit detection, pre-pulse

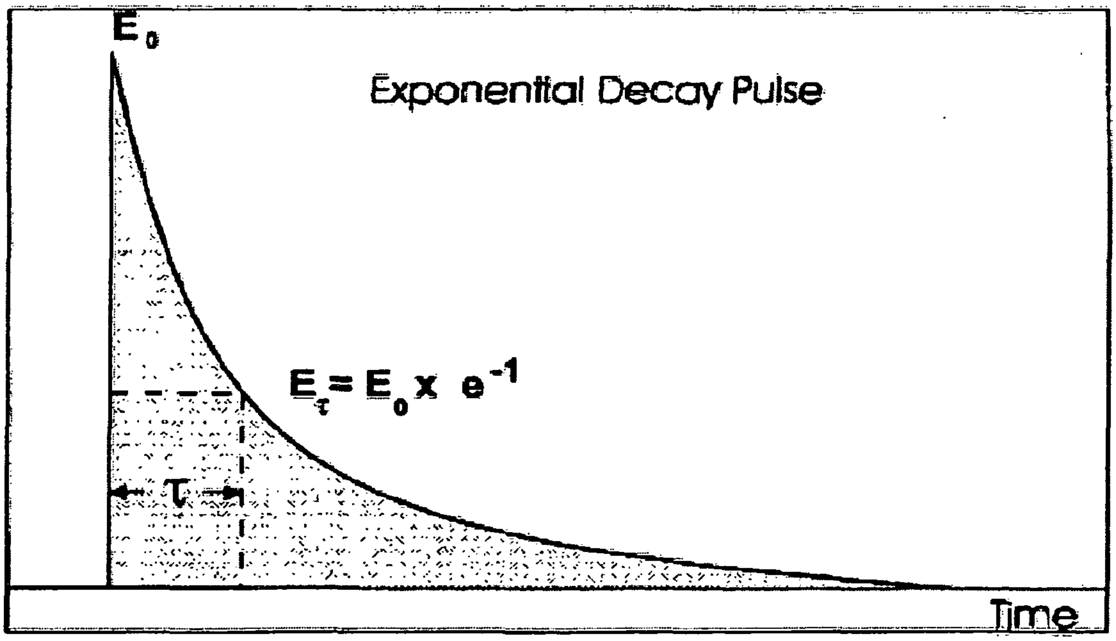


Scheme 1 Exponential decay pulse; relation with pulse time

experiments have resulted in unsuccessful or disappointing transformations.

Detailed specifications of the EasyjecT PLUS: Output Voltage: 100-3500 Volts (50V steps) in high voltage mode or 20-450 Volts (2V steps) in low voltage mode. Shunt resistor: 20 Ohms - Infinite (10 values). Capacitance range: 0.5μF/25μF in high voltage mode or 150-3000μF in low voltage mode. Pulse time: 10μs to 7 seconds. DOUBLE PULSE facility with inter pulse time 0 to 30 s. Program storage: 8 internal or 8 per smart card. Safety detection monitoring by visual and audible alarms for open and short circuit situations also if arcing should occur. Printer is included. Dimensions: Main unit (425 x 220 x 510mm) Key Pad (100 x 270 x 35mm) Chamber (260 x 67 x 92mm) Power 190-250Volts or 90-220 Volts Max 250Watts. Wave Form: Decaying exponential waveform with RC time constant dependant upon capacitor, sample and shunt resistor selected. In Examples 2-4 Gene Pulser II (Biorad)was used.

Electroporation cuvettes: Throughout the experiments with EasyjecT PLUS D2000, sterile 4mm electroporation cuvettes with cap (EquiBio, UK cat # ECU-104) were used. Each cuvette is individually wrapped and gamma-irradiated. Specifically designed sterile pipettes were used to further improve aseptic procedures. Total capacity is 800 μl.

Electroporation medium: Just before electroporation, cells were resuspended in Optimix® medium (EquiBio, UK, cat #EKIT-E1). Optimix is a QC-tested fully optimised medium, designed for the electroporation of eukaryotic cells. Optimix improves both transfection efficiencies and survival rates over phosphate-buffered saline (PBS) or other standard culture medium. The composition of Optimix has been carefully formulated to help protect cells during the electroporation process, also providing additional salts and critical molecules that help in the regeneration process following the destabilisation caused by the electrical discharge through the cell. The Optimix kit is ready to use and contains enough material for approx. 24 experiments. The kit is shipped at ambient temperature, however, it is important that some of the components are stored at either 4°C or -20°C on arrival. Optimix comprises 1 x 200ml of washing solution, 4 x 2.5ml of Optimix, 4x ATP and 4 x glutathione. Prior to use use, 5.5 mg ATP and 7.7 mg gltathione is mixed with 2.5 ml Optimix buffer and frozen in aliquots at -20°C.

Electrical parameters: Unless otherwise mentioned, typical mRNA electroporation settings were 300 V, 150 μF and an internal shunt resistance is put at infinity (+∞). Total volume in the cuvette is 200 μl containing 2-5 million cells resuspended in Optimix medium.

Electroporation mathematics: The electric field E is expressed in Volts per centimeter using the following formula:

$$E = V.d^{-1}$$

For which V is the output voltage of the electroporation apparatus and d is the distance between the electrodes of the cuvette. In our electroporation

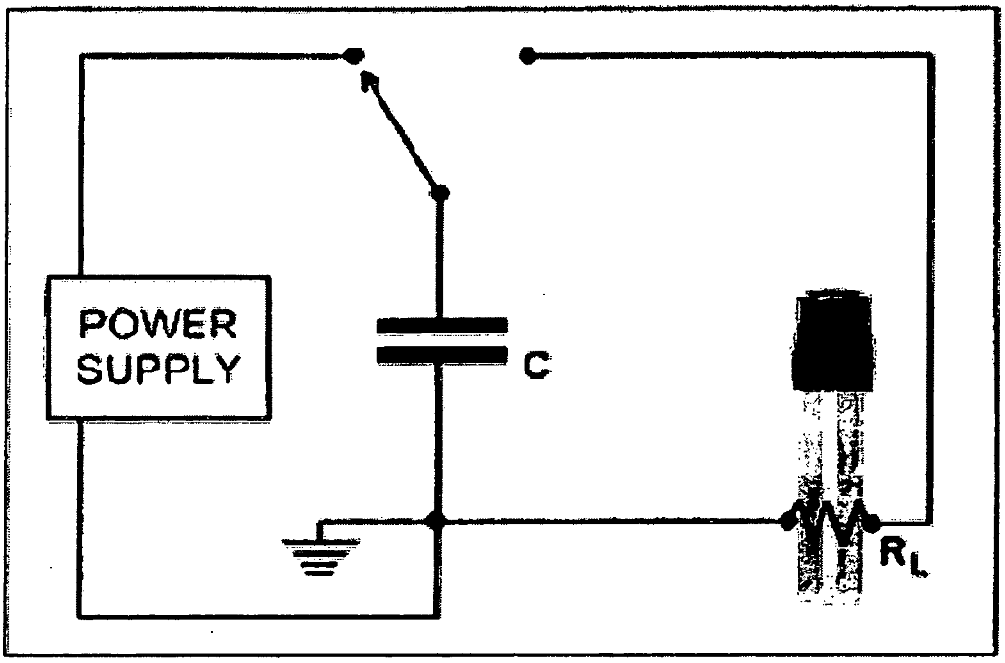


Scheme. 2: electroporation system; basic design for low voltage

technology, d is always 0.4 cm (4mm cuvette). Therefore, E is directly correlated with V and can be calculated according to:

$$E = V.(0.4)^{-1}$$

Pulse time ($\tau$) is by definition the elapsed time, in seconds, from the beginning of the pulse, when the electric field is maximum **($E_0$)** until the electric field has decreased to $e^{-1}$ (0.368) of the initial value $E_0$. Practically, this value is measured by the microprocessor of the electroporation unit. The pulse time for an ideal system can be calculated as follows:

$$\tau = R.C$$

in which **C** is the capacitance (expressed in Farads) and **R** is the resistance of the electrical circuit. The pulse time gives an estimation of the duration of the membrane pore formation process and is inversely correlated by the volume of electroporation medium in the cuvette and the directly correlated with the cell concentration in the cuvette and the resistance of the medium.

Cell lines: T2 cells (TAP-deficient, HLA-A2$^+$, TxB hybrid), EBV-LG2 (HLA-A2- EBV-transformed B lymphocytes), and SK23-MEL (Melan-A$^+$ HLA-A2$^+$ melanoma cell line) were kindly provided by Dr. Pierre Van der Bruggen (Ludwig institute for Cancer Research, Brussels, Belgium). K562 cells were obtained from the American Type Culture Collection (ATCC n° CCL-243, Rockville, MD, USA). Cell lines were cultured in complete medium consisting of Iscove's medium (IMDM) supplemented with L-glutamine (2 mM), penicillin (100 U/ml), streptomycin (100 μg/ml), amphotericin B (1.25 μg/ml Fungizone) and 10% fetal calf serum (FCS; Sera Lab, Sussex, UK). Cells were maintained in logarithmic phase growth at 37°C in a humidified atmosphere supplemented with 5% $CO_2$. All cell culture reagents were purchased from Gibco BRL (Paisley, UK).

Melan-A-specific CTL clone: The CD8$^+$ TIL 1235 clone recognizing the immunodominant HLA-A0201-restricted Melan-A$_{27-35}$ epitope (AAGIGILTV; SEQ ID NO:34) was a kind gift of Dr. J. Wunderlich (NIH, Bethesda, USA) and was cultured as described earlier with minor modifications (Reeves, M. E. et al., Cancer Res., 56:5672-5677 (1996)). Briefly, the TIL clone was maintained in AIM-V medium (Gibco BRL) supplemented with 5% pooled human AB serum (Sigma, Bornem, Belgium) and 500 IU/ml interleukin (IL)-2 (R&D Systems, Minneapolis, MN, USA) and used as responder population in DC coculture experiments.

Source of primary cells: Bone marrow (BM) samples were aspirated by sternal puncture from hematologically normal patients undergoing cardiac surgery, after informed consent. Peripheral blood mononuclear cells (PBMC) were obtained from healthy volunteers or hemochromatosis patients. The 6 PBMC donors used in this study are designated by letters A to F. Mononuclear cells were isolated by Ficoll-Hypaque gradient separation (LSM, ICN Biomedicals Inc., Costa Mesa, CA, USA). Monocytes were directly isolated and used for DC culture, as described below. PBMC for DC/T-cell cocultures were cryopreserved in a solution consisting of 90% FCS and 10% DMSO and stored at -80°C until use.

$CD34^+$ cell sorting: After Ficoll-Hypaque separation, mononuclear BM cells were indirectly stained using supernatant of the 43A1 hybridoma (anti-CD34) kindly donated by Dr. H-J. Bühring, University of Tübingen, Germany (Buhring, H. J. et al., Leukemia, 5:854-860 (1991)), followed by fluorescein isothiocyanate (FITC)-conjugated rabbit anti-mouse immunoglobulins (DAKO, Glostrup, Denmark). The CD34 labeled cells were then sorted on a $FACStar^{PLUS}$ cell sorter (Becton Dickinson, Erembodegem, Belgium) equipped with an air-cooled argon ion laser ILT model 5500-A (Ion Laser Technology, Salt Lake City, UT, USA). Sort windows were set to include cells with low side scatter and with positive green fluorescence ($CD34^+$). Purities of >95% were routinely obtained.

*In vitro* culture of DC: 34-DC cultures were cultured as described previously (Lardon, F. et al., Immunology, 91:553-559 (1997)). Briefly, 1-2.$10^5$ $CD34^+$ cells were cultured in 2 ml of complete medium supplemented with 100 ng/ml granulocyte-macrophage colony-stimulating factor (GM-CSF; Leucomax, Novartis Pharma, Basel, Switzerland), 2.5 ng/ml tumor necrosis factor (TNF)-$\alpha$ (Roche Molecular Biochemicals, Mannheim, Germany) and 50 ng/ml stem cell factor (SCF; Biosource, Nivelle, Belgium) until day 5; afterwards, SCF was replaced by 1000 U/ml IL-4 (R&D Systems), which was added for the next 5-9 days. After 12 days of culture, a 15-20 fold total cell expansion was observed and cells exhibited typical markers of mature DC including CD1a, CD80, CD86 and HLA-DR (Fig. 3C). For 34-LC, we used the protocol described by Herbst, B. et al., Blood, 88:2541-2548 (1996). Briefly, sorted $CD34^+$ cells were first cultured for 8 days in complete medium containing 100 ng/ml IL-3, 100 ng/ml IL-6 and 50 ng/ml SCF (all from Biosource), followed by LC differentiation in GM-CSF (100 ng/ml) and IL-4 (1000 U/ml) for the next 4 weeks. After 25 days of culture, a 75-100-fold increase in the total number of nucleated cells was observed and cells expressed high levels of CD1a and CD40, intermediate levels of HLA-DR and low levels of CD80 and CD86 and were able to efficiently take up FITC-dextran at 37°C (data not shown).

Immature monocyte-derived DC (iMo-DC) were generated from PBMC as described by Romani, N. et al., J. Exp. Med., 180:83-93 (1996). Briefly, PBMC were allowed to adhere in AIM-V medium for 2 h at 37°C. The non-adherent fraction was removed, and adherent cells were further cultured for 5-7 days in IMDM supplemented with 2.5% autologous heat-inactivated plasma. GM-CSF (100 ng/ml) and IL-4 (1000 U/ml) were added to the cultures every 2-3 days starting from day 0. Maturation of iMo-DC was induced by adding 2.5 ng/ml TNF-$\alpha$ and 100 ng/ml lipopolysaccharide (LPS; Sigma) for 24 h starting from day 6 of the Mo-DC culture.

Alternatively, monocytes derived from PBMCs were allowed to adhere in AIM-V medium (Gibco BRL, Paisly, UK) for 2 h at 37°C in 6-well culture plates (20x$10^6$ PBMC/well). After careful removal of the non-adherent fraction, cells were cultured in serum-free AIM-V medium supplemented with 100 ng/ml GM-CSF (Leucomax, Novartis Pharma, Basel, Switzerland) for 2 days. To obtain mature DC, poly-I:C (Sigma, Cambridge, UK) was added 24 hours after starting the culture at a concentration of 25 $\mu$g/ml. The typical yield and purity of the DC culture was 1-2x$10^6$ cells/well containing 60-70% of DC. For electroporation experiments, monocytes were isolated from PBMC by magnetic isolation using CD14 microbeads (Miltenyi Biotec, Bergisch Gladbach, Germany) according to manufacturer's instructions. Routinely, 4-8x$10^6$ monocytes were obtained starting from 100x$10^6$ PBMC with purity levels $\geq$ 85%.

HLA-A typing of DC: HLA-A2 subtyping was determined on BM-derived mononuclear cells and PBMC by indirect staining with the supernatant of the BB7-2 hybridoma (anti-HLA-A2; ATCC), followed by FITC-conjugated rabbit anti-mouse immunoglobulins (DAKO). HLA-A2 staining was analyzed by flow cytometry using a FACScan analytical flow cytometer (Becton Dickinson, Erembodegem, Belgium).

Synthetic peptides: An influenza virus-specific HLA-A*0201-restricted matrix protein M1 peptide (M1; amino acids (aa) 58-66, GILGFVFTL; SEQ ID NO:32) was used for activation or for detection of matrix protein M1 peptide specific T-cells when pulsed on respectively DC and T2 cells. A human papillomavirus (HPV) HLA-A2-restricted E7 protein-specific peptide (E7; amino acids (aa) 11-20, YMLDLQPETT; SEQ ID NO:33) was used in control experiments when pulsed on T2 cells. Melan-A peptide (MA; aa 27-35, AAGIGILTV; SEQ ID NO:34) was also used. Peptides (>95% pure) were purchased from Sigma-Genosys (Cambridge, UK). Both peptides were dissolved in 100% DMSO to 10 mg/ml, further diluted to 1 mg/ml in serum-free IMDM and stored in aliquots at -70°C. Peptides were used at a final concentration of 20 mM. The peptides (>95% pure) were purchased from Sigma-Genosys (Cambridge, UK). The peptides were dissolved in 100% DMSO to 10 mg/ml, further diluted to 1 mg/ml in serum-free IMDM and stored in aliquots at -80°C. The peptides were used at a final concentration of 20 $\mu$M. Peptide-pulsing of DC: T2 cells, HLA-$A2^+$ iMo-DC or DC were washed twice with IMDM and subsequently incubated (2x$10^6$ cells/ml) for 1 to 2 h at room temperature in 5 ml conical polystyrene tubes or 15 ml conical tubes with 20 $\mu$g/ml peptide in serum-free IMDM medium supplemented with 2.5 $\mu$g/ml $\beta$2-microglobulin (Sigma). Afterwards, the cells were washed and used respectively as stimulators for PBMC or as restimulators in cytokine release assays. Plasmids: For plasmid cDNA transfection, a pEGFP-N1 plasmid (CLONTECH Laboratories, Palo Alto, CA, USA) was used encoding an enhanced green fluorescent protein (EGFP) gene under the control of a CMV promoter plasmid pGEM4Z/EGFP/A64 (kindly provided by Dr. E. Gilboa, Duke University Medical Center, Durham, NC, USA) contained the EGFP gene under the control of T7 promoter. Plasmid pcDNA1.1/Melan-A contained the Melan-A/MART-1 gene driven by a CMV promoter was kindly provided by Dr. Pierre Van der Bruggen. pcDNA1.1/Amp (Invitrogen, Carlsbad, CA, USA) was used as a backbone control vector. Plasmid pCMV-Luciferase (CLONTECH Laboratories, Polo Alter, CA, USA) carried a luciferase gene under the control of a CMV promoter and was used as a control plasmid. Plasmid pGEM4Z/M1/A64 (kindly provided by Dr. A. Steinkasserer, University of Erlangen, Erlangen, Germany) encoding an influenza M1 gene under the control of a T7 promoter (SEQ ID NO:31). Plasmids were propagated in *E. Coli* strain DH5$\alpha$ (Gibco BRL) or supercompetent cells (Stratagene, La Jolla, CA, USA) and purified on endotoxin-free QIAGEN®-tip 500 columns (Qiagen, Chatsworth, CA, USA).

Production of *in vitro* transcribed (IVT) mRNA: For *in vitro* transcriptions, plasmids were linearized, purified using a Genieprep kit (Ambion, Austin, TX, USA) or a PCR purification Kit (Qiagen) and used as DNA templates for the *in vitro* transcription reaction. pcDNA1.1/Melan-A was used as such for *in vitro* transcription under the control of a T7 promoter. EGFP cDNA, isolated as a 0.8 kb HindIII-NotI fragment from pEGFP-H1, was first subcloned into pcDNA1.1/Amp and subsequently cloned as a BamHI-XbaI fragment into pSP64 (Promega, Madison, WI, USA) that allows *in vitro* transcription under the control of an SP6 promoter. Transcription was carried out in a final 20-100 $\mu$l reaction mix at 37°C for 3-4 h using the SP6 MessageMachine kit (Ambion) to generate 5' $m^7$GpppG-capped IVT mRNA. Transcription reactions with Spe I (MBI Fermentas, St. Leon Rot, Germany) linearized plasmids pGEM4Z/EGFP/A64 or pGEM4Z/M1/A64 as templates were carried out in a final 20$\mu$l reaction mix at 37°C using the T7 MessageMachine Kit (Ambion, Austin, TX, USA) to generate 5' capped *in vitro* transcribed (IVT) mRNA. Purification of IVT mRNA was performed by DNase I digestion followed by LiCl precipitation and 70% ethanol wash, according to manufacturer's instructions. For each experiment, at least three different batches of mRNA were used. mRNA quality was checked by agarose-formaldehyde gel electrophoresis. RNA concentration was assayed by spectrophotometrical analysis at $OD_{260}$. RNA was stored at -80°C in small aliquots (1 $\mu$g/$\mu$l).

Cell transfections: Prior to electroporation, K562 cells were washed twice with serum-free IMDM and resuspended to a final concentration of 5-10x$10^6$ cells/ml in Opti-MEM (Gibco BRL). After phenotypic analysis (performed in order to confirm the presence of $CD1a^+HLA\text{-}DR^+$ DC in the cultures), 34-DC, 34-LC and Mo-DC were routinely harvested after respectively 12, 25 and 6 days of culture (unless stated otherwise), washed twice with serum-free IMDM, and resuspended to a final concentration of 10-40x$10^6$ cells/ml in Opti-MEM. Subsequently, 0.5 ml of the cell suspension was mixed with 20 $\mu$g of IVT mRNA, and electroporated in a 0.4 cm cuvette using an Easyject Plus device (EquiBio, Kent, UK). In K562 cells, various voltages, capacitances and electroporation volumes were compared in order to assess their effect on mRNA transfection efficiency (see Results section). Electroporation of mRNA into CD14 microbead-isolated monocytes was done as described in Van Tenderloo, V. P. et al., Blood, 98:49 (2001), with minor modifications. Briefly, prior to electroporation, CD14 microbead-isolated monocytes were washed twice with Optimix Washing Solution (EquiBio, Ashford, Middlesex, UK) and resuspended to a final concentration of 50x$10^6$ cells/ml in Optimix electroporation buffer (EquiBio). Subsequently 0.2 ml of the cell suspension was mixed with 20 $\mu$g of IVT mRNA and electroporated in a 0.4 cm cuvette at 300 V and 150 $\mu$F using an Easyject Plus device (EquiBio). Plasmid DNA electroporation was performed as previously described (Van Tendeloo, V.F.I. et al., Gene Ther., 5:700-707 (1998)). After electroporation, fresh complete medium (including cytokines for DC) was added to the cell suspension and cells were further incubated at 37°C in a humidified atmosphere supplemented with 5% $CO_2$.

Lipofection of mRNA was performed using the cationic lipid DMRIE-C (Gibco BRL) according to manufacturer's instructions with minor modifications (Van Tendeloo, V.F.I, et al., Gene Ther., 5:700-707 (1998)). Briefly, K562 cells were washed twice with serum-free IMDM and resuspended to a final concentration of 1-2.$10^6$ cells/ml in Opti-MEM. 34-DC, 34-LC and Mo-DC were harvested after respectively 12, 25 and 6 days of culture, washed twice with serum-free IMDM, and resuspended to a final concentration of 1-2.$10^6$ cells/ml in Opti-MEM. Five $\mu$g of IVT mRNA, diluted in 250 $\mu$l Opti-MEM, was mixed with DMRIE-C, also diluted in 250 $\mu$l Opti-MEM, at a lipid:RNA ratio of 4:1. After 5-15 min of incubation at room temperature in order to allow RNA-lipid complexation, lipoplexes were added to the cells and allowed to incubate for 2 hours at 37°C. Alternatively, 5-20 $\mu$g of IVT mRNA was pulsed to the cells in the absence of DMRIE-C for 3-4 h at 37°C. Plasmid DNA lipofection was performed as described previously (Van Tendeloo, V.F.I, et al., Gene Ther., 5:700-707 (1998)). After lipofection or passive pulsing, fresh complete medium (including cytokines for DC) was added to each well. EGFP analysis: EGFP-transfected cells were checked for EGFP expression 24-48 h after transfection by flow cytometric (FCM) analysis. Briefly, cells (1-5 x $10^5$) were washed once in phosphate-buffered saline (PBS) supplemented with 1% FCS and resuspended in 0.5 ml of PBS supplemented with 1% BSA and 0.1% sodium azide. Ethidium bromide (EB) at a final concentration of 10 $\mu$g/ml was added directly prior to FCM analysis on a FACScan analytical flow cytometer (Becton Dickinson) to assess cell viability. For EGFP analysis in DC cultures, gating was performed on cells exhibiting a large forward scatter (FSC) and side scatter (SSC) profile, i.e. DC, in order to allow exclusion of contaminating autologous lymphocytes. Gated DC were then evaluated for EGFP expression. Immunophenotyping of DC: Immunophenotyping was performed as described previously (Van Tendeloo, V.F.I, et al., Gene Ther.,

5:700-707 (1998)). The following monoclonal antibodies were used: CD1a-fluorescein isothiocyanate (FITC) (Ortho Diagnostic Systems, Beerse, Belgium), CD1a-phycoerythrin (PE) (Caltag Laboratories, San Francisco, CA, USA), CD14-PE, HLA-DR-PE, HLA-DR-FITC (PharMingen, San Diego, CA, USA), CD4-PE, CD80-PE (Becton Dickinson), CD80-FITC (PharMingen, San Diego, CA, USA), CD40-FITC (BioSource, Zoersel, Belgium), CD86-PE (PharMingen, San Diego, CA, USA), CD86-FITC (Serotec, Oxford, UK), CD13-FITC (DAKO), CD14-FITC (Becton Dickinson, Erembodegem, Belgium) and the non-conjugated CD83 (HB-15 clone; Immunotech, Marseille, France). Immunophenotyping with CD83 was followed by staining with a secondary rabbit anti-mouse (RAM)-FITC antibody (Dako, Glostrup, Denmark). Nonreactive isotype-matched antibodies (Becton Dickinson) were used as controls. Ethidium bromide was added prior to FCM analysis on a FACScan analytical flow cytometer (Beckton Dickinson) to assess cell viability and to exclude dead cells from the analysis. Gating was also performed to exclude remaining lymphocytes in the DC cultures. In particular, for immunophenotyping of enhanced green fluorescent protein (EGFP) mRNA-electroporated DC, the following phycoerythrin (PE)-labeled monoclonal antibodies were used: CD1a-PE, HLA-DR-PE, CD80-PE, CD14-PE, CD86-PE, and the secondary RAM-PE antibody (Dako, Glostrup, Denmark) for CD83 staining.

Interferon (IFN)-γ release assay: 34-DC, 34-LC and iMo-DC were used as stimulator cells 24 h after transfection. To study the effect of maturation, 6-day-cultured iMo-DC were allowed to mature for 24 h in the presence of TNF-α and LPS prior to transfection and used as stimulators 24 h after transfection. Alternatively, iMo-DC were transfected with mRNA on day 6 of culture and, after 12-16 h to allow protein expression, TNF-α and LPS were added to induce final DC maturation. After an additional 24 h, mature transfected Mo-DC were used as stimulators. In some experiments, iMo-DC pulsed with the Melan-A, an irrelevant influenza M1 peptide or an irrelevant human papilloma virus E7 peptide were used as stimulators. Stimulators were either washed twice and resuspended in AIM-V medium supplemented with 10% pooled human AB serum and 40 IU/ml IL-2. Responder CTL were washed vigorously 3-4 times and resuspended in AIM-V medium. Then, CTL (1 x $10^5$ cells) were coincubated with stimulator cells (1x$10^5$ cells) in 96-round bottom plates for 24 h at 37°C in a total volume of 200 μl. Alternatively, stimulators and responder PBMC were washed and resuspended in IMDM + 5% hAB serum. Then, responder PBMC (1x$10^5$ cells) were coincubated with stimulator cells (1x$10^4$ cells) in 96-well round-bottom plates for 6 hours at 37°C in a total volume of 100 μl. Triplicate supernatant samples from these cocultures were tested for specific IFN-γ secretion by an IFN-γ ELISA (Biosource, Nivelle, Belgium). To normalize data, the background IFN-γ secretion (defined as IFN-γ released by the CTL exposed to unmodified DC) was subtracted from each of the observed measurements. Measurements are presented as IU/ml released by $10^5$ responder cells/24 h.

IFN-γ secreting cell assay: PBMC primed and cultured as described above (1x$10^6$) were restimulated for 3 hours in 24-well plates with T2 cells (1x$10^5$) pulsed with M1 peptide or E7 peptide as control. Next, IFN-γ-secreting cells were analyzed by a flow cytometric IFN-γ Secretion Assay Detection Kit (Miltenyi Biotec, Bergisch Gladbach, Germany) according to manufacturer's instructions. Cells were also stained with CD8-FITC (Becton Dickinson) and 5x$10^5$ cells were analyzed per sample by flow cytometry. Analysis was done by gating on the lymphocyte population.

Allogeneic mixed leukocyte reaction (MLR): Immature and mature DC were used for stimulation of allogeneic PBMC. Briefly, immature or mature DC were cocultured with 20x$10^6$ allogeneic PBMC (ratio 1:10) in 10 ml IMDM supplemented with 5% human (h) AB serum (Sigma) in T25 culture flasks. On day 4 of culture, 5 ml fresh medium (IMDM + 5% hAB serum) was added to the cultures. On day 7 of culture, cells were analyzed for reactivity. For this, stimulated PBMC (1x$10^5$ cells) were restimulated with PBMC from the DC donor (1x$10^4$ cells) in 96-well round bottom plates for 6 hours at 37°C in a total volume of 100 μl. Supernatant samples from these cocultures were tested for IFN-γ secretion by IFN-γ ELISA (Biosource, Nivelle, Belgium).

Induction of MHC class I-restricted influenza-specific T cells: M1 peptide-pulsed immature, M1 peptide-pulsed mature DC and matrix protein mRNA-electroporated mature DC were used for antigen-specific stimulation of PBMC. Briefly, 2x$10^6$ antigen-loaded DC were cocultured with 20x$10^6$ autologous PBMC (ratio 1:10) in 10 ml IMDM supplemented with 5% hAB serum in T25 culture flasks. On day 4 of culture, 5 ml fresh medium (IMDM + 5% hAB serum) was added to the cultures. On day 7 of culture, cells were analyzed for antigen specificity.

Example 1

A. Optimization of IVT mRNA transfection in K562 cell: In preliminary experiments to optimize mRNA electroporation, we used leukemic K562 cells, as these cells were readily transfectable by plasmid electroporation (Baum, C. et al., Biotechniques, 17:1058-1062 (1994)). The EGFP reporter gene was used to assess mRNA transfection efficiency. Various electroporation settings were tested and transfection efficiency was determined by FCM analysis of EGFP expression (Fig. 1A). Of all tested electrical settings, a voltage of 300 V combined with a capacitance of 150 μF in a total cuvette volume of 200 μl resulted in the highest EGFP expression (Table 1).

Table 1: Optimization of mRNA electroporation parameters in K562 cells

Electroporation

| | voltage (V) | capacitance (µF) | cell volume (µl) | efficiency (%) | viability (%) |
|---|---|---|---|---|---|
| DNA | 260 | 1050 | 500 | 40 | 49 |
| | 300 | 150 | 200 | 28 | 85 |
| RNA | 300 | 150 | 200 | 89 | 85 |
| | 300 | 300 | 200 | 83 | 59 |
| | 260 | 1050 | 500 | 81 | 73 |
| | 250 | 1500 | 500 | 80 | 69 |

Lipofection

| | lipid | lipid:DNA ratio | incubation time (h) | efficiency (%) | viability (%) |
|---|---|---|---|---|---|
| DNA | DMRIE-C | 3:1 | 6 | 26 | 88 |
| RNA | DMRIE-C | 4:1 | 2 | 22 | 80 |

K562 cells were transfected as described in the Materials and Methods section by electroporation or lipofection. Cells were analyzed 24 h after electroporation by FCM for EGFP expression to estimate transfection efficiency (= % EGFP$^+$ cells) as well as by ethidium bromide exclusion for cell viability. Results are the mean of four independent experiments each with a different IVT mRNA batch (standard error of the mean < 2.5%).

The vast majority of the viable cell fraction expressed EGFP to a significant extent. The percentage of EGFP-expressing K562 cells was markedly higher following mRNA electroporation than following plasmid cDNA transfection, even when cDNA electroporation was performed at optimal DNA electroporation settings, i.e. 260 V and 1050 µF (Fig. 1A). Furthermore, mRNA electroporation at optimal settings showed a significantly reduced cell mortality rate as compared to cDNA electroporation at optimal settings (15% versus 51%, respectively). DMRIE-C-mediated RNA and DNA lipofection showed a somewhat similar outcome in terms of efficiency and viability although optimal lipid:nucleic acid ratio (4:1 versus 3:1) as well as incubation time (2 h versus 6 h) varied for RNA and DNA lipofection, respectively (Table 1).

As RNA is extremely labile and has a short half-life time compared to DNA, we also studied kinetics of EGFP expression following mRNA electroporation (Fig. 1B). Transgene expression in K562 cells peaked at 24-48 h and rapidly declined to background levels after 6 days.

B. Efficiency of IVT mRNA transfection in different types of DC: Immature Mo-DC (iMo-DC) were generated from adherent PBMC in the presence of GM-CSF and IL-4. At day 5-6 of culture, Mo-DC were electroporated with EGFP mRNA. Optimization experiments revealed optimal settings similar to those of K562 cells (300 V, 150 µF), leading to maximal transfection efficiency combined with the lowest level of cell death. FCM analysis of EGFP expression showed more than 60% EGFP-expressing iMo-DC (Fig. 2A & Table 2). Mortality in Mo-DC after mRNA electroporation ranged from 15-30% (mean cell mortality rate 22±8%), although untransfected Mo-DC cultures already exhibited some degree of cell death (5-10%). When gating on the viable population, 85% of viable Mo-DC expressed EGFP to some extent. TNFα+LPS-induced maturation of Mo-DC prior to transfection showed a significant decrease in electroporation and lipofection efficiency (Table 2). DC maturation following mRNA transfection had no effect on transgene expression (data not shown). Lipofection of EGFP mRNA in Mo-DC resulted in a much lower efficiency (7.5±0.5%) and was slightly more toxic (mean cell mortality rate 28±10%) to the cells than mRNA electroporation, as shown in Fig 2A. Passive pulsing of DC with mRNA did not resuit in any detectable EGFP expression. Kinetic analysis of mRNA expression in Mo-DC showed a maximum 24 h after electroporation, followed by a slow decline in function of time (Fig. 2B). Five days after mRNA electroporation, EGFP was still detectable in a substantial proportion of Mo-DC (31% EGFP$^+$ cells), in contrast to transgene expression kinetics in K562 cells (9% EGFP$^+$ cells after 5 days). Monitoring of cell viability after mRNA electroporation revealed a somewhat stable viability in function of time (Fig. 2B).

mRNA transfection in bone marrow CD34$^+$ progenitor-derived DC (34-DC) and CD34$^+$ progenitor-derived Langerhans cells (34-LC) was also tested. Up to 72% and 53%, respectively, of these DC types were readily transfected by mRNA electroporation (Fig. 2C), but not by mRNA lipofection or mRNA pulsing (Table 2). Viability was always higher than 80% for both 34-DC and 34-LC (Fig. 2C). Table 2 summarizes efficiency of mRNA-based electroporation, lipofection and passive pulsing in the different types of DC.

Table 2: Efficiency of mRNA transfection in different types of DC

| | Transfection efficiency (%) | | | |
|---|---|---|---|---|
| Method of transfection | iMo-DC | mMo-DC | 34-LC | 34-DC |
| | | | | |
| Electroporation | 63±9 | 33±8 | 50±3 | 73±3 |
| | | | | |
| Lipofection | 7.5±2.5 | 4±2.3 | <BG | <BG |
| | | | | |
| Passive pulsing | <BG | <BG | <BG | <BG |
| | | | | |

Different types of DC were transfected with IVT EGFP mRNA using electroporation, lipofection or passive pulsing. One day after transfection, EGFP expression was analyzed by FCM to estimate transfection efficiency (% $EGFP^+$ DC). iMo-DC, immature Mo-DC; mMo-DC, mature Mo-DC; 34-LC, $CD34^+$ progenitor-derived Langerhans cells; 34-DC, $CD34^+$ progenitor-derived dendritic cells. Results are the mean ± standard deviation (SD) of at least three independent experiments for passive pulsing, lipofection or electroporation; < BG, EGFP expression below background fluorescence.

Phenotype and maturation of mRNA-electroporated DC: Since DC have a delicate phenotype which can easily be disturbed by culture or transfection conditions, we assessed by FCM analysis whether electroporated DC retained their respective phenotype as well as their capacity to differentiate into mature DC. Control and EGFP mRNA-transfected Mo-DC were stained using monoclonal antibodies binding to characteristic DC markers including CD1a, HLA-DR, CD80, CD86 and CD83. Electroporation of mRNA showed no effect on the phenotype of Mo-DC, as electroporated Mo-DC co-expressing EGFP retained high levels of CD1a, HLA-DR and CD86 (Fig. 3A). Mock electroporation (electroporation without mRNA) gave similar results (data not shown). The capacity of mRNA-electroporated Mo-DC to differentiate to mature Mo-DC was evaluated by expression of mature DC markers including CD80 and CD83. Fig. 3B shows that mRNA electroporation itself did not induce DC maturation, but that the maturation potential after electroporation was retained since mRNA-transfected immature Mo-DC were able to upregulate CD83 and CD80 in the presence of a maturation cocktail (TNF-$\alpha$ + LPS).

Also, the phenotype of 34-DC was also not affected by mRNA electroporation (Fig. 3C). $EGFP^+$ 34-DC co-expressed HLA-DR, CD1a, CD80 and CD86. Similar findings were observed in 34-LC, with the exception that 34-LC exhibited lower levels of CD80 and CD86, compatible with their similarity to immature Langerhans-like DC (data not shown).

C. MHC class I-restricted antigen presentation by mRNA-transfected DC: Given the high transfection efficiency in Mo-DC, we investigated to what extent mRNA-transfected Mo-DC could process antigen and present MHC class I-restricted antigenic epitopes to an antigen-specific CTL clone. Therefore, we introduced mRNA encoding Melan-A/MART-1 into HLA-$A2^+$ Mo-DC using electroporation, lipofection or passive pulsing. Mo-DC electroporated or lipofected with Melan-A mRNA markedly stimulated an HLA-$A2^+$ Melan-A-specific CTL clone, as judged by IFN-$\gamma$ secretion (Fig. 4). Mo-DC passively pulsed with Melan-A mRNA did not result in any CTL stimulation. HLA-$A2^+$ Mo-DC electroporated with EGFP mRNA or HLA-$A2^-$ Mo-DC electroporated with Melan-A mRNA did not stimulate the CTL clone to produce IFN-$\gamma$. Both HLA-$A2^+$ Melan-$A^+$ SK23-MEL melanoma cells and HLA-$A2^+$ Mo-DC, pulsed with the immunodominant Melan-$_{A27-35}$ peptide AAGIGILTV, were used as positive controls and induced strong IFN-$\gamma$ production by the CTL clone. HLA-$A2^+$ Mo-DC pulsed with the M1 influenza peptide did not elicit any specific IFN-$\gamma$ production. Mo-DC electroporated with Melan-A IVT mRNA stimulated the CTL clone more than twice stronger than mRNA-lipofected Mo-DC (Fig. 4), suggesting a correlation with the difference in transfection efficiency between the two gene transfer methods (Table 2). The observation that transfection efficiency and CTL activation were correlated, was also made when comparing efficiency of mRNA electroporation (Table 2) and the capacity to stimulate the CTL clone in the other types of DC (Table 3). Electroporation of HLA-$A2^+$ 34-DC and 34-LC with Melan mRNA, but not EGFP mRNA, led to specific CTL activation. In concordance with the absence of any detectable transfection level (Table 2), lipofection of 34-DC and 34-LC or passive pulsing of all types of DC with Melan-A mRNA did not result in any IFN-$\gamma$ detectable above background levels (Table 3).

Table 3: CTL activation by different types of DC

| | CTL activation (IU IFN-$\gamma$/ml/24 h) | | | |
|---|---|---|---|---|
| Method of transfection | iMo-DC | mMo-DC | 34-LC | 34-DC |
| | | | | |
| Electroporation | 11.3$\pm$2.2 | 5.8$\pm$1.8 | 6.9$\pm$1.4 | 7.7$\pm$3 |
| | | | | |
| Lipofection | 3.7$\pm$1.1 | 1.5$\pm$0.8 | <BG | <BG |
| | | | | |
| Passive pulsing | <BG | <BG | <BG | <BG |
| | | | | |

Different types of DC were transfected with IVT Melan mRNA using electroporation, lipofection or passive pulsing. One day after transfection, $10^5$ transfected DC were cocultured for 24 h with $10^5$ Melan-A-specific CTL at 37°C. Afterwards, supernatants were collected and IFN-$\gamma$ secretion was checked by IFN-$\gamma$ ELISA, as described in the Materials and Methods section. Results are the mean $\pm$ SD of at least five independent experiments for electroporation and of 3 independent experiments for passive pulsing and lipofection. iMo-DC, immature Mo-DC; mMo-DC, mature Mo-DC; 34-LC, $CD34^+$ progenitor-derived Langerhans cells; 34-DC, $CD34^+$ progenitor-derived DC; <BG, IFN-$\gamma$ production below background.

D. Effect of maturation on mRNA loading in Mo-DC : Mo-DC obtained by culturing PBMC in the presence of GM-CSF and IL-4 for 5-7 days exhibit predominantly an immature phenotype (Romani, N. et al., J. Immunol. Methods, 196:137-151 (1996)). These immature Mo-DC are specialized in capturing large amounts of antigens from the environment (Sallusto, F., Lanzavecchia, A., J. Exp. Med., 179:1109-1118 (1994)). However, for optimal presentation to CTL, Mo-DC need to undergo a maturation process which can be induced by bacterial products (e.g. LPS), inflammatory cytokines (e.g. TNF-$\alpha$) and/or CD40 ligation by T helper cells (Bancherau, J., Steinmann, R. N., Nature, 392:245-252 (1998)). Therefore, in order to test whether maturation and the sequence of loading affected the antigen-presenting capacity of Mo-DC, we evaluated the ability of Mo-DC loaded with Melan-A by mRNA electroporation to stimulate the CTL clone prior to and after maturation with LPS+TNF-$\alpha$. Figure 5 clearly indicates that the most potent CTL activation was obtained when mRNA loading by electroporation or lipofection was performed prior to maturation of Mo-DC. When maturation occurred prior to mRNA loading, there was a significant decrease in IFN-$\gamma$ secretion by TIL cells (Fig. 5), likely to be correlated with the lower degree of transfectability of mature Mo-DC which were matured with LPS and TNF-$\alpha$ (Table 2).

E. cDNA loading versus mRNA toadying: In contrast to Mo-DC, 34-LC and 34-DC can also be transfected by plasmid DNA electroporation (Van Tendeloo, V.F.I. et al., Gene Ther., 5:700-707 (1998)). Therefore, we evaluated whether plasmid DNA-transfected DC can also induce antigen-specific CTL activation. HLA-$A2^+$ 34-LC electoporated with plasmid DNA or IVT mRNA encoding Melan-A were incubated with the Melan-A specific CTL to evaluate IFN-$\gamma$ secretion (Fig. 6). Strikingly, we reproducibly obtained similar IFN-$\gamma$ levels with Melan-A cDNA- as with control vector-transfected 34-LC, indicating nonspecific CTL stimulation. Transfection with two other irrelevant plasmids (pEGFP-N1 and pCMV-Luciferase) resulted in a similar nonspecific CTL stimulation. This phenomenon was never observed in mock-transfected (electroporation without plasmid DNA) 34-LC or when the DNA was digested by DNase I prior to electroporation (Fig. 6). Similar observations were made in 34-DC (data not shown).

Example 2: EGFP RNA-transfection of immature monocyte-derived dendritic cells (generated from leukapheresis products and matured by a cocktail of It-1ß + IL-6 + TNF$\alpha$ + $PEG_2$ under GMP conditions for clinical application) by electroporation. Monocyte-derived immature Dendritic Cells (DC) were generated from leukapheresis products as described (Feuerstein, B. et al., J. Immunol. Methods 245: 15-29 (2000)). Immature DC (d6) were washed twice in RPMI and once in washing-solution of the Optimix®-Kit (EQUIBIO, Maidstone Kent, U.K.). DC were adjusted to a final cell concentration of $10\times10^6$/ml in Optimix®-Medium. Then 0,2 ml of the cell suspension were mixed with 20 $\mu$g in vitro transcribed EGFP RNA in a 1,5 ml reaction tube. After incubation at room temperature for a maximum of 3 minutes the cell suspension were transferred in a 0,4-cm-gap electroporation-cuvette. Pulse were triggered at a voltage of 300 V and a capacitance of 150 $\mu$F with the Gene Pulser II (BioRad, Munich, Germany) resulting in pulse time of 7-10 msec. Immediately after that the cell suspensions were transferred to 6-well-plates ($1\times10^6$ DC/ well/3 ml culture medium supplemented with GM-CSF and IL-4). In the half number of the wells terminal maturation was induced by addition of It-1ß, IL-6, TNF-$\alpha$ and $PGE_2$ as described (Feuerstein, B. et al., J. Immunol. Methods 245: 15-29 (2000)). 48 h after electroporation the DC were counterstained with the indicated mouse mAbs and PE-conjugated anti-mouse Ig followed by FACS-analysis. The results are summarised in Fig. 7.

The addition of a maturation cocktail after transfection leads to a population of Dendritic Cells that is more mature as indicated by expression of CD83 and CD25 by a much higher percentage of DC. This is important as only mature DC induce immunity *in vivo* while immature ones can induce tolerance (Roncarolo, M.G. et al., Exp. Med. 15;193(2):F5-9. Review. (2001)).

Monocyte-derived immature Dendritic Cells (DC) were processed as described above, and following addition of the maturation stimulus the longevity of EGFP expression in mature transfected DC was examined. Expression of EGFP is maintained in the majority of cells even after 4 days. The results are summarised in Fig. 8.

Example 3: EGFP RNA-transfection of monocyte-derived dendritic cells by electroporation - Titration of Voltage.

A. Influence of voltage on cell size and granularity: Monocyte-derived immature Dendritic Cells (DC) were generated from leukapheresis products as described (Feuerstein, B. et al., J. Immunol. Methods 245: 15-29 (2000)). Immature DC (d6) were washed twice in RPMI and once in washing-solution of the Optimix®-Kit (EQUIBIO, Maidstone Kent, U.K.). DC were adjusted to a final cell concentration of $10\times10^6$/ml in Optimix®-Medium. Then 0,2 ml of the cell suspension were mixed with or without 20 μg in vitro transcribed EGFP RNA in a 1,5 ml reaction tube. After incubation at room temperature for a maximum of 3 minutes the cell suspension were transferred in a 0,4-cm-gap electroporation-cuvette. Pulse were triggered at the indicated voltage and a capacitance of 150 μF with the Gene Pulser II (BioRad, Munich, Germany) resulting in pulse time of 7-10 ms. Immediately after that the cell suspensions were transferred to 6-well-plates ($1\times10^6$ DC/ well/3 ml culture medium). Terminal maturation was induced by addition of It-1ß, IL-6, TNF-a and $PGE_2$ as described (Feuerstein, B. et al., J. Immunol. Methods 245: 15-29 (2000)). 48 h after electroporation the DC were analyzed. The contour-plots of Fig. 9A show on the x-axis the forward side scatter and on y-axis the sideward scatter.

The Forward and Side Scatter analysis addition reveals that for monocyte-derived Dendritic Cells that are generated from leukapheresis products, RNA-transfected by electroporation, and fully matured by adding a maturation cocktail consisting of of It-1ß, IL-6, TNF-a and $PGE_2$ (Feuerstein, B. et al., J. Immunol. Methods, 245: 15-29 (2000)) the use of 260 V is slightly better as the integrity of the cells is somewhat better preserved.

B. Influence of voltage on CD83 and CD25: Immature DC (d6) - see Fig. 9A - were washed twice in RPMI and once in washing-solution of the Optimix®-Kit (EQUIBIO, Maidstone Kent, U.K.). DC were adjusted to a final cell concentration of $10\times10^6$/ml in Optimix®-Medium. Then 0,2 ml of the cell suspension were mixed with or without 20 μg *in vitro* transcribed EGFP RNA in a 1,5 ml reaction tube. After incubation at room temperature for a maximum of 3 minutes the cell suspension was transferred in a 0,4-cm-gap electroporation-cuvette. Pulses were triggered at the indicated voltage and a capacitance of 150 μF with the Gene Pulser II (BioRad, Munich, Germany) resulting in pulse time of 7-10 msec. Immediately after that the cell suspensions were transferred to 6-well-plates ($1\times10^6$ DC/ well/3 ml culture medium). Terminal maturation was induced by addition of IL-1ß, IL-6, TNF-a and $PGE_2$. 48 h after electroporation the DC were counterstained with the indicated mouse mAbs and PE-conjugated anti-mouse Ig followed by FACS-analysis. The results are shown in Fig. 9B.

The phenotypic analysis reveals that for monocyte-derived Dendritic Cells that are generated from leukapheresis products, RNA-transfected by electroporation, and fully matured by adding a maturation cocktail consisting of of IL-1ß, IL-6, TNF-a and $PGE_2$ (Feuerstein, B. et al., J. Immunol. Methods, 245: 15-29 (2000)) the use of 260 V is slightly better as more cells are in the upper right quadrant, i.e. expressing both EGFP and the maturation markers CD83 and CD25.

Example 4: EGFP RNA-transfection of already matured monocyte-derived dendritic cells (generated from leukapheresis cells and matured by a cocktail of IL-1ß + IL-6 + TNFα + $PEG_2$ under GMP conditions for clinical application) by electroporation.

A: Monocyte-derived immature Dendritic Cells (DC) were generated from leukapheresis products as described (Feuerstein, B. et al., J. Immunol. Methods, 245:15-29 (2000)). Immature DC (d6) were induced to undergo terminal maturation by addition of IL-1ß, IL-6, TNF-α and $PGE_2$ as described in Feuerstein, B. et al., J. Immunol. Methods 245: 15-29 (2000). Mature DC were transfected with EGFP-RNA by electroporation as described in Example 2.

While DC matured by TNFα + LPS are transfected only to a mean of 33 %, from the results depicted in Figs. 10A and H it can be concluded that mature monocyte-derived Dendritic Cells (matured by an optimised maturation cocktail consisting of IL-1ß + IL-6 + TNFα + $PGE_2$) are efficiently transfected, and maintain EGFP expression over the 48h time period tested.

B: Mature monocyte-derived Dendritic Cells (DC) are efficiently transfected, and maintain their mature phenotype (high expression of CD83, CD80, CD25, CD40, HLA-DR and MHC class I) over the 48h time period tested as it is confirmed by Fig. 10B-G.

The addition of a maturation cocktail after transfection leads to a population of Dendritic Cells that is more mature as indicated by expression of CD83 and CD25 by a much higher percentage of DC. This is important as only mature DC induce immunity *in vivo* while immature ones can induce tolerance (Roncarolo, M.G et al., J. Exp. Med. 15;193(2):F5-9. Review. (2000)).

Example 5: mRNA-electroporated mature dendritic cells retain transgene expression, phenotypical properties and stimulatory capacity after cryopreservation.

Table 4: Transgene expression in cryopreserved mRNA-electroporated K562 cells

| | % EGFP+ cells | MFI EGFP+ cells | % dead cells |
|---|---|---|---|
| Control: EGFP expression in mRNA-electroporated K562 cells (n=2) | | | |
| 3 h after EP | 66 ± 1 | 109 ± 15 | 19 ± 2 |
| 24 h after EP | 72 ± 1 | 363 ± 62 | 17 ± 1 |
| 48 h after EP | 73 ± 9 | 319 ± 67 | 18 ± 8 |
| Protocol1 : K562 cells frozen 3 hours after mRNA electroporation (n=3) | | | |
| 3 h after thawing | 60 ± 2 | 286 ± 11 | 31 ± 2 |
| 24 h after thawing | 73 ± 1 | 454 ± 19 | 18 ± 1 |
| 48 h after thawing | 80 ± 1 | 207 ± 2 | 11 ± 1 |
| Protocol 2: K562 cells frozen 24 hours after mRNA electroporation (n=3) | | | |
| 3 h after thawing | 72 ± 1 | 363 ± 62 | 22 ± 1 |
| 24 h after thawing | 77 ± 1 | 178 ± 43 | 13 ± 2 |
| 48 h after thawing | 80 ± 1 | 97 ± 43 | 7 ± 1 |

K562 cells were electroporated with EGFP mRNA and cryopreserved 3 or 24 hours after transfection. For cryopreservation, K562 cells were resuspended in cryotubes (Nunc CryoTube Vials, Nalgene Nunc International, Denmark) at a concentration of 10 x $10^6$ per ml in pure FCS. Next, the suspension was mixed on ice with an equal volume of FCS supplemented with 20% DMSO (Sigma, St. Louis, MO, USA). Cell suspensions were slowly frozen (-1°C/min) to -80°C by using a cryo freezing container (Nalgene Nunc International). Cells were frozen at -80°C for more than 24 hours before use in further experiments. Cells were analyzed at different time points before and after cryopreservation by FCM for EGFP expression to estimate transfection efficiency (= % EGFP+ cells) and the mean fluorescence intensity of $EGFP^+$ cells (= MFI EGFP+ cells). The number of dead cells was determined by ethidium bromide staining (= % dead cells). Results are shown as mean ± standard error.

There was slightly less cell survival in cultures frozen 3 hours after the electroporation as compared to cultures frozen 24 hours after electroporation (p=0.0025). Cells need to recover for a short time after the electroporation. Electroporated mRNA was still functional after cryopreservation. In cultures that had been frozen 3 hours after the electroporation, the MFI of expressed EGFP almost doubled between 3 and 24 hours after thawing (p=0.0009).

Table 5: Transgene expression in cryopreserved mRNA-electroporated DC

| | % EGFP+ cells | MFI EGFP+ cells | % dead cells |
|---|---|---|---|
| Control 1: EGFP expression in mRNA-electroporated iMo-DC (n=3) | | | |
| 24 h after EP | 73 ± 5 | 246 ± 63 | 12 ± 3 |
| 48 h after EP | 59 ± 10 | 218 ± 57 | 24 ± 9 |
| Cryopreservation: iMo-DC cells frozen 18 h after mRNA electroporation (n=4) | | | |
| 6 h after thawing | 61 ± 2 | 215 ± 14 | 26 ± 1 |
| 24 after thawing | 27 ± 1 | 251 ± 24 | 64 ± 2 |

(continued)

| | | | |
|---|---|---|---|
| Control 2: EGFP expression in mRNA-electroporated iMo-DC + maturation (n=3) | | | |
| 24 h after EP<br>48 h after EP | 71 ± 3<br>73 ± 3 | 431 ± 60<br>428 ± 64 | 13 ± 1<br>11 ± 1 |
| | | | |
| Cryopreservation: mMo-DC cells frozen 24 h after mRNA electroporation (n=4) | | | |
| 6 after thawing<br>24 h after thawing | 63 ± 1<br>60 ± 1 | 464 ± 24<br>390 ± 19 | 20 ± 1<br>25 ± 2 |

Immature Mo-DC were electroporated with EGFP mRNA. Cells were cryopreserved as immature DC 18 hours after transfection or as mature DC 24 hours after transfection. Maturation was induced by adding a maturation cocktail (TNF-$\alpha$ + $PGE_2$ + IL-1 + IL-6) directly after transfection. Cells were analyzed by FCM at different time points before and after cryopreservation for EGFP expression, in order to estimate transfection efficiency (= % EGFP+ cells) and the mean fluorescence intensity of EGFP+ cells (= MFI EGFP+ cells). The number of dead cells was determined by ethidium bromide staining (= % dead cells). Results are shown as mean ± standard error. EP, electroporation.

As seen in a non-frozen control of immature and mature DC (Table 5, respectively control 1 and control 2), viability is not significantly affected by this electroporation in function of time (p-value respectively 0.1849 and 0.1362) and cells express high levels of EGFP (Table 5 ; Figure 11 A&B).

Immature DC that were frozen 18 hours after electroporation seemed to survive the freezing cycle well 6 hours after thawing. There was a small increase in cell mortality (+13%, p=0.0008), but the MFI of EGFP expressing cells was approximately the same as in non-frozen control DC (p=0.5185). However, 24 hours after thawing, there was high level of cell mortality in the frozen cultures as compared to non-frozen control DC that have been cultured for 48 hours after electroporation (64% versus 24%, p=0.0017) (Table 2 ; Figure 11 A).

For the cryopreservation of mature DC, immature Mo-DC were electroporated, followed by a 2 hour incubation in medium supplemented with GM-CSF and IL-4, in order to allow transgene expression to start. Following this, the DC maturation cocktail was added and the level of EGFP expression and cell survival was determined 24 and 48 hours after transfection. DC were frozen 24 hours after mRNA electroporation and transgene expression and cell survival was determined 6 and 24 hours after thawing (Table 2 ; Figure 1 B). Six hours after thawing, DC cultures appeared to survive the freezing and have a similar number of EGFP+ cells and MFI level of EGFP+ cells as compared to non-frozen cultures (p-value respectively 0.0033 and 0.5183). Mature DC survived the thawing procedure better than frozen immature DC (64% cell death for immature DC versus 25% for mature DC after 24 hours of culture, p=0.00004).

## Claims

**1.** A method for transfection of hematopoietic cells or stem cells not including human embryonic stem cells with one or more or a mixture of naked linear RNAs, which method comprises electroporation of a suspension containing the hematopoietic cells or stem cells and the naked linear RNAs to be transfected at a capacitance of 100 to 250 μF, at a voltage from 200 to 350 V and a pulsing time from 1 to 40 ms, wherein the concentration of the cells in the suspension is 100 to $1\times10^9$ cells per ml.

**2.** The method of claim 1, wherein the pulse is an exponential decay pulse.

**3.** The method of claim 1 or 2, wherein the electroporation is performed at a voltage from 250 to 300 V.

**4.** The method according to any one of claims 1 to 3, wherein the capacitance is 150 to 250 μF.

**5.** The method according to any one of claims 1 to 4, wherein the pulsing time is from 2.5 to 25 ms, and preferably from 7 to 10 ms.

**6.** The method according to any one of claims 1 to 5, wherein the naked linear RNAs to be transfected include modified or unmodified, defined or undefined RNA.

**7.** The method of claim 6, wherein the naked linear RNAs to be transfected is mRNA.

**8.** The method according to any one of claims 1 to 6, wherein

(i) the concentration of the cells in the suspension is $1\times10^5$ to $5\times10^7$ cells per ml, preferably 1 to $4\times10^7$ cells per ml; and/or
(ii) the concentration of the naked linear RNAs to be transfected is $1\times10^{-7}$ to $1\times10^{-5}$ mmol/ml, preferably $4\times10^{-6}$ to $6\times10^{-6}$ mmol/ml.

**9.** The method according to any one of claims 1 to 8, wherein

(i) the hematopoietic cells or stem cells are mammalian cells, preferably human cells not including human embryonic stem cells; and/or
(ii) the stem cells include derivatives of such stem cells and are preferably embryonic stem cells not including human embryonic stem cells, and the hematopoietic cells include mononuclear cells, hematopoietic stem cells and derivatives thereof, marrow $CD34^+$ progenitor derived dendritic cells, $CD34^+$ progenitor derived Langerhans cells, monocyte-derived dendritic cells (Mo-DC), and most preferably are Mo-DC including immature Mo-DC and mature Mo-DC.

**10.** The method of claim 1 or 2, wherein the hematopoietic cells are Mo-DC, the naked linear polynucleotides are mRNA and the transfection is performed at a low voltage, low capacitance and a high cell concentration, preferably a voltage of 250 to 300 V, a capacitance of 150 to 250 μF and a cell concentration of $1\times10^7$ to $4\times10^7$ cells/ml, at a pulse time of 7 to 10 ms.

**11.** The method according to any one of claims 1 to 6 and 10, wherein the naked linear RNAs

(i) encode proteins or peptides to be expressed in the eukaryotic cells, said proteins or peptides may or may not have a function in the eukaryotic cells, preferably the linear polynucleotides encode tumor antigens, microbial antigens, viral antigens, immunostimulatory or tolerogenic molecules, anti-apoptotic molecules, adhesion and homing molecules and antigen processing molecules;
(ii) are functional or regulatory sequences including, but not limited to, differentiation-regulating genes, differentiation-associated genes and tissue-specific genes.

**12.** The method according to any one of claims 1 to 11, wherein the hematopoietic cells are human mature Mo-DC and/or wherein the linear RNAs to be transfected is mRNA, and wherein the method may further comprise further maturation of the transfected MoDC by providing a maturation stimulus.

**13.** The method according to any one of claims 1 to 11, wherein the hematopoietic cells are human immature Mo-DC and/or wherein the naked linear RNAs to be transfected is mRNA, and wherein the method may further comprise maturation of the transfected cells by providing a maturation structure.

**14.** The method according to claim 12 or 13, wherein the maturation structures comprise one or more of the compounds selected from the group IL-1ß, IL-6, TNF-$\alpha$, $PGE_2$, lipopolysaccharide, immunostimulatory DNA sequences, CD40 ligand and poly-I:C, and preferably is a mixture comprising It-1ß, IL-6, TNF-$\alpha$ and $PGE_2$.

**15.** The method of claims 1 to 14, wherein the method further comprises cryoconservation of the transfected cells.

**Patentansprüche**

**1.** Verfahren zur Transfektion von hämatopoetischen Zellen oder Stammzellen außer humanen embryonalen Stammzellen mit einer oder mehreren oder einem Gemisch von nackten linearen RNAs, wobei das Verfahren eine Elektroporation einer Suspension, die die hämatopoetischen Zellen oder Stammzellen und die mit der Transfektion einzuführenden nackten linearen RNAs enthält, mit einer Kapazität von 100 bis 250 $\mu$F, einer Spannung von 200 bis 350 V und einer Pulszeit von 1 bis 40 ms umfasst, wobei die Konzentration der Zellen in der Suspension 100 bis 1 x $10^9$ Zellen pro ml beträgt.

**2.** Verfahren gemäß Anspruch 1, wobei der Puls ein exponentiell abfallender Puls ist.

**3.** Verfahren gemäß Anspruch 1 oder 2, wobei die Elektroporation bei einer Spannung von 250 bis 300 V durchgeführt wird.

**4.** Verfahren gemäß einem der Ansprüche 1 bis 3, wobei die Kapazität 150 bis 250 $\mu$F beträgt.

**5.** Verfahren gemäß einem der Ansprüche 1 bis 4, wobei die Pulszeit 2,5 bis 25 ms und vorzugsweise 7 bis 10 ms beträgt.

**6.** Verfahren gemäß einem der Ansprüche 1 bis 5, wobei die mit der Transfektion einzuführenden nackten linearen RNAs modifizierte, definierte oder undefinierte RNA umfassen.

**7.** Verfahren gemäß Anspruch 6, wobei es sich bei den mit der Transfektion einzuführenden nackten linearen RNAs um mRNA handelt.

**8.** Verfahren gemäß einem der Ansprüche 1 bis 6, wobei

(i) die Konzentration der Zellen in der Suspension 1 x $10^5$ bis 5 x $10^7$ Zellen pro ml, vorzugsweise 1 bis 4 x $10^7$ Zellen pro ml beträgt; und/oder
(ii) die Konzentration der mit der Transfektion einzuführenden nackten linearen RNAs 1 x $10^{-7}$ bis 1 x $10^{-5}$ mmol/ml, vorzugsweise 4 x $10^{-6}$ bis 6 x $10^{-6}$ mmol/ml, beträgt.

**9.** Verfahren gemäß einem der Ansprüche 1 bis 8, wobei

(i) die hämatopoetischen Zellen oder Stammzellen Säugerzellen, vorzugsweise humane Zellen, außer humanen embryonalen Stammzellen sind; und/oder
(ii) die Stammzellen Derivate solcher Stammzellen umfassen und vorzugsweise embryonale Stammzellen außer humanen embryonalen Stammzellen sind und die hämatopoetischen Zellen mononukleäre Zellen, hämatopoetische Stammzellen und Derivate davon, von CD34-positiven Vorläuferzellen des Knochenmarks abgeleitete dendritische Zellen, von CD34-positiven Vorläuferzellen abgeleitete Langerhans-Zellen, von Monocyten abgeleitete dendritische Zellen (Mo-DC) umfassen und am meisten bevorzugt Mo-DC einschließlich unreifer Mo-DC und reifer Mo-DC sind.

**10.** Verfahren gemäß Anspruch 1 oder 2, wobei die hämatopoetischen Zellen Mo-DC sind, die nackten linearen Polynucleotide mRNA sind und die Transfektion bei einer niedrigen Spannung, niedrigen Kapazität und hohen Zellkonzentration, vorzugsweise einer Spannung von 250 bis 300 V, einer Kapazität von 150 bis 250 $\mu$F und einer Zellkonzentration von 1 x $10^7$ bis 4 x $10^7$ Zellen/ml, mit einer Pulszeit von 7 bis 10 ms durchgeführt wird.

**11.** Verfahren gemäß einem der Ansprüche 1 bis 6 und 10, wobei die nackten linearen RNAs

(i) Proteine oder Peptide codieren, die in den eukaryontischen Zellen exprimiert werden sollen, wobei die Proteine oder Peptide eine Funktion in den eukaryontischen Zellen aufweisen können oder auch nicht, wobei die linearen Polynucleotide vorzugsweise Tumorantigene, mikrobielle Antigene, virale Antigene, immunstimulatorische oder tolerogene Moleküle, antiapoptotische Moleküle, Adhäsionsmoleküle, Homing-Rezeptoren und antigenprozessierende Moleküle codieren;
(ii) funktionelle oder regulatorische Sequenzen sind, einschließlich unter Anderem differenzierungsregulierender Gene, differenzierungsassoziierter Gene und gewebespezifischer Gene.

**12.** Verfahren gemäß einem der Ansprüche 1 bis 11, wobei die hämatopoetischen Zellen humane reife Mo-DC sind und/oder wobei es sich bei den mit der Transfektion einzuführenden linearen RNAs um mRNA handelt und wobei das Verfahren weiterhin eine weitere Reifung der mit der Transfektion eingeführten MoDC durch Bereitstellen eines Reifungsreizes umfassen kann.

**13.** Verfahren gemäß einem der Ansprüche 1 bis 11, wobei die hämatopoetischen Zellen humane unreife Mo-DC sind und/oder wobei es sich bei den mit der Transfektion einzuführenden nackten linearen RNAs um mRNA handelt und wobei das Verfahren weiterhin eine Reifung der transfizierten Zellen durch Bereitstellen einer Reifungsstruktur umfassen kann.

**14.** Verfahren gemäß Anspruch 12 oder 13, wobei die Reifungsstrukturen eine oder mehrere der Verbindungen umfassen, die aus der Gruppe IL-1$\beta$, IL-6, TNF-$\alpha$, $PGE_2$, Lipopolysaccharid, immunstimulatorische DNA-Sequenzen, CD40-Ligand und Poly-I:C ausgewählt sind und vorzugsweise ein Gemisch sind, das IL-1$\beta$, IL-6, TNF-$\alpha$ und $PGE_2$ umfasst.

**15.** Verfahren gemäß Anspruch 1 bis 14, wobei das Verfahren weiterhin die Kryokonservierung der transfizierten Zellen umfasst.

## Revendications

**1.** Procédé de transfection de cellules hématopoïétiques ou de cellules souches ne comprenant pas de cellules souches embryonnaires humaines avec un ou plusieurs ou un mélange d'ARN nus linéaires, lequel procédé comprend l'électroporation d'une suspension contenant les cellules hématopoïétiques ou les cellules souches et les ARN nus linéaires à transfecter à une capacité électrique de 100 à 250 $\mu$F, une tension de 200 à 350 V et un temps d'impulsion de 1 à 40 ms, la concentration des cellules dans la suspension étant de 100 à 1 x $10^9$ cellules par ml.

**2.** Procédé selon la revendication 1, dans lequel l'impulsion est une impulsion à décroissance exponentielle.

**3.** Procédé selon la revendication 1 ou 2, dans lequel l'électroporation est réalisée à une tension de 250 à 300 V.

**4.** Procédé selon l'une quelconque des revendications 1 à 3, dans lequel la capacité électrique est de 150 à 250 $\mu$F.

**5.** Procédé selon l'une quelconque des revendications 1 à 4, dans lequel le temps d'impulsion est de 2,5 à 25 ms, et de préférence de 7 à 10 ms.

**6.** Procédé selon l'une quelconque des revendications 1 à 5, dans lequel les ARN nus linéaires à transfecter comprennent de l'ARN modifié ou non modifié, défini ou indéfini.

**7.** Procédé selon la revendication 6, dans lequel les ARN nus linéaires à transfecter sont de l'ARNm.

**8.** Procédé selon l'une quelconque des revendications 1 à 6, dans lequel

(i) la concentration des cellules dans la suspension est de 1 x $10^5$ à 5 x $10^7$ cellules par ml, de préférence de 1 à 4 x $10^7$ cellules par ml ; et/ou
(ii) la concentration des ARN nus linéaires à transfecter est de 1 x $10^{-7}$ à 1 x $10^{-5}$ mmoles/ml, de préférence de 4 x $10^{-6}$ à 6 x $10^{-6}$ mmoles/ml.

**9.** Procédé selon l'une quelconque des revendications 1 à 8, dans lequel

(i) les cellules hématopoïétiques ou les cellules souches sont des cellules de mammifères, de préférence des cellules humaines ne comprenant pas de cellules souches embryonnaires humaines ; et/ou

(ii) les cellules souches comprennent des dérivés de telles cellules souches et sont de préférence des cellules souches embryonnaires ne comprenant pas de cellules souches embryonnaires humaines, et les cellules hématopoïétiques comprennent les cellules mononucléaires, les cellules souches hématopoïétiques et leurs dérivés, les cellules dendritiques dérivées du progéniteur $CD34^+$ de la moelle, les cellules de Langerhans dérivées du progéniteur $CD34^+$, les cellules dendritiques dérivées de monocytes (Mo-DC) et, de manière préférée entre toutes, sont des Mo-DC comprenant des Mo-DC immatures et des Mo-DC mûres.

**10.** Procédé selon la revendication 1 ou 2, dans lequel les cellules hématopoïétiques sont des Mo-DC, les polynucléotides nus linéaires sont de l'ARNm et la transfection est réalisée à basse tension, à faible capacité électrique et à forte concentration cellulaire, de préférence à une tension de 250 à 300 V, à une capacité électrique de 150 à 250 $\mu$F et à une concentration de cellules de 1 x $10^7$ à 4 x $10^7$ cellules/ml, à un temps d'impulsion de 7 à 10 ms.

**11.** Procédé selon l'une quelconque des revendications 1 à 6 et 10, dans lequel les ARN nus linéaires

(i) codent pour des protéines ou des peptides à exprimer dans les cellules eucaryotes, lesdites protéines ou lesdits peptides pouvant ou pouvant ne pas avoir de fonction dans les cellules eucaryotes, de préférence les polynucléotides linéaires codent pour des antigènes tumoraux, des antigènes microbiens, des antigènes viraux, des molécules immunostimulantes ou tolérogènes, des molécules anti-apoptotiques, des molécules d'adhésion et de guidage et des molécules d'apprêtement antigénique ;
(ii) sont des séquences fonctionnelles ou régulatrices comprenant, mais sans s'y limiter, les gènes de régulation de la différenciation, les gènes associés à la différenciation et les gènes spécifiques des tissus.

**12.** Procédé selon l'une quelconque des revendications 1 à 11, dans lequel les cellules hématopoïétiques sont des Mo-DC humaines mûres et/ou dans lequel les ARN linéaires à transfecter sont de l'ARNm, et le procédé pouvant en outre comprendre une maturation supplémentaire des MoDC transfectées par la fourniture d'un stimulus de maturation.

**13.** Procédé selon l'une quelconque des revendications 1 à 11, dans lequel les cellules hématopoïétiques sont des Mo-DC humaines immatures et/ou dans lequel les ARN nus linéaires à transfecter sont de l'ARNm, et le procédé pouvant en outre comprendre une maturation des cellules transfectées par la fourniture d'une structure de maturation.

**14.** Procédé selon la revendication 12 ou 13, dans lequel les structures de maturation comprennent un ou plusieurs des composés choisis dans le groupe IL-1$\beta$, IL-6, TNF-$\alpha$, $PGE_2$, lipopolysaccharide, séquences d'ADN immunostimulantes, ligand du CD40 et poly-I:C, et est de préférence un mélange comprenant IL-1$\beta$, IL-6, TNF-$\alpha$ et $PGE_2$.

**15.** Procédé selon les revendications 1 à 14, le procédé comprenant en outre la cryoconservation des cellules transfectées.

**Fig. 1A**

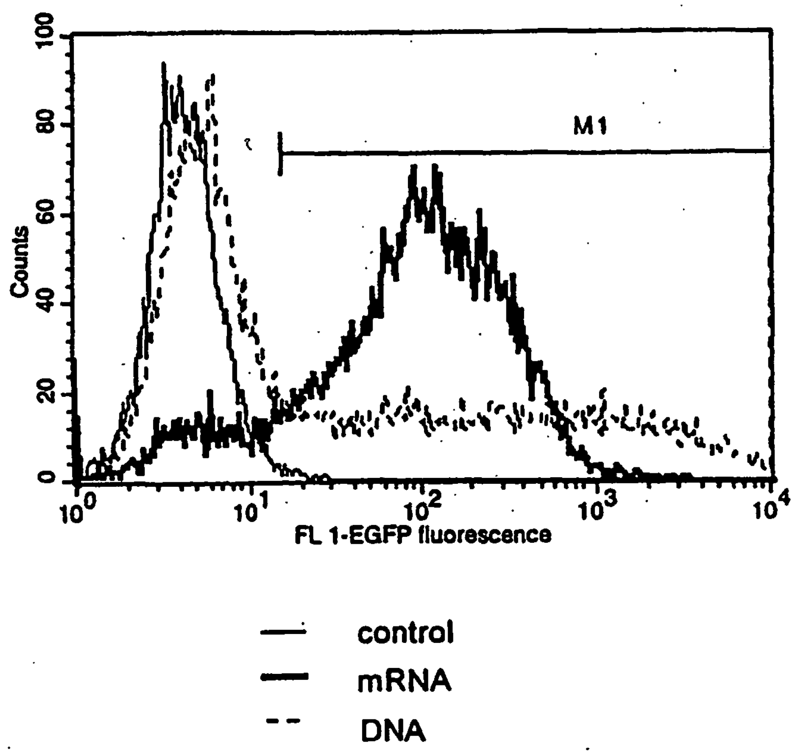
Counts
0
20
40
60
80
100
M1
$10^0$
$10^1$
$10^2$
$10^3$
$10^4$
FL 1-EGFP fluorescence
control
mRNA
DNA

**Fig. 1B**

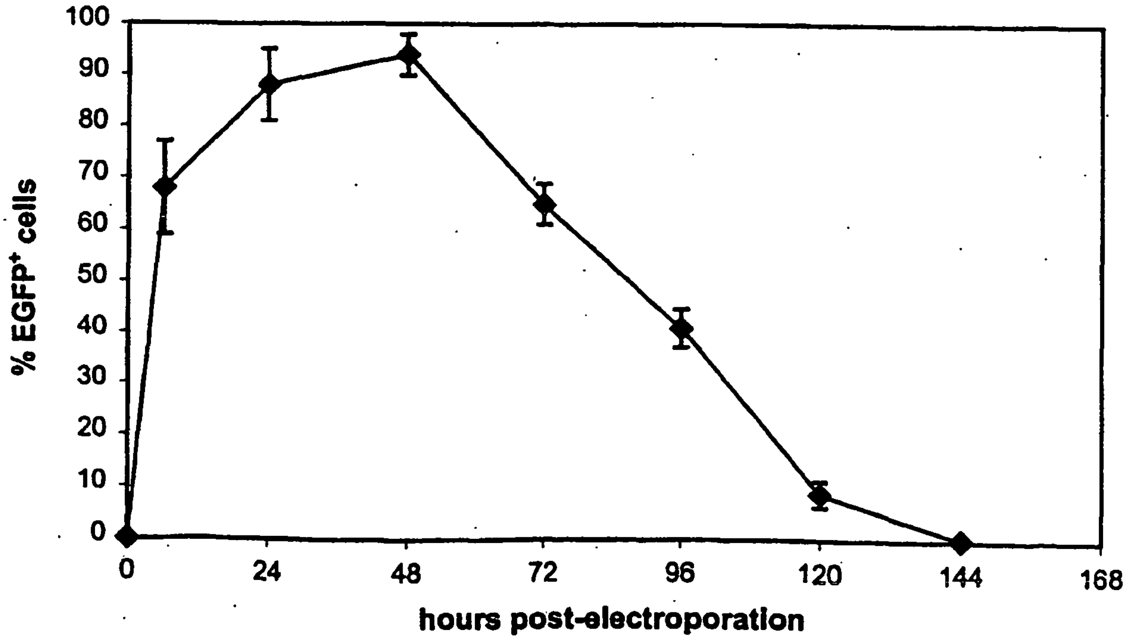
% EGFP+ cells
0
10
20
30
40
50
60
70
80
90
100
0
24
48
72
96
120
144
168
hours post-electroporation

Fig. 2A

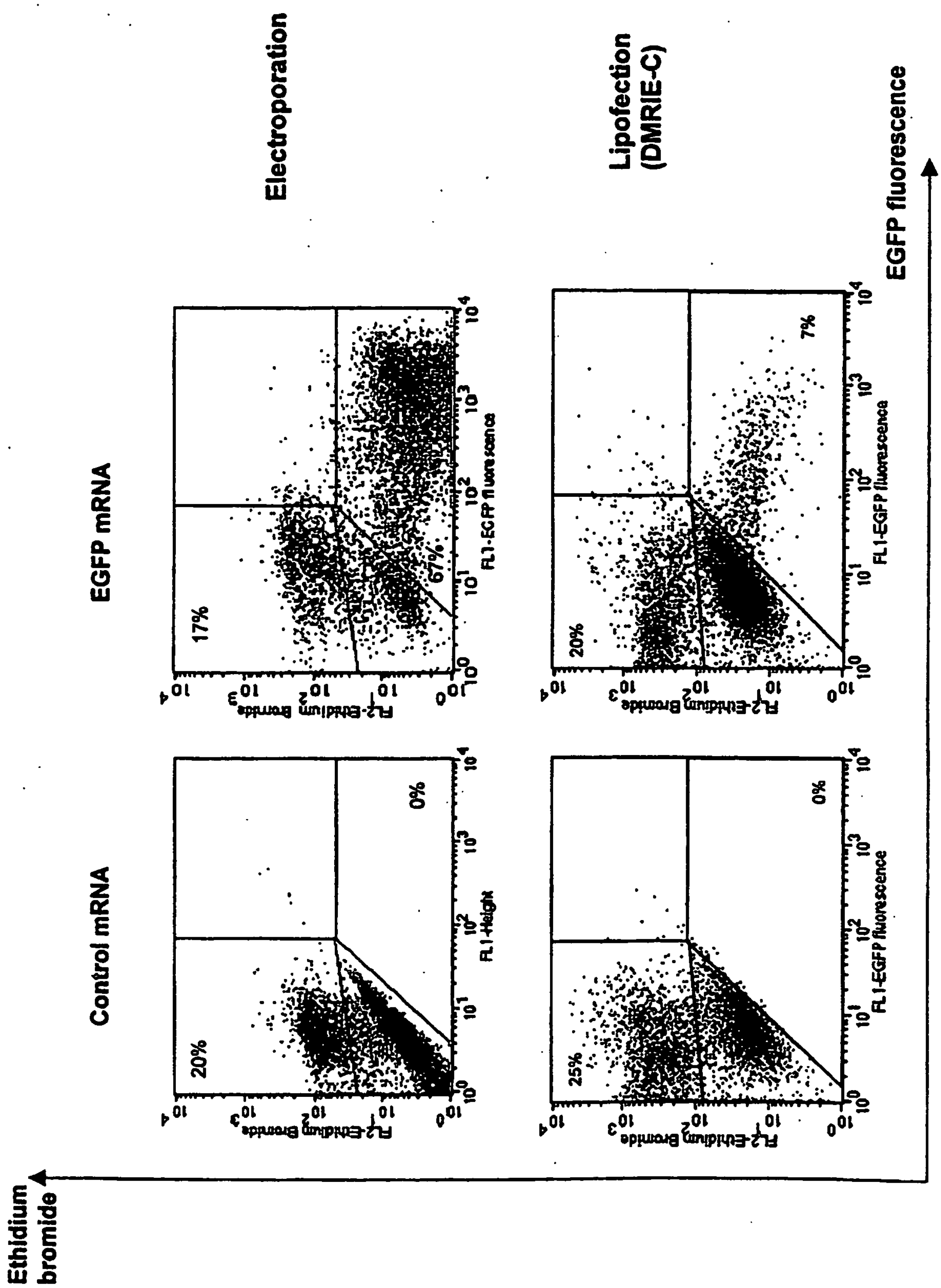
Ethidium bromide
Control mRNA
EGFP mRNA
Electroporation
Lipofection (DMRIE-C)
EGFP fluorescence
20%
0%
17%
67%
25%
0%
20%
7%
FL2-Ethidium Bromide
FL1-Height
FL1-EGFP fluorescence

**Fig. 2B**

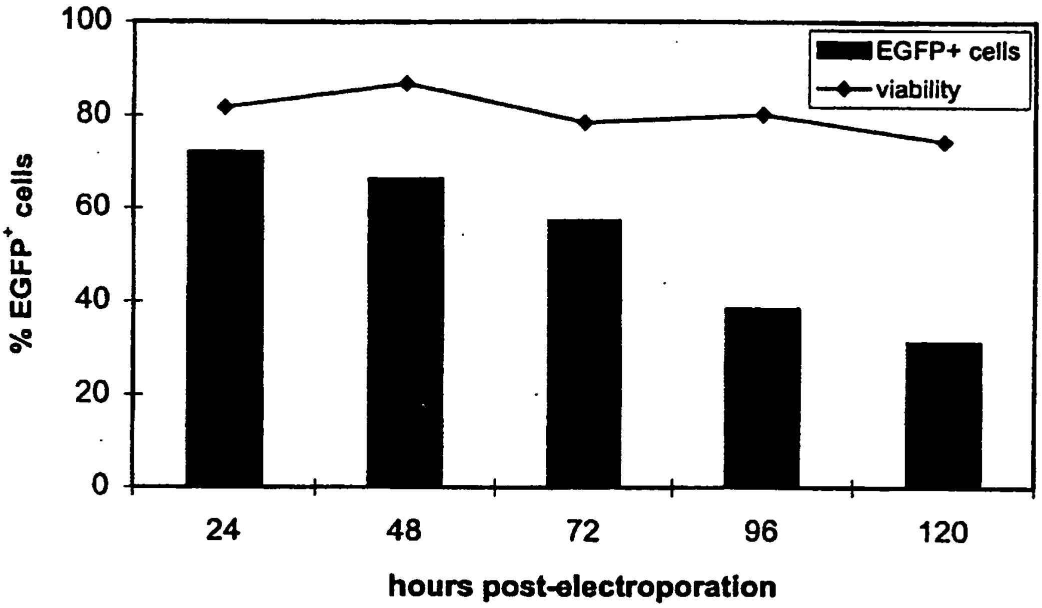
100
80
60
40
20
0
% EGFP+ cells
EGFP+ cells
viability
24
48
72
96
120
hours post-electroporation

Fig. 2C

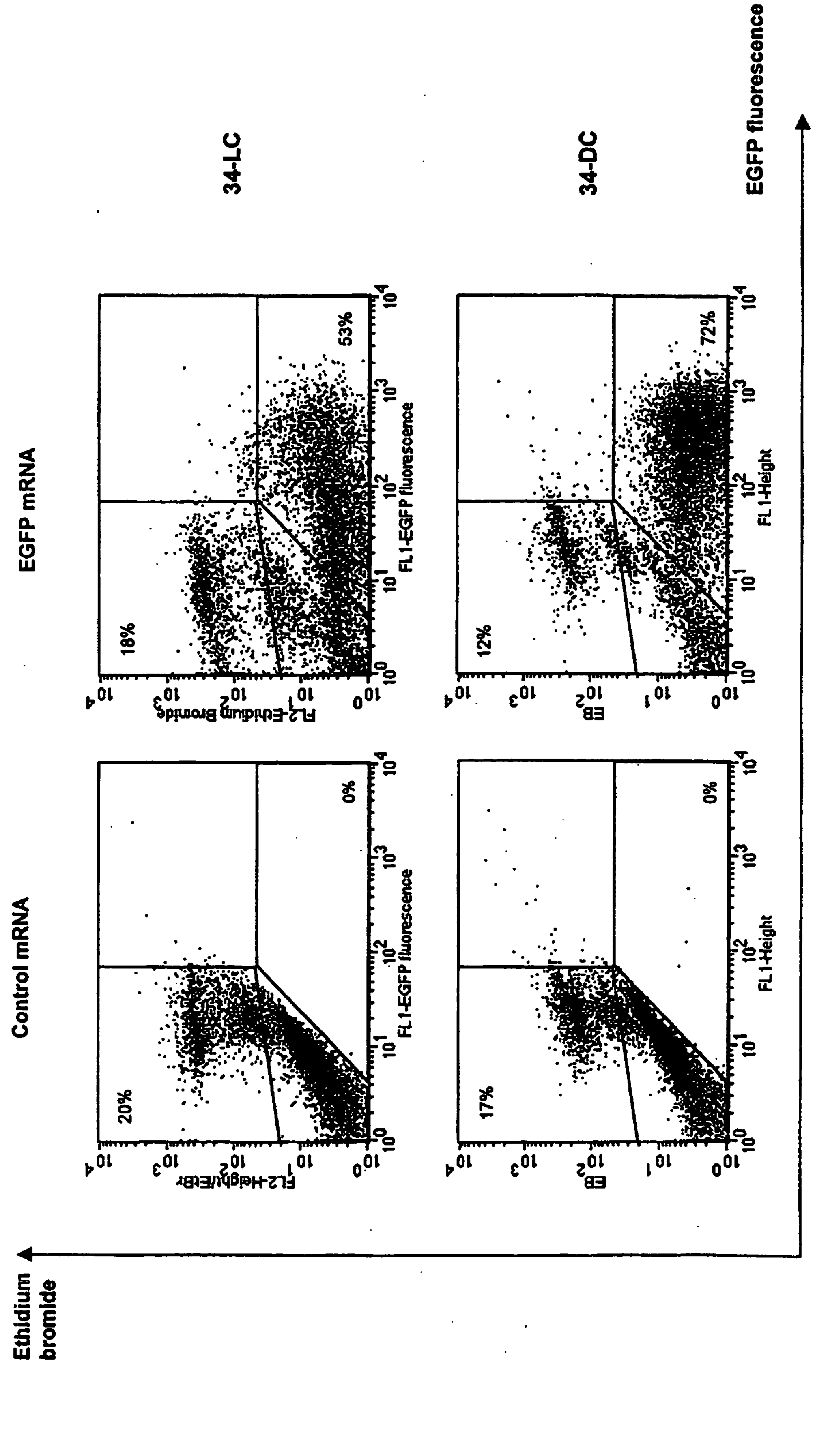
Control mRNA
EGFP mRNA
34-LC
34-DC
Ethidium bromide
EGFP fluorescence
20%
0%
18%
53%
17%
0%
12%
72%
FL2-Height/EtBr
FL1-EGFP fluorescence
FL2-Ethidium Bromide
EB
FL1-Height

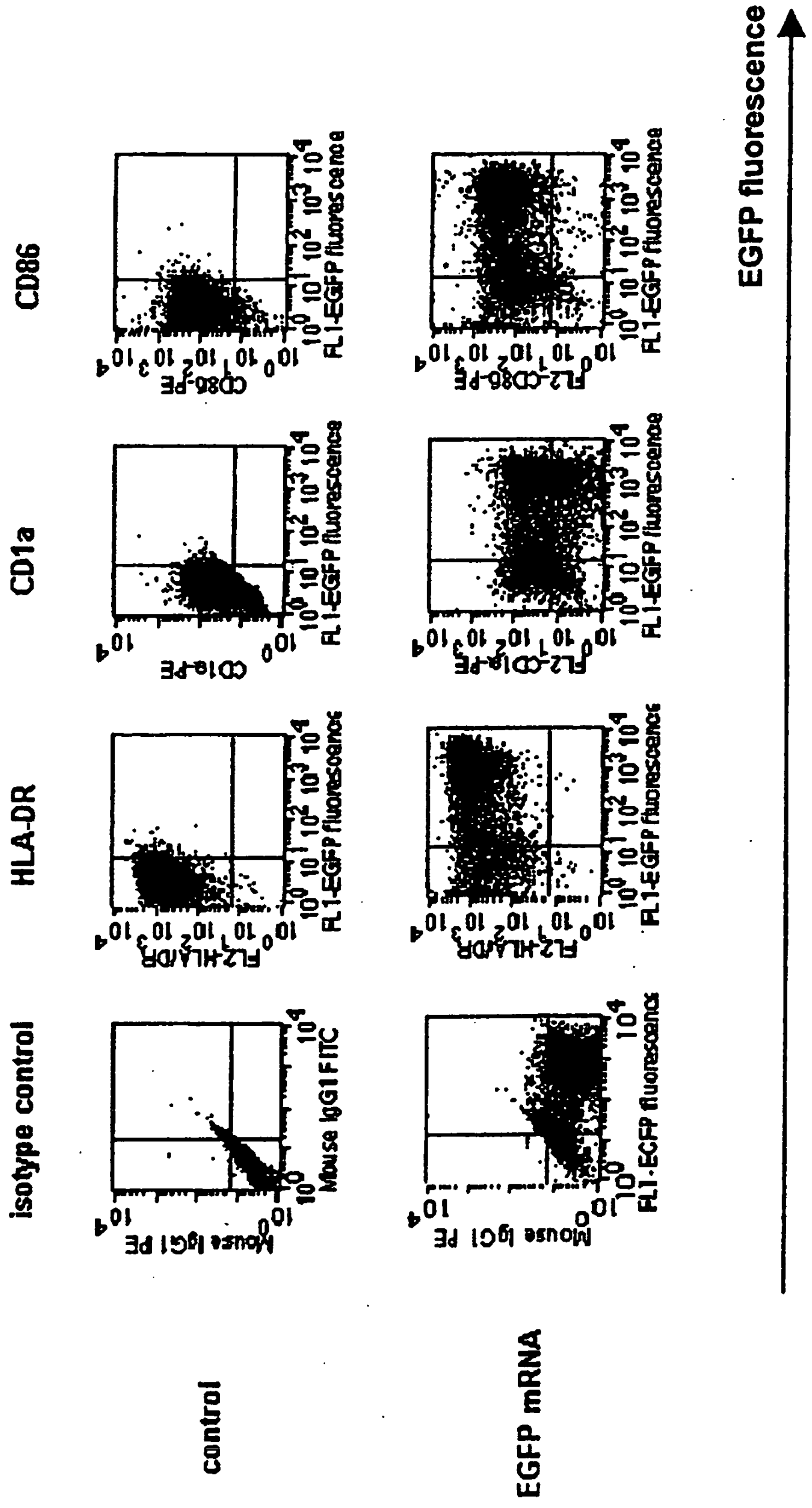
isotype control
HLA-DR
CD1a
CD86
control
EGFP mRNA
Mouse IgG1 PE
Mouse IgG1 FITC
FL1-EGFP fluorescence
FL2-HLA/DR
CD1a-PE
FL2-CD1a-PE
CD86-PE
FL2-CD86-PE
EGFP fluorescence

Fig. 3A

Fig. 3B

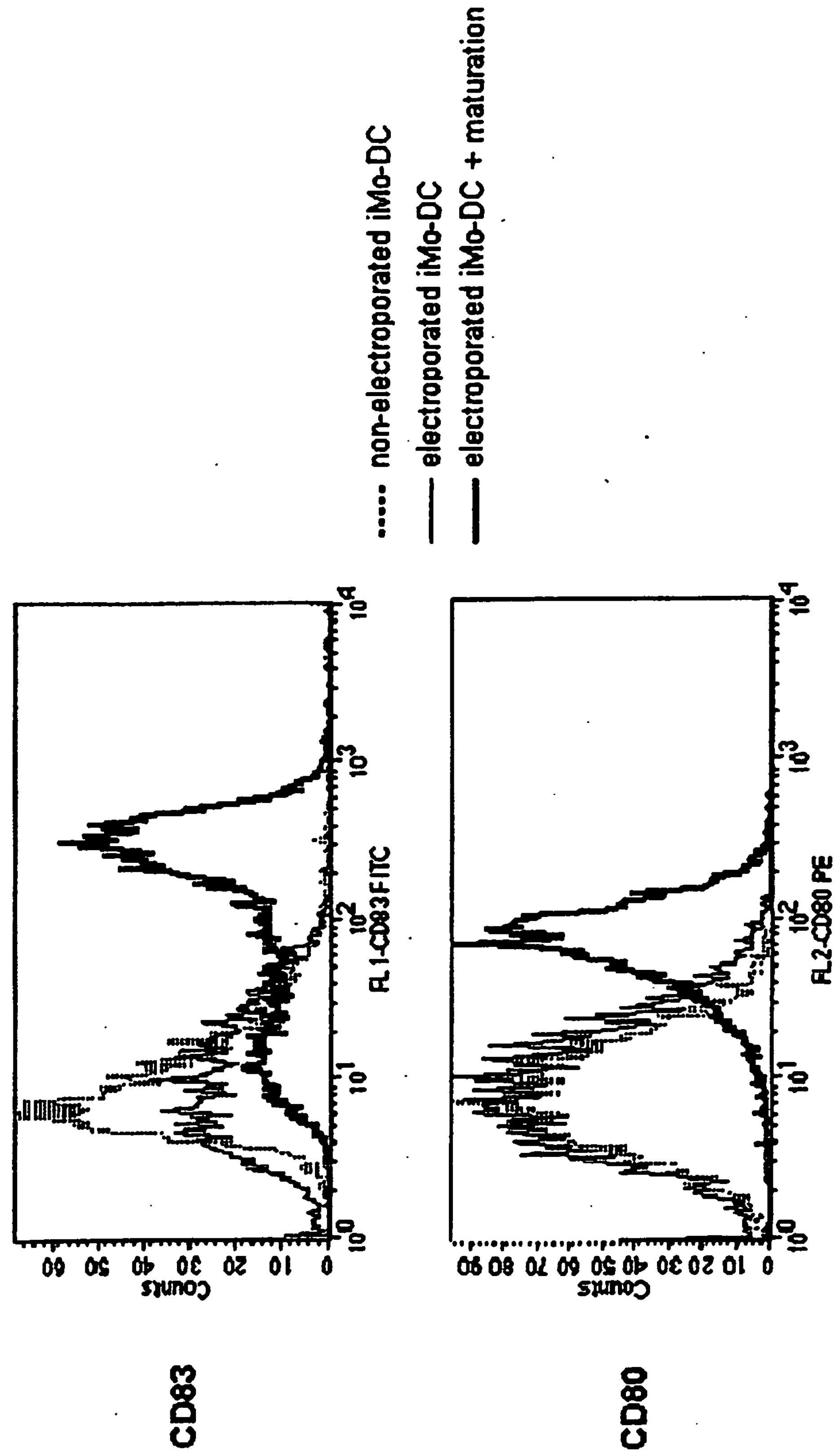

CD83
CD80
Counts
FL1-CD83 FITC
FL2-CD80 PE
non-electroporated iMo-DC
electroporated iMo-DC
electroporated iMo-DC + maturation

Fig. 3C

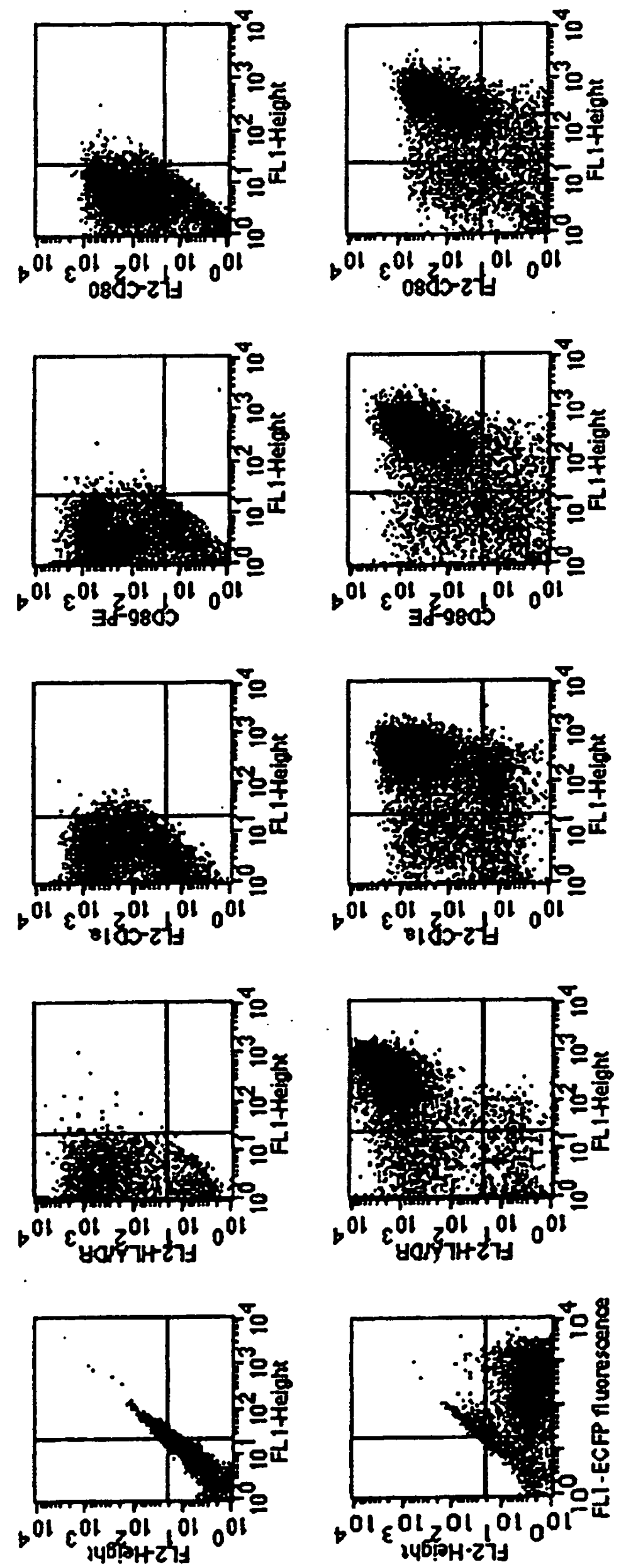
FL2-CD80
FL1-Height
CD86-PE
FL2-CD1a
FL2-HLA/DR
FL2-Height
FL1-ECFP fluorescence

Fig. 4

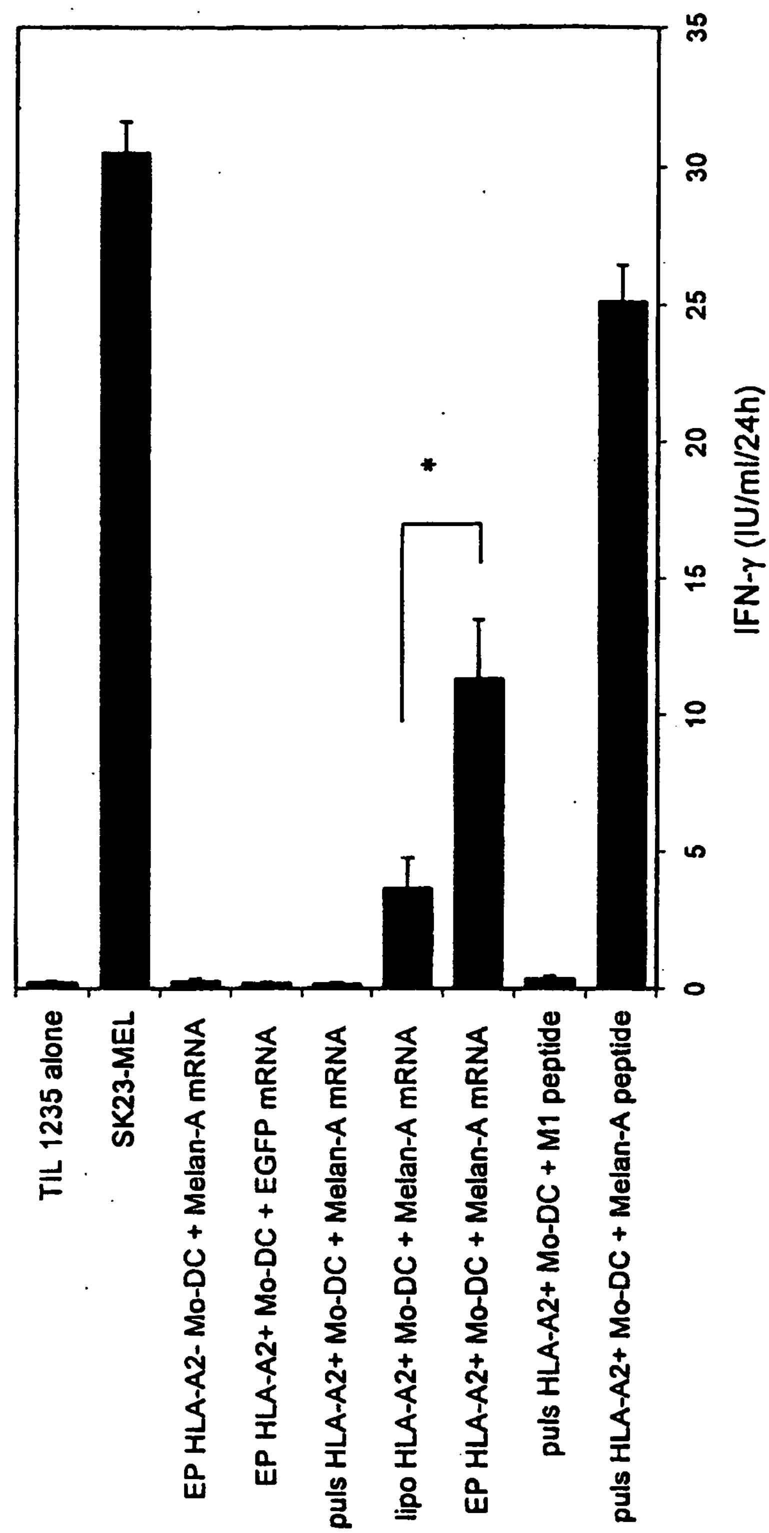
TIL 1235 alone
SK23-MEL
EP HLA-A2- Mo-DC + Melan-A mRNA
EP HLA-A2+ Mo-DC + EGFP mRNA
puls HLA-A2+ Mo-DC + Melan-A mRNA
lipo HLA-A2+ Mo-DC + Melan-A mRNA
EP HLA-A2+ Mo-DC + Melan-A mRNA
puls HLA-A2+ Mo-DC + M1 peptide
puls HLA-A2+ Mo-DC + Melan-A peptide
*
0
5
10
15
20
25
30
35
IFN-γ (IU/ml/24h)

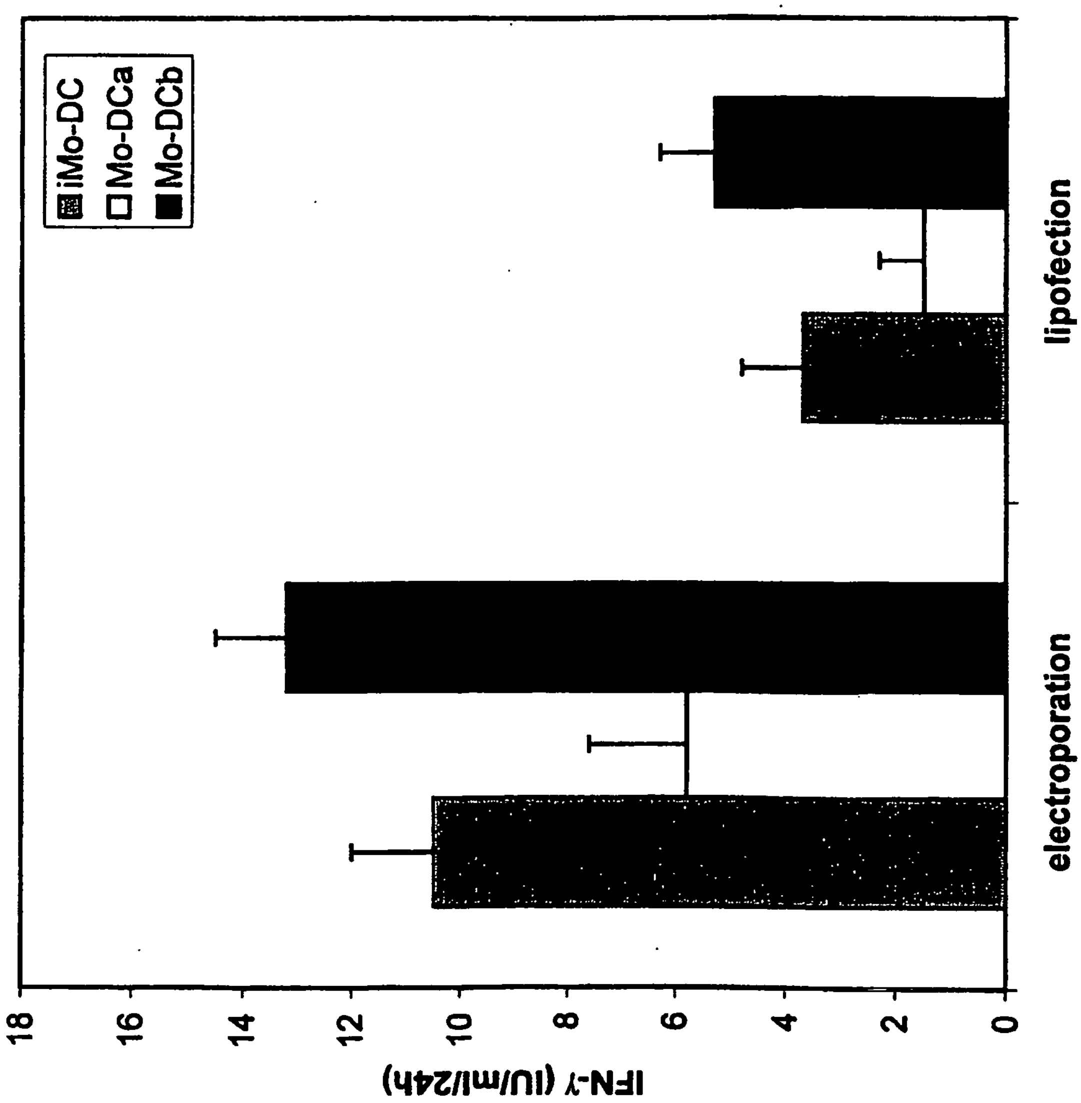
iMo-DC
Mo-DCa
Mo-DCb
IFN-γ (IU/ml/24h)
0
2
4
6
8
10
12
14
16
18
electroporation
lipofection

Fig. 5

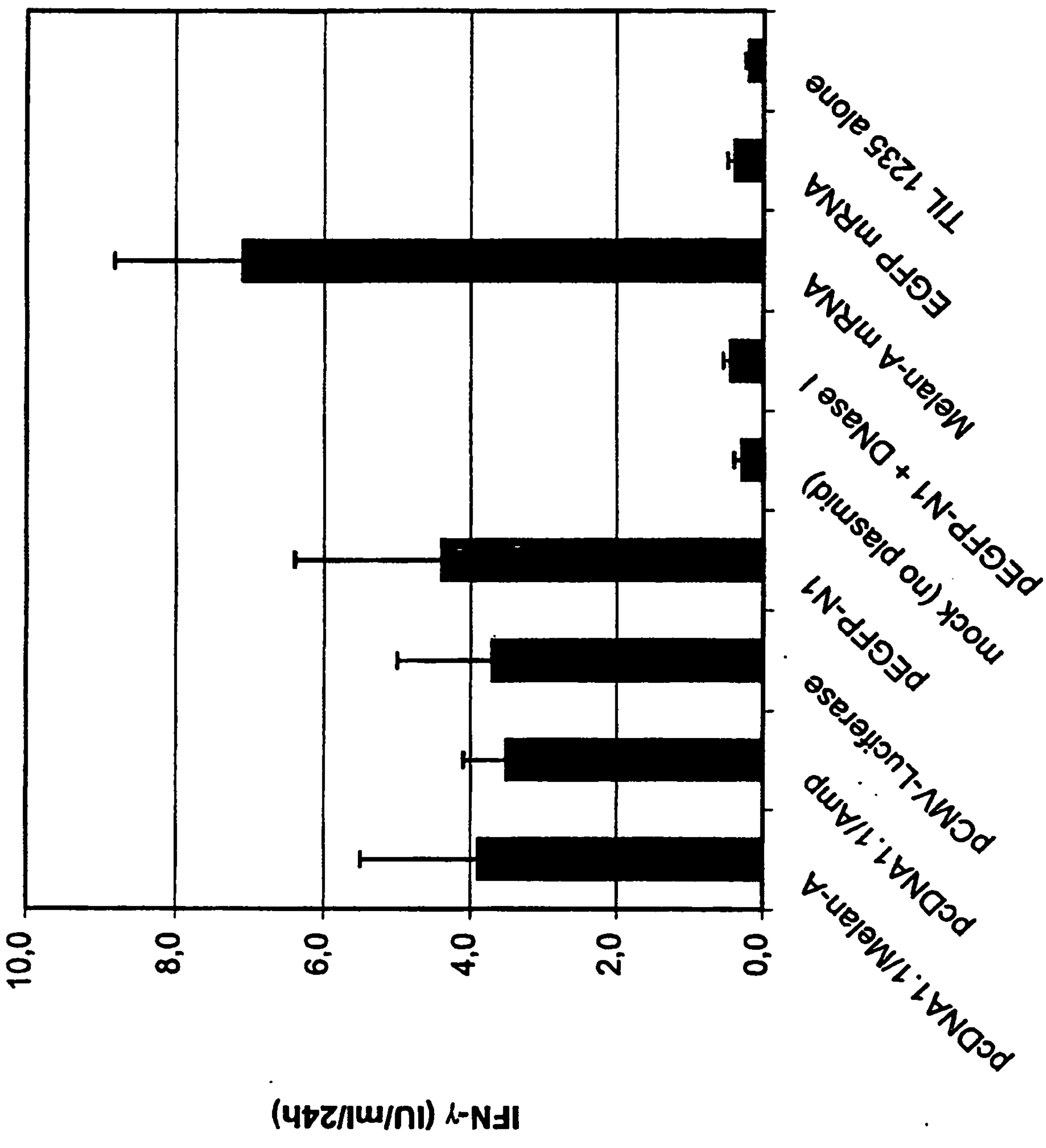
IFN-γ (IU/ml/24h)
10,0
8,0
6,0
4,0
2,0
0,0
pcDNA1.1/Melan-A
pcDNA1.1/Amp
pCMV-Luciferase
pEGFP-N1
mock (no plasmid)
pEGFP-N1 + DNase I
Melan-A mRNA
EGFP mRNA
TIL 1235 alone

Fig. 6

Fig. 7

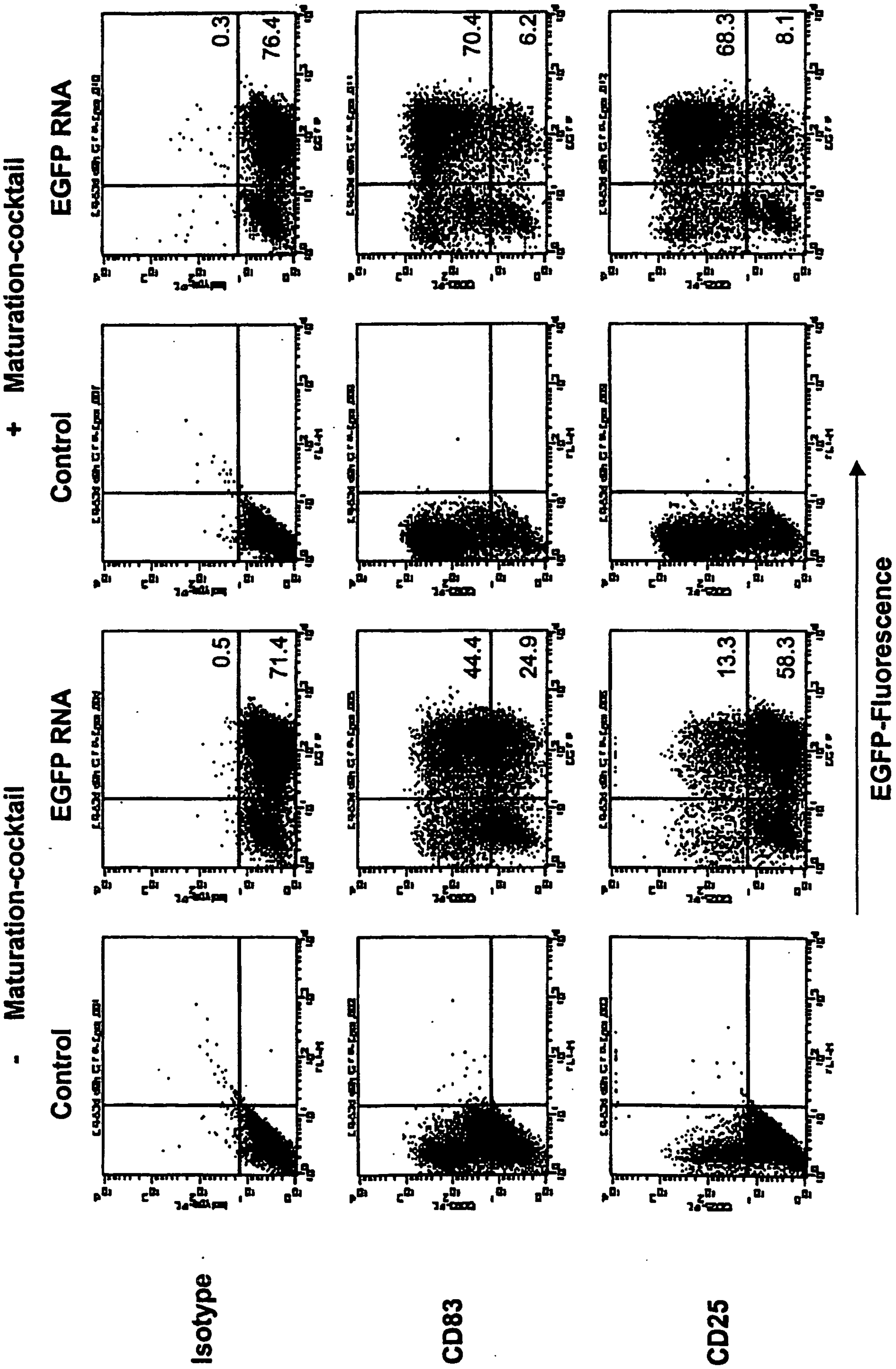
- Maturation-cocktail
+ Maturation-cocktail
Control
EGFP RNA
Control
EGFP RNA
Isotype
CD83
CD25
0.5
71.4
44.4
24.9
13.3
58.3
0.3
76.4
70.4
6.2
68.3
8.1
EGFP-Fluorescence

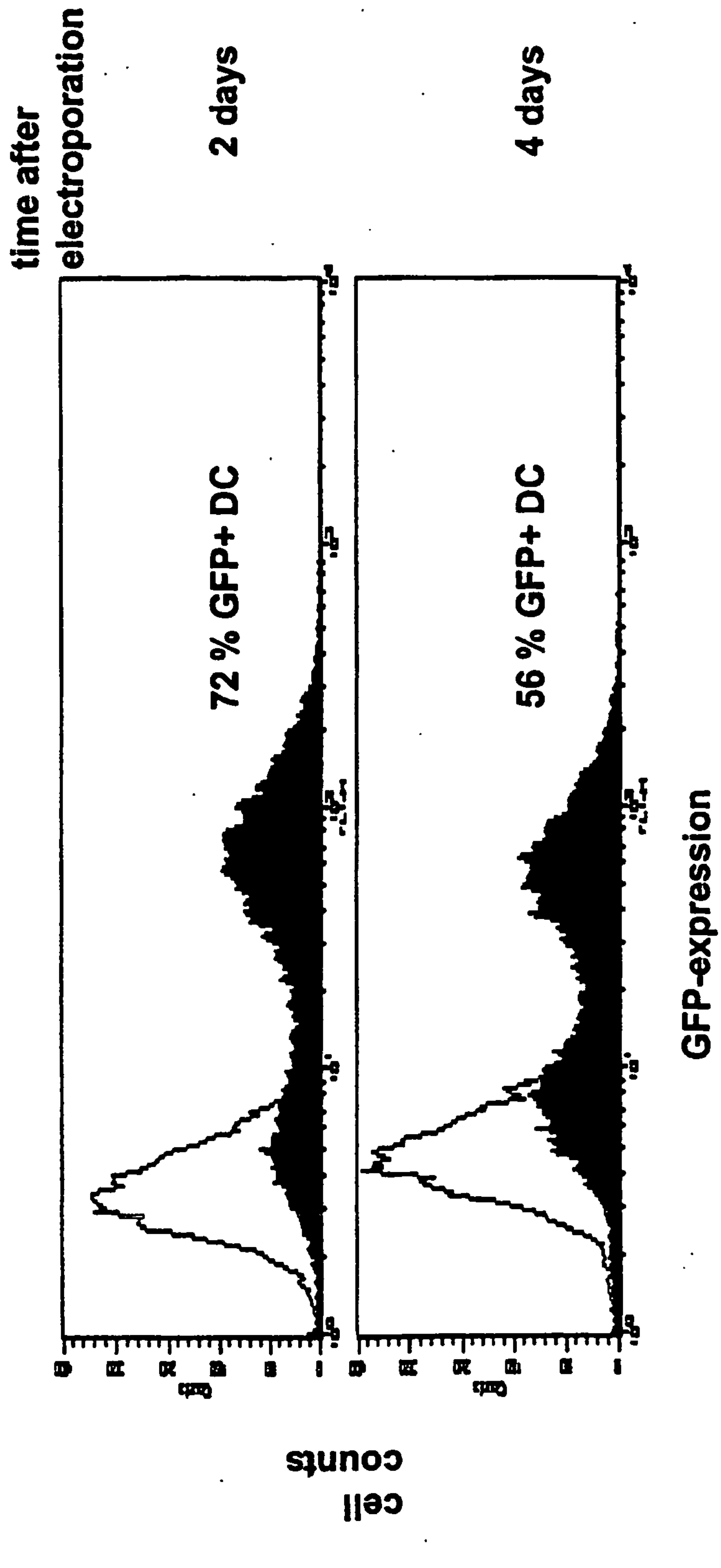
time after electroporation
2 days
4 days
72 % GFP+ DC
56 % GFP+ DC
cell counts
GFP-expression

Fig. 8

Fig. 9A

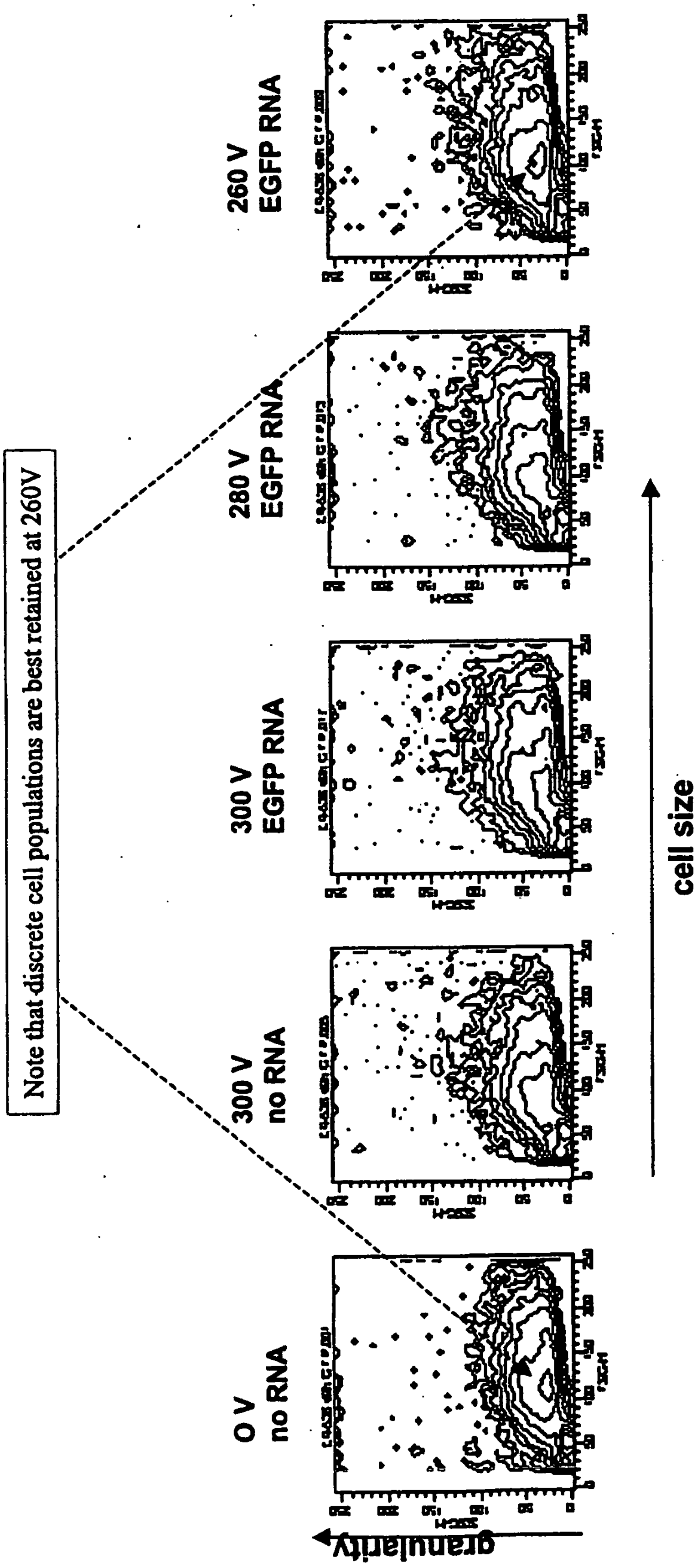
Note that discrete cell populations are best retained at 260V
0 V
no RNA
300 V
no RNA
300 V
EGFP RNA
280 V
EGFP RNA
260 V
EGFP RNA
cell size
granularity

Fig. 9B

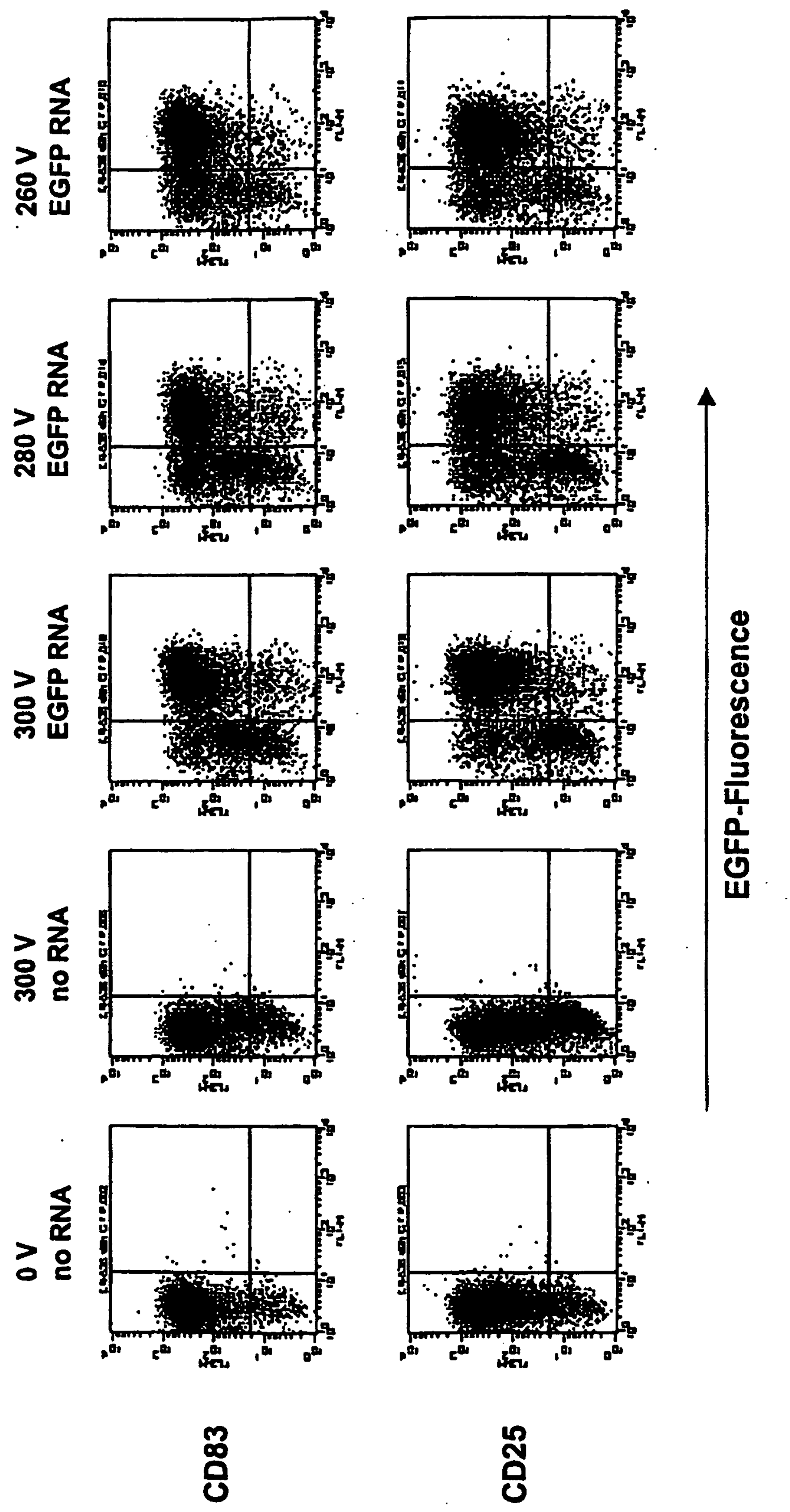
0 V
no RNA
300 V
no RNA
300 V
EGFP RNA
280 V
EGFP RNA
260 V
EGFP RNA
CD83
CD25
EGFP-Fluorescence

Fig. 10A

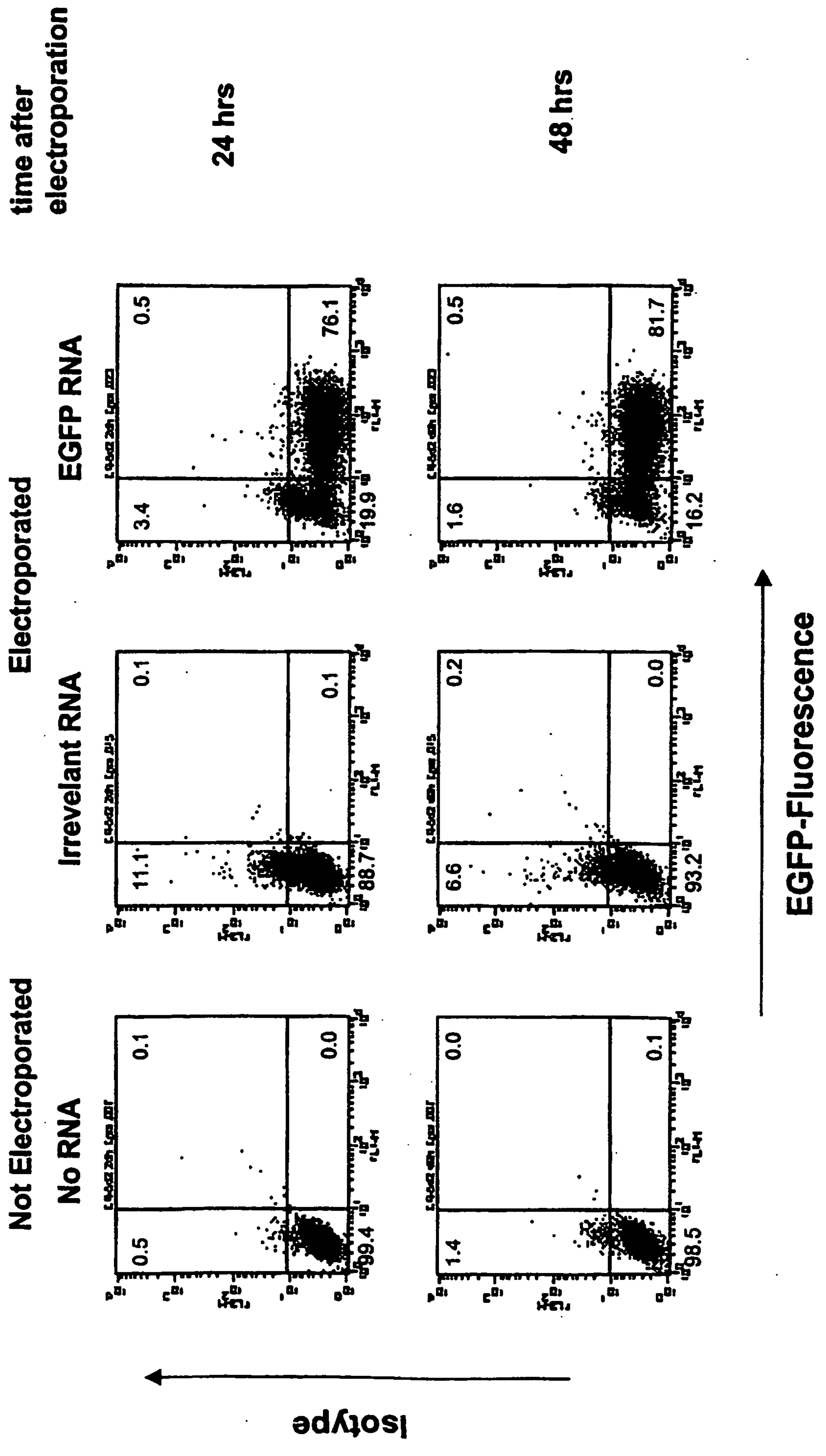
Not Electroporated
No RNA
Electroporated
Irrevelant RNA
EGFP RNA
time after electroporation
24 hrs
48 hrs
0.5
0.1
0.0
99.4
1.4
0.0
0.1
98.5
11.1
0.1
0.1
88.7
6.6
0.2
0.0
93.2
3.4
0.5
76.1
19.9
1.6
0.5
81.7
16.2
Isotype
EGFP-Fluorescence

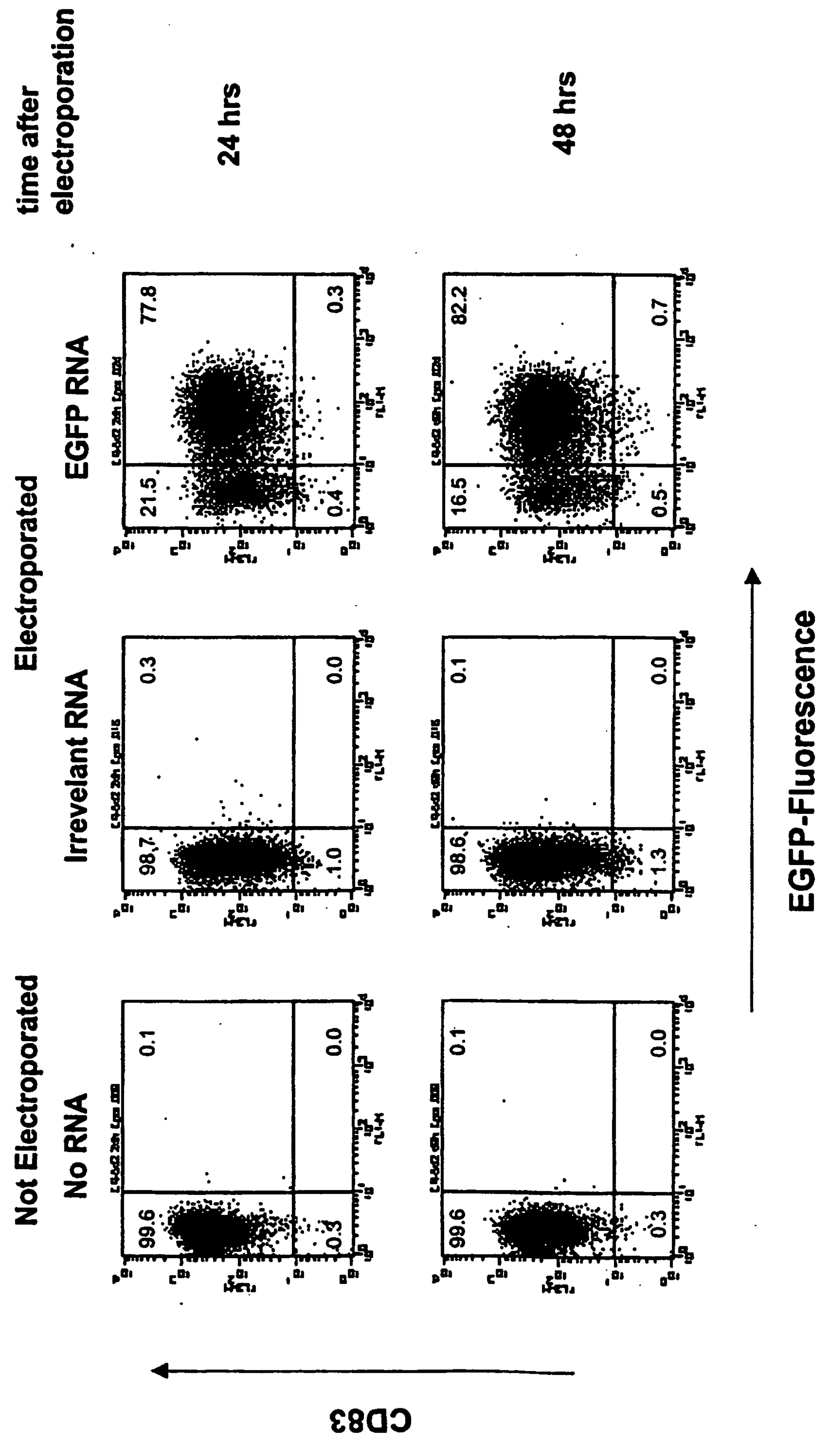
time after electroporation
24 hrs
48 hrs
Not Electroporated
No RNA
Electroporated
Irrevelant RNA
EGFP RNA
99.6
0.1
0.3
0.0
98.7
0.3
1.0
0.0
21.5
77.8
0.4
0.3
99.6
0.1
0.3
0.0
98.6
0.1
1.3
0.0
16.5
82.2
0.5
0.7
CD83
EGFP-Fluorescence

Fig. 10B

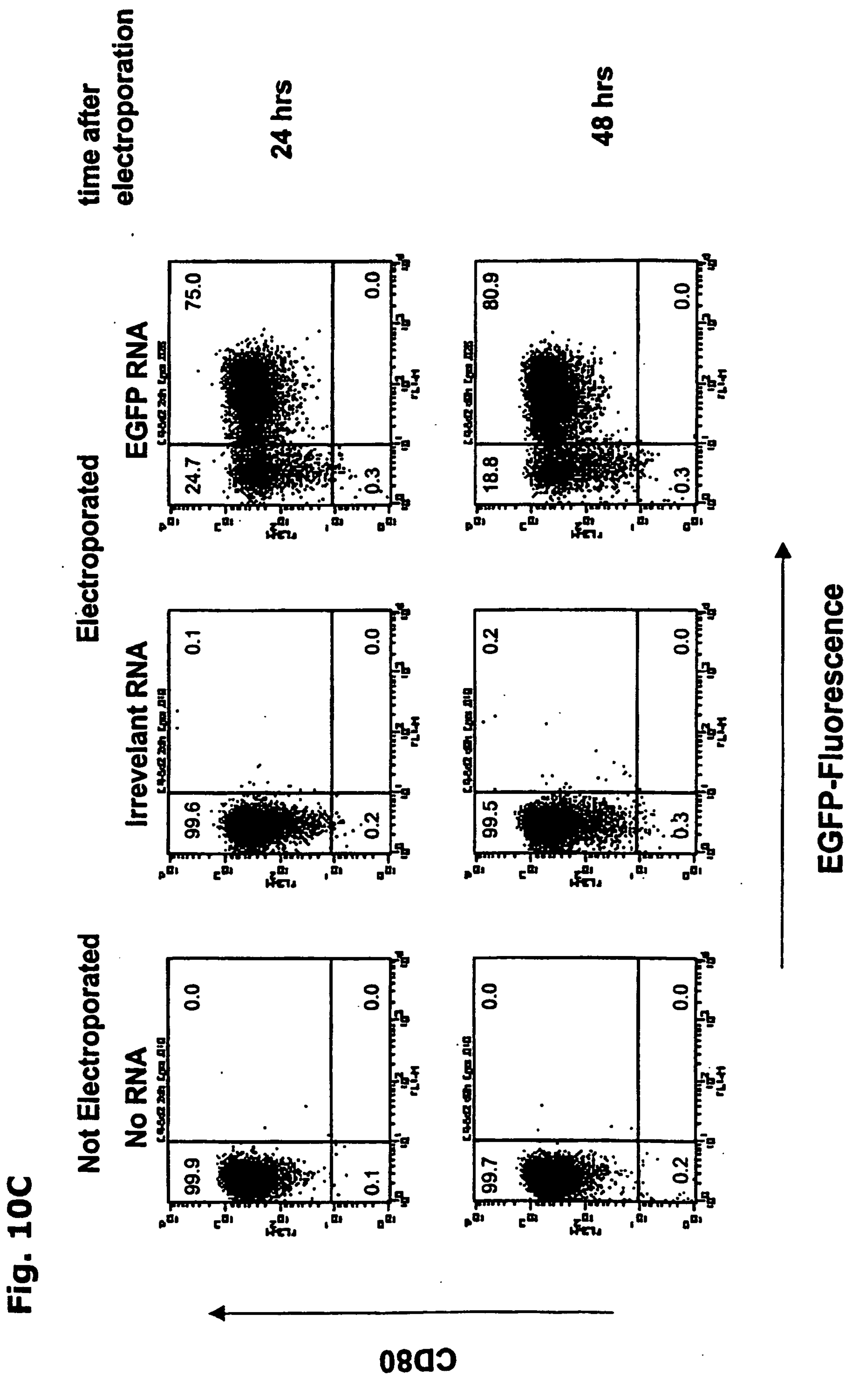
Fig. 10C
Not Electroporated
No RNA
Electroporated
Irrevelant RNA
EGFP RNA
time after electroporation
24 hrs
48 hrs
99.9
0.0
0.1
0.0
99.6
0.1
0.2
0.0
24.7
75.0
0.3
0.0
99.7
0.0
0.2
0.0
99.5
0.2
0.3
0.0
18.8
80.9
0.3
0.0
EGFP-Fluorescence
CD80

Fig. 10D

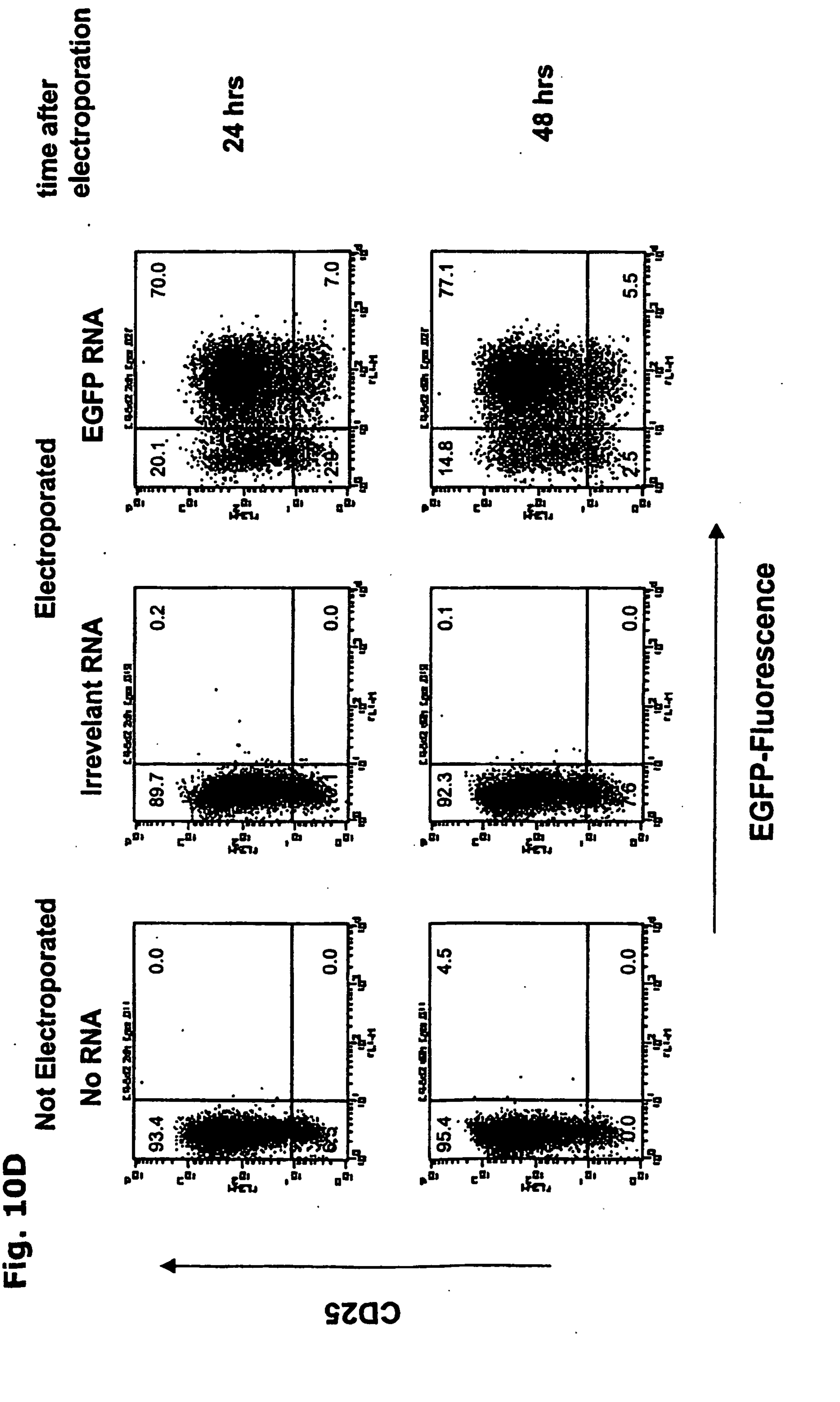
Not Electroporated
No RNA
Electroporated
Irrevelant RNA
EGFP RNA
time after electroporation
24 hrs
48 hrs
93.4
0.0
0.0
89.7
0.2
0.0
20.1
70.0
7.0
95.4
4.5
0.0
92.3
0.1
0.0
14.8
77.1
5.5
2.5
CD25
EGFP-Fluorescence

Fig. 10E

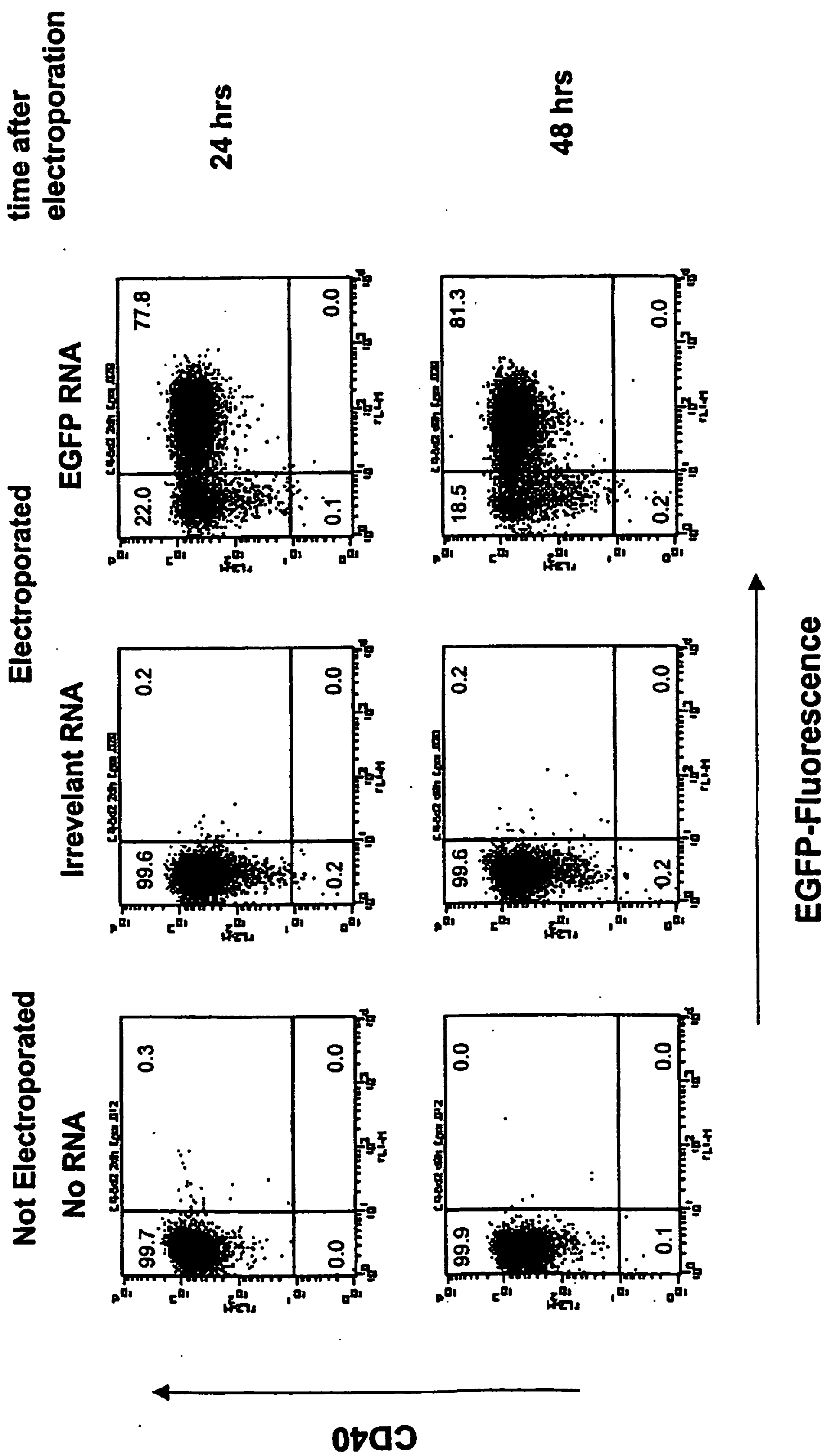
time after electroporation
24 hrs
48 hrs
Not Electroporated
No RNA
Electroporated
Irrevelant RNA
EGFP RNA
99.7
0.3
0.0
0.0
99.6
0.2
0.2
0.0
22.0
77.8
0.1
0.0
99.9
0.0
0.1
0.0
99.6
0.2
0.2
0.0
18.5
81.3
0.2
0.0
CD40
EGFP-Fluorescence

Fig. 10F

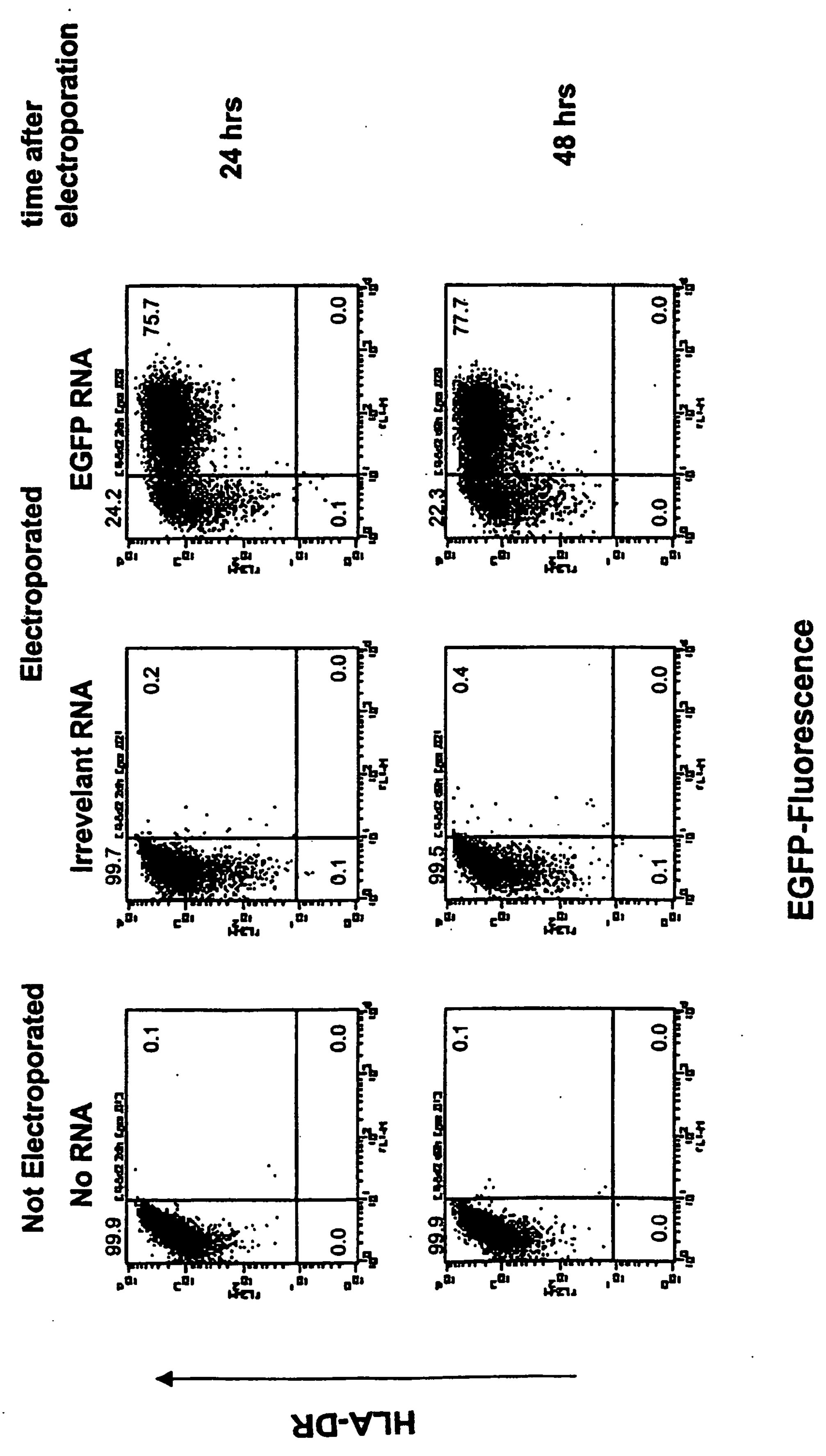

time after electroporation
24 hrs
48 hrs
Not Electroporated
No RNA
Electroporated
Irrevelant RNA
EGFP RNA
99.9
0.1
0.0
0.0
99.7
0.2
0.1
0.0
24.2
75.7
0.1
0.0
99.9
0.1
0.0
0.0
99.5
0.4
0.1
0.0
22.3
77.7
0.0
0.0
EGFP-Fluorescence
HLA-DR

Fig. 10G

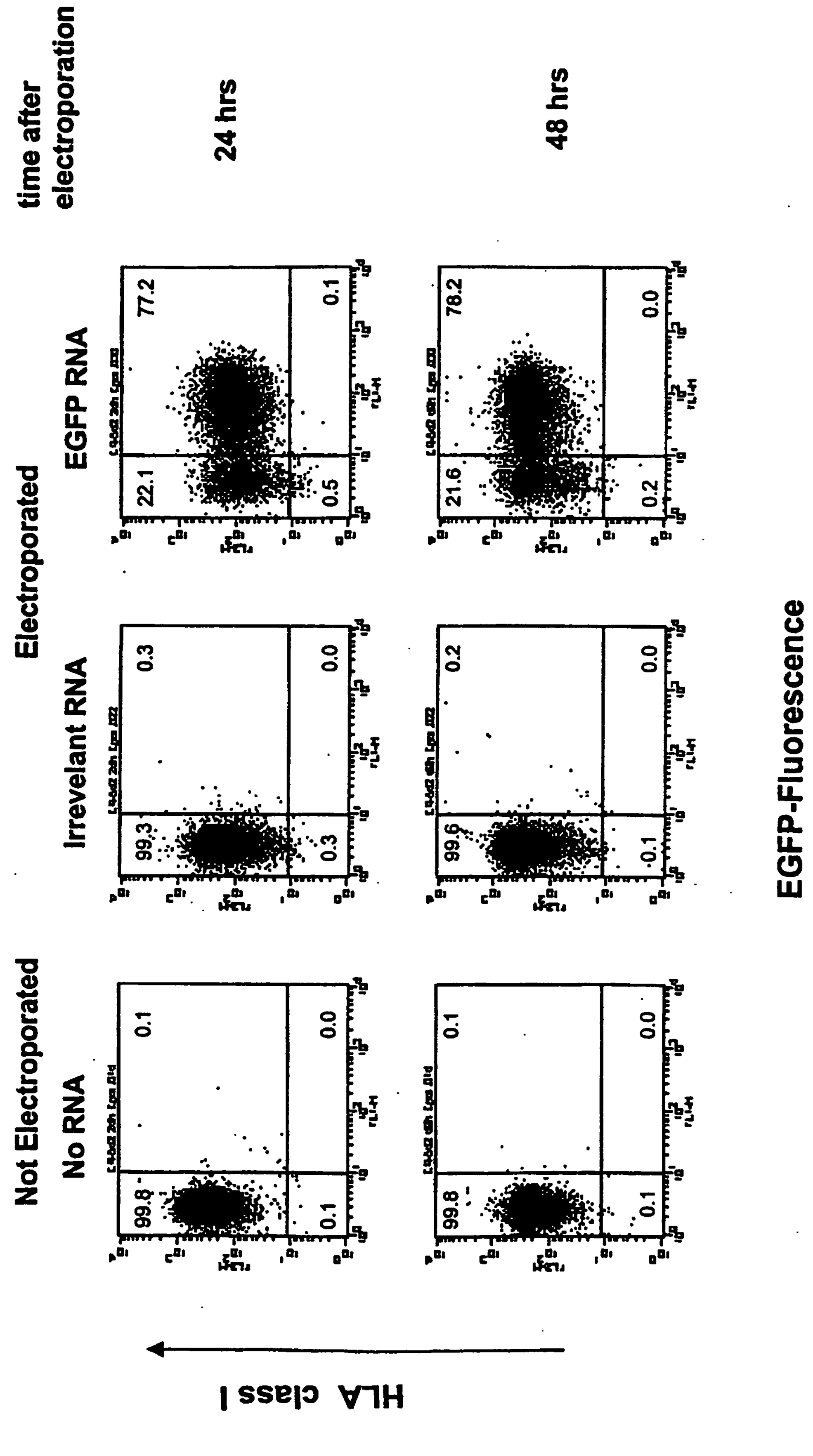
time after electroporation
24 hrs
48 hrs
Not Electroporated
No RNA
Electroporated
Irrevelant RNA
EGFP RNA
99.8
0.1
0.1
0.0
99.3
0.3
0.3
0.0
22.1
77.2
0.5
0.1
99.8
0.1
0.1
0.0
99.6
0.2
0.1
0.0
21.6
78.2
0.2
0.0
EGFP-Fluorescence
HLA class I

**Fig. 10H**

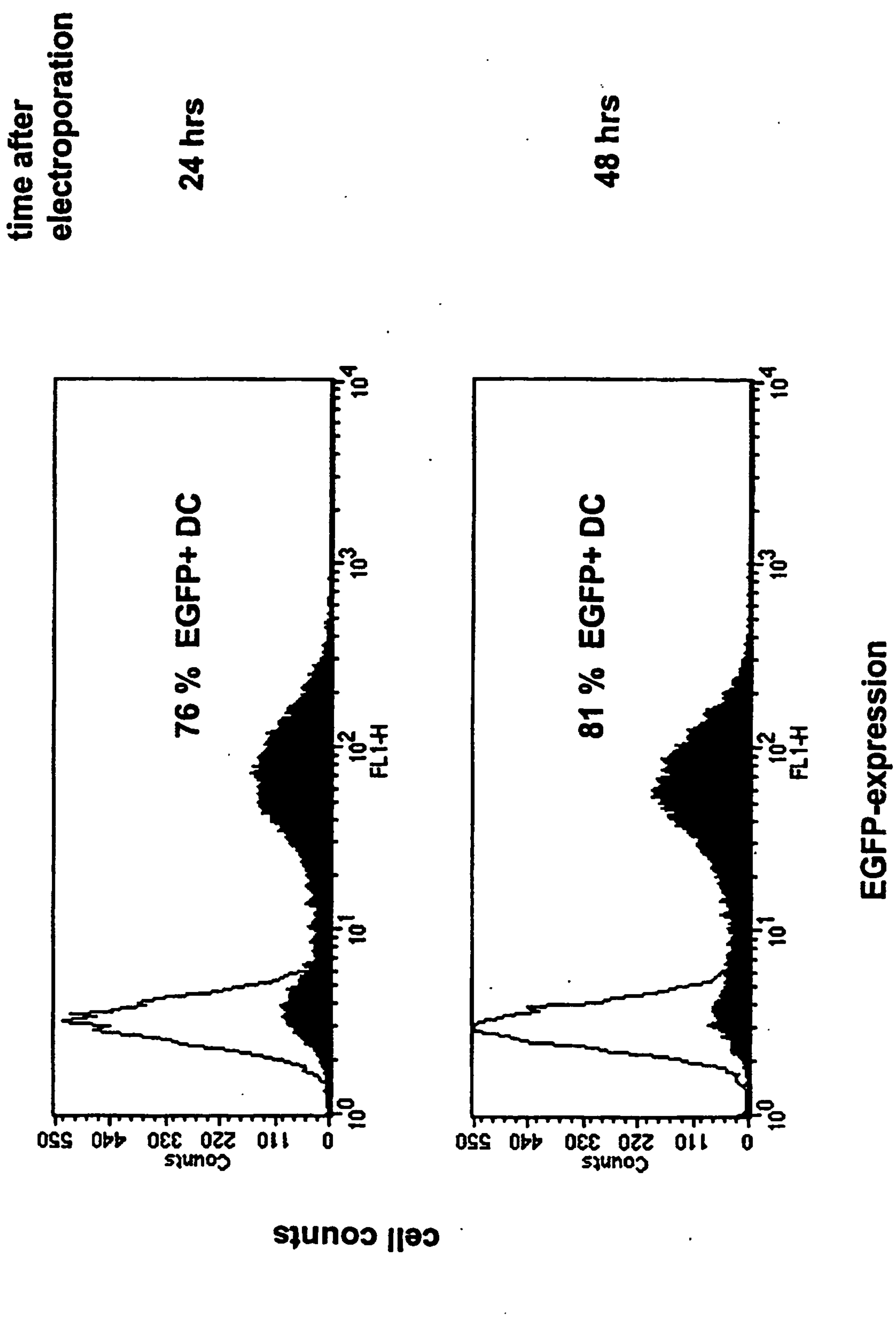
time after electroporation
24 hrs
48 hrs
76 % EGFP+ DC
81 % EGFP+ DC
Counts
0 110 220 330 440 550
FL1-H
$10^0$ $10^1$ $10^2$ $10^3$ $10^4$
cell counts
EGFP-expression

Fig. 11

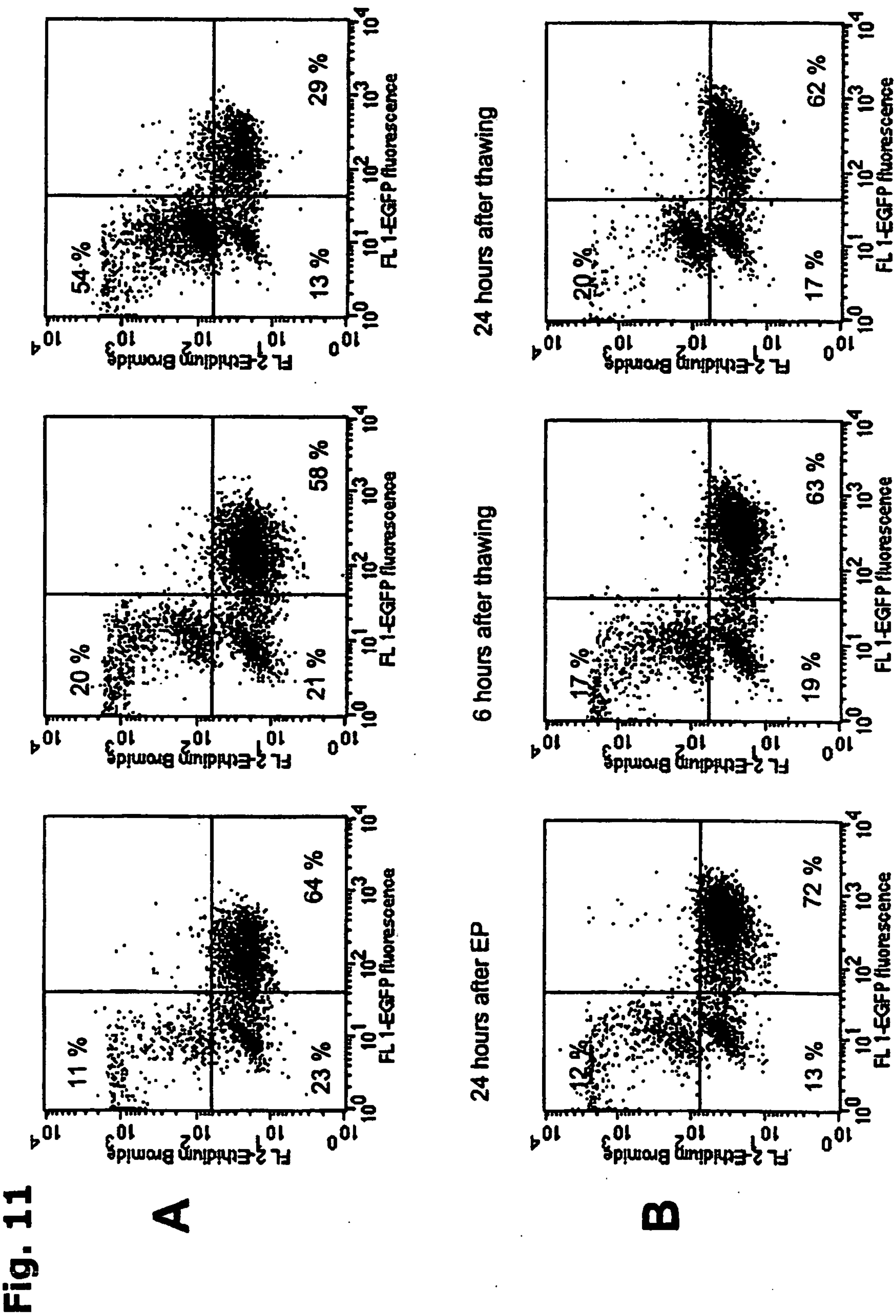
A
B
24 hours after EP
6 hours after thawing
24 hours after thawing
11 %
64 %
23 %
20 %
58 %
21 %
54 %
29 %
13 %
12 %
72 %
13 %
17 %
63 %
19 %
20 %
62 %
17 %
FL 1-EGFP fluorescence
FL 2-Ethidium Bromide

Fig. 12A

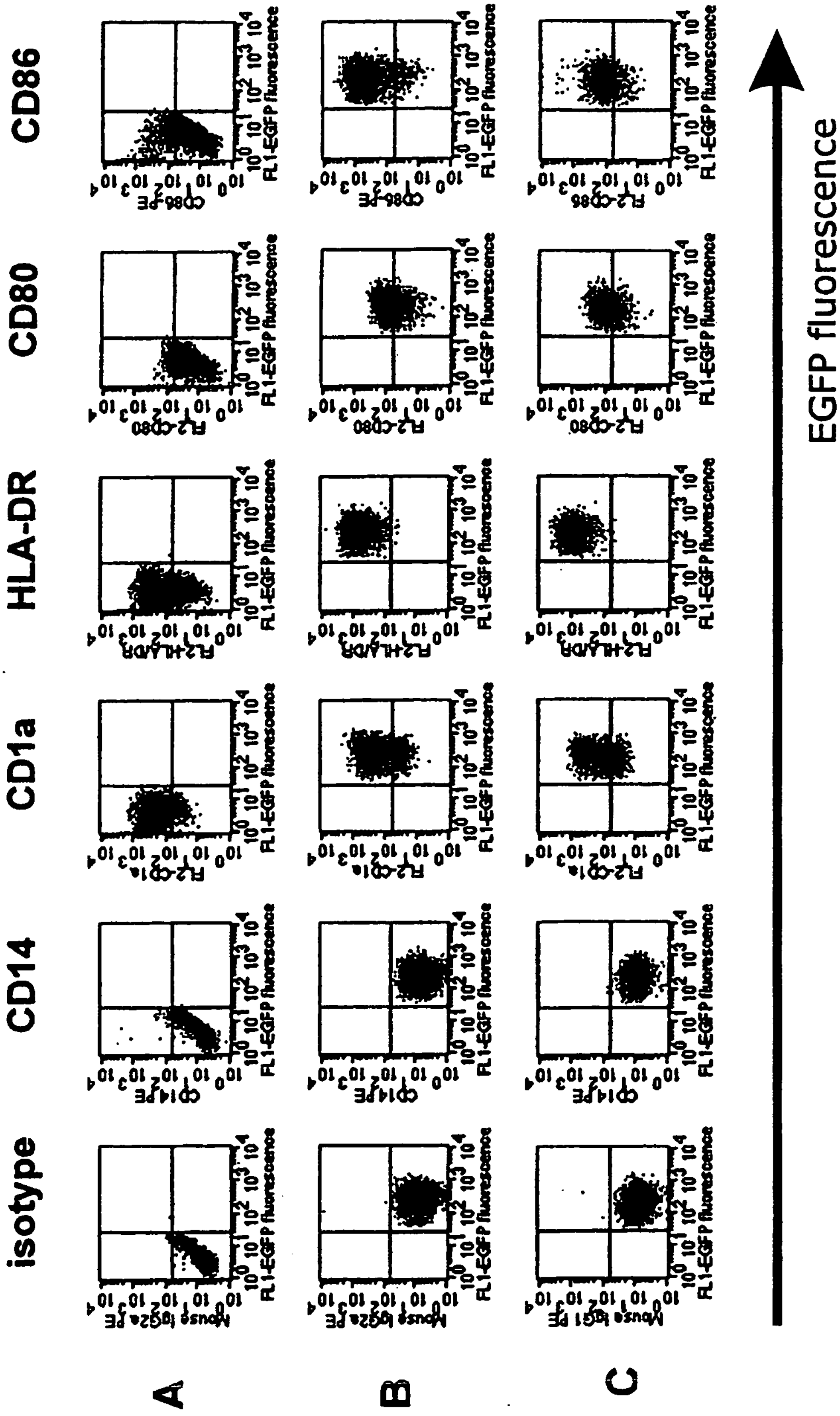

Fig. 12B

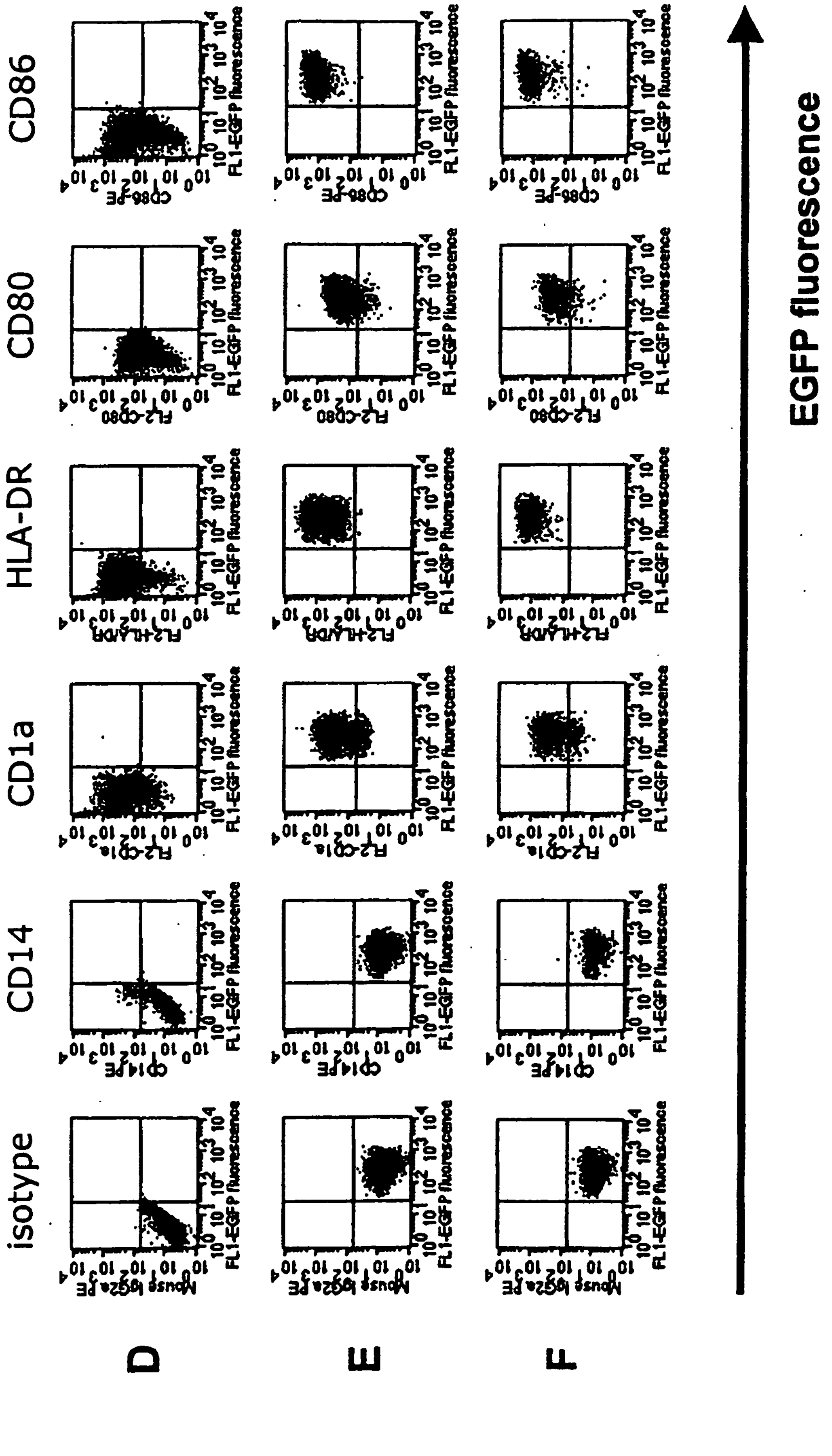
isotype
CD14
CD1a
HLA-DR
CD80
CD86
D
E
F
Mouse IgG2a PE
CD14PE
FL2-CD1a
FL2-HLA/DR
FL2-CD80
CD86-PE
FL1-EGFP fluorescence
EGFP fluorescence

Fig. 13

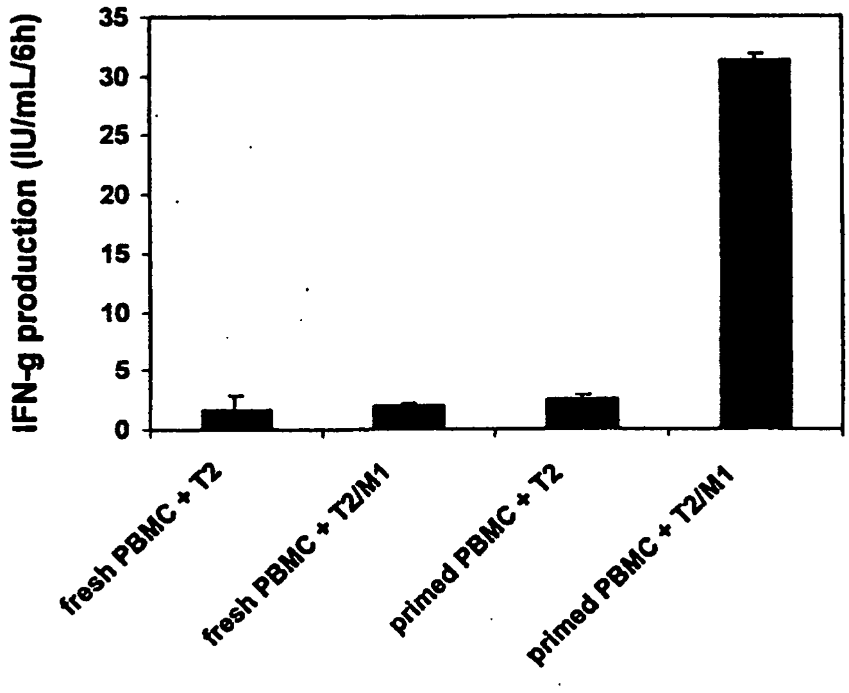
IFN-g production (IIU/mL/6h)
35
30
25
20
15
10
5
0
fresh PBMC + T2
fresh PBMC + T2/M1
primed PBMC + T2
primed PBMC + T2/M1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5766902 A
- US 5554528 A

### Non-patent literature cited in the description


- **BANCHEREAU, J. ; STEINMAN, R.M.** *Nature,* 1998, vol. 392, 245-252
- **AVIGAN, D.** *Blood Rev.,* 1999, vol. 13, 51-64
- **TARTE, K. ; KLEIN, B.** *Leukemia,* 1999, vol. 13, 653-663
- **DIETZ, A.B. ; VUK, P.S.** *Blood,* 1998, vol. 91, 392-398
- **BROSSART, P. et al.** *J. Immunol.,* 1997, vol. 158, 3270-3276
- **SPECHT, J.M. et al.** *J. Exp. Med.,* 1997, vol. 186, 1213-1221
- **LUO, D. ; SALTZMAN, W.M.** *Nat. Biotechnol.,* 2000, vol. 18, 33-37
- **LU, D. et al.** *Cancer Gene Ther.,* 1994, vol. 1, 245-252
- **KARIKO, K. et al.** *Biochim. Biophys. Acta,* 1998, vol. 1369, 320-334
- **SAWAI, K. et al.** *Mol. Genet. Metab.,* 1998, vol. 64, 44-51
- **KIM, C. J. et al.** *J. Immunother.,* 1997, vol. 20, 276-286
- **DIETZ, A. B. ; VUK, P.S.** *Blood,* 1998, vol. 91, 392-398
- **JENNE, L. et al.** *Gene Ther.,* 2000, vol. 7, 1575-1583
- **JONULEIT, H et al.** *Gene Ther.,* 2000, vol. 7, 249-254
- **ZHANG, W. et al.** *Hum. Gene Ther.,* 1999, vol. 10, 1151-1161
- **YING, H. et al.** *Nat. med.,* 1999, vol. 5, 823-827
- **ARTHUR, J.F. et al.** *Cancer Gene Ther.,* 1997, vol. 4, 17-25
- **VAN TENDELOO, V.F.I et al.** *Gene Ther.,* 1998, vol. 5, 700-707
- **NAIR, S. K. et al.** *Nat. Biotechnol.,* 1998, vol. 16, 364-369
- **VAN TENDELOO V.F.I et al.** *Gene Ther.,* 1998, vol. 5, 700-707
- **VAN TENDELOO, V.F.I et al.** *Gene Ther.,* 2000, vol. 7, 1431-1437
- **VAN BOCKSTAELE, D. ; BERNEMAN, Z.N.** *Cytometry,* 2000, vol. 41, 31-35
- **LURQUIN, P.F.** *Mol. Biotechnol.,* 1997, vol. 7, 5-35
- **MATTHEWS, K.E. et al.** Mol. Biotechnol. vol. 48
- **SPENCER, S.C.** *Biochem. and Biotechnol.,* 1993, vol. 42, 75-82
- **STROBEL, I. et al.** *Gene Therapy,* 2000, vol. 7, 2028-2035
- *Poster at the 6th Symposium on dendritic cells,* 26 May 2000
- *Keystone Symposia,* 12 March 2001
- **PORGADOR, A. et al.** *J. Exp. Med.,* 1998, vol. 188, 1075-1082
- **CELLA, M. et al.** *Curr. Opin. Immunol.,* 1997, vol. 9, 10-16
- **MORSE, M. A. et al.** *Cancer Res.,* 1998, vol. 58, 2965-2968
- **PONSAERTS et al.** *Journal of Immunology,* 2002
- **GORDILLO, G. M.** *Transpl. Immunol.,* 1999, vol. 7, 83-94
- **KLINMAN, D. M. et al.** *Proc. Natl. Acad. Sci. USA,* 1996, vol. 93, 2879-2883
- **KLINMAN, D. M. et al.** *Vaccine,* 1999, vol. 17, 19-25
- **NAIR et al.** *Nat. Biotechnol.,* 1998, vol. 16, 364-369
- **REEVES, M. E. et al.** *Cancer Res.,* 1996, vol. 56, 5672-5677
- **BUHRING, H. J. et al.** *Leukemia,* 1991, vol. 5, 854-860
- **LARDON, F. et al.** *Immunology,* 1997, vol. 91, 553-559
- **HERBST, B. et al.** *Blood,* 1996, vol. 88, 2541-2548
- **ROMANI, N. et al.** *J. Exp. Med.,* 1996, vol. 180, 83-93
- **VAN TENDERLOO, V. P. et al.** *Blood,* 2001, vol. 98, 49
- **VAN TENDELOO, V.F.I. et al.** *Gene Ther.,* 1998, vol. 5, 700-707
- **BAUM, C. et al.** *Biotechniques,* 1994, vol. 17, 1058-1062

- **ROMANI, N. et al.** *J. Immunol. Methods,* 1996, vol. 196, 137-151
- **SALLUSTO, F. ; LANZAVECCHIA, A.** *J. Exp. Med.,* 1994, vol. 179, 1109-1118
- **BANCHERAU, J. ; STEINMANN, R. N.** *Nature,* 1998, vol. 392, 245-252
- **FEUERSTEIN, B. et al.** *J. Immunol. Methods,* 2000, vol. 245, 15-29
- **RONCAROLO, M.G. et al.** *Exp. Med.,* 2001, vol. 193 (2), F5-9
- **RONCAROLO, M.G et al.** *J. Exp. Med.,* 2000, vol. 193 (2), F5-9

SEQUENCE LISTING

<110> Schuler, Gerold N.V. Antwerps Innovatiecentrum

<120> Improved Transfection of Eucaryotic Cells with Linear Polynucleotides by Electroporation

<130> 021505wo/JH/ml

<140>
<141>

<160> 34

<170> PatentIn Ver. 2.1

<210> 1
<211> 4733
<212> DNA
<213> Artificial Sequence

<220>
<223> Description of Artificial Sequence: pEGFP-N1 cloning vector (Clonetech)

<220>
<221> CDS
<222> (679) .. (1395)

<400> 1

```
tagttattaa tagtaatcaa ttacggggtc attagttcat agcccatata tggagttccg 60

cgttacataa cttacggtaa atggcccgcc tggctgaccg cccaacgacc cccgcccatt 120

gacgtcaata atgacgtatg ttcccatagt aacgccaata gggactttcc attgacgtca 180

atgggtggag tatttacggt aaactgccca cttggcagta catcaagtgt atcatatgcc 240

aagtacgccc cctattgacg tcaatgacgg taaatggccc gcctggcatt atgcccagta 300

catgacctta tgggactttc ctacttggca gtacatctac gtattagtca tcgctattac 360

catggtgatg cggttttggc agtacatcaa tgggcgtgga tagcggtttg actcacgggg 420

atttccaagt ctccacccca ttgacgtcaa tgggagtttg ttttggcacc aaaatcaacg 480

ggactttcca aaatgtcgta acaactccgc cccattgacg caaatgggcg gtaggcgtgt 540

acggtgggag gtctatataa gcagagctgg tttagtgaac cgtcagatcc gctagcgcta 600

ccggactcag atctcgagct caagcttcga attctgcagt cgacggtacc gcgggcccgg 660

gatccaccgg tcgccacc atg gtg agc aag ggc gag gag ctg ttc acc ggg   711
                    Met Val Ser Lys Gly Glu Glu Leu Phe Thr Gly
                    1               5                   10

gtg gtg ccc atc ctg gtc gag ctg gac ggc gac gta aac ggc cac aag   759
Val Val Pro Ile Leu Val Glu Leu Asp Gly Asp Val Asn Gly His Lys
            15                  20                  25

ttc agc gtg tcc ggc gag ggc gag ggc gat gcc acc tac ggc aag ctg   807
Phe Ser Val Ser Gly Glu Gly Glu Gly Asp Ala Thr Tyr Gly Lys Leu
```

```
        30                  35                  40

acc ctg aag ttc atc tgc acc acc ggc aag ctg ccc gtg ccc tgg ccc    855
Thr Leu Lys Phe Ile Cys Thr Thr Gly Lys Leu Pro Val Pro Trp Pro
    45                  50                  55

acc ctc gtg acc acc ctg acc tac ggc gtg cag tgc ttc agc cgc tac    903
Thr Leu Val Thr Thr Leu Thr Tyr Gly Val Gln Cys Phe Ser Arg Tyr
60                  65                  70                  75

ccc gac cac atg aag cag cac gac ttc ttc aag tcc gcc atg ccc gaa    951
Pro Asp His Met Lys Gln His Asp Phe Phe Lys Ser Ala Met Pro Glu
                80                  85                  90

ggc tac gtc cag gag cgc acc atc ttc ttc aag gac gac ggc aac tac    999
Gly Tyr Val Gln Glu Arg Thr Ile Phe Phe Lys Asp Asp Gly Asn Tyr
            95                  100                 105

aag acc cgc gcc gag gtg aag ttc gag ggc gac acc ctg gtg aac cgc    1047
Lys Thr Arg Ala Glu Val Lys Phe Glu Gly Asp Thr Leu Val Asn Arg
        110                 115                 120

atc gag ctg aag ggc atc gac ttc aag gag gac ggc aac atc ctg ggg    1095
Ile Glu Leu Lys Gly Ile Asp Phe Lys Glu Asp Gly Asn Ile Leu Gly
    125                 130                 135

cac aag ctg gag tac aac tac aac agc cac aac gtc tat atc atg gcc    1143
His Lys Leu Glu Tyr Asn Tyr Asn Ser His Asn Val Tyr Ile Met Ala
140                 145                 150                 155

gac aag cag aag aac ggc atc aag gtg aac ttc aag atc cgc cac aac    1191
Asp Lys Gln Lys Asn Gly Ile Lys Val Asn Phe Lys Ile Arg His Asn
                160                 165                 170

atc gag gac ggc agc gtg cag ctc gcc gac cac tac cag cag aac acc    1239
Ile Glu Asp Gly Ser Val Gln Leu Ala Asp His Tyr Gln Gln Asn Thr
            175                 180                 185

ccc atc ggc gac ggc ccc gtg ctg ctg ccc gac aac cac tac ctg agc    1287
Pro Ile Gly Asp Gly Pro Val Leu Leu Pro Asp Asn His Tyr Leu Ser
        190                 195                 200

acc cag tcc gcc ctg agc aaa gac ccc aac gag aag cgc gat cac atg    1335
Thr Gln Ser Ala Leu Ser Lys Asp Pro Asn Glu Lys Arg Asp His Met
    205                 210                 215

gtc ctg ctg gag ttc gtg acc gcc gcc ggg atc act ctc ggc atg gac    1383
Val Leu Leu Glu Phe Val Thr Ala Ala Gly Ile Thr Leu Gly Met Asp
220                 225                 230                 235

gag ctg tac aag taaagcggcc gcgactctag atcataatca gccataccac        1435
Glu Leu Tyr Lys

atttgtagag gttttacttg ctttaaaaaa cctcccacac ctccccctga acctgaaaca 1495

taaaatgaat gcaattgttg ttgttaactt gtttattgca gcttataatg gttacaaata 1555

aagcaatagc atcacaaatt tcacaaataa agcatttttt tcactgcatt ctagttgtgg 1615

tttgtccaaa ctcatcaatg tatcttaagg cgtaaattgt aagcgttaat attttgttaa 1675

aattcgcgtt aaatttttgt taaatcagct cattttttaa ccaataggcc gaaatcggca 1735
```

aaatccctta taaatcaaaa gaatagaccg agatagggtt gagtgttgtt ccagtttgga 1795

acaagagtcc actattaaag aacgtggact ccaacgtcaa agggcgaaaa accgtctatc 1855

agggcgatgg cccactacgt gaaccatcac cctaatcaag ttttttgggg tcgaggtgcc 1915

gtaaagcact aaatcggaac cctaaaggga gcccccgatt tagagcttga cggggaaagc 1975

cggcgaacgt ggcgagaaag gaagggaaga aagcgaaagg agcgggcgct agggcgctgg 2035

caagtgtagc ggtcacgctg cgcgtaacca ccacacccgc cgcgcttaat gcgccgctac 2095

agggcgcgtc aggtggcact tttcggggaa atgtgcgcgg aacccctatt tgtttatttt 2155

tctaaataca ttcaaatatg tatccgctca tgagacaata accctgataa atgcttcaat 2215

aatattgaaa aaggaagagt cctgaggcgg aaagaaccag ctgtggaatg tgtgtcagtt 2275

agggtgtgga aagtccccag gctccccagc aggcagaagt atgcaaagca tgcatctcaa 2335

ttagtcagca accaggtgtg gaaagtcccc aggctcccca gcaggcagaa gtatgcaaag 2395

catgcatctc aattagtcag caaccatagt cccgccccta actccgccca tcccgcccct 2455

aactccgccc agttccgccc attctccgcc ccatggctga ctaatttttt ttatttatgc 2515

agaggccgag gccgcctcgg cctctgagct attccagaag tagtgaggag gcttttttgg 2575

aggcctaggc ttttgcaaag atcgatcaag agacaggatg aggatcgttt cgcatgattg 2635

aacaagatgg attgcacgca ggttctccgg ccgcttgggt ggagaggcta ttcggctatg 2695

actgggcaca acagacaatc ggctgctctg atgccgccgt gttccggctg tcagcgcagg 2755

ggcgcccggt tctttttgtc aagaccgacc tgtccggtgc cctgaatgaa ctgcaagacg 2815

aggcagcgcg gctatcgtgg ctggccacga cgggcgttcc ttgcgcagct gtgctcgacg 2875

ttgtcactga agcgggaagg gactggctgc tattgggcga agtgccgggg caggatctcc 2935

tgtcatctca ccttgctcct gccgagaaag tatccatcat ggctgatgca atgcggcggc 2995

tgcatacgct tgatccggct acctgcccat tcgaccacca agcgaaacat cgcatcgagc 3055

gagcacgtac tcggatggaa gccggtcttg tcgatcagga tgatctggac gaagagcatc 3115

aggggctcgc gccagccgaa ctgttcgcca ggctcaaggc gagcatgccc gacggcgagg 3175

atctcgtcgt gacccatggc gatgcctgct tgccgaatat catggtggaa aatggccgct 3235

tttctggatt catcgactgt ggccggctgg gtgtggcgga ccgctatcag gacatagcgt 3295

tggctacccg tgatattgct gaagagcttg gcggcgaatg ggctgaccgc ttcctcgtgc 3355

tttacggtat cgccgctccc gattcgcagc gcatcgcctt ctatcgcctt cttgacgagt 3415

tcttctgagc gggactctgg ggttcgaaat gaccgaccaa gcgacgccca acctgccatc 3475

acgagatttc gattccaccg ccgccttcta tgaaaggttg ggcttcggaa tcgttttccg 3535

ggacgccggc tggatgatcc tccagcgcgg ggatctcatg ctggagttct tcgcccaccc 3595

tagggggagg ctaactgaaa cacggaagga gacaataccg gaaggaaccc gcgctatgac 3655

```
ggcaataaaa agacagaata aaacgcacgg tgttgggtcg tttgttcata aacgcggggt 3715
tcggtcccag ggctggcact ctgtcgatac cccaccgaga ccccattggg gccaatacgc 3775
ccgcgtttct tccttttccc caccccaccc cccaagttcg ggtgaaggcc cagggctcgc 3835
agccaacgtc ggggcggcag gccctgccat agcctcaggt tactcatata tactttagat 3895
tgatttaaaa cttcattttt aatttaaaag gatctaggtg aagatccttt ttgataatct 3955
catgaccaaa atcccttaac gtgagttttc gttccactga gcgtcagacc ccgtagaaaa 4015
gatcaaagga tcttcttgag atcctttttt tctgcgcgta atctgctgct tgcaaacaaa 4075
aaaaccaccg ctaccagcgg tggtttgttt gccggatcaa gagctaccaa ctctttttcc 4135
gaaggtaact ggcttcagca gagcgcagat accaaatact gtccttctag tgtagccgta 4195
gttaggccac cacttcaaga actctgtagc accgcctaca tacctcgctc tgctaatcct 4255
gttaccagtg gctgctgcca gtggcgataa gtcgtgtctt accgggttgg actcaagacg 4315
atagttaccg gataaggcgc agcggtcggg ctgaacgggg ggttcgtgca cacagcccag 4375
cttggagcga acgacctaca ccgaactgag atacctacag cgtgagctat gagaaagcgc 4435
cacgcttccc gaagggagaa aggcggacag gtatccggta agcggcaggg tcggaacagg 4495
agagcgcacg agggagcttc cagggggaaa cgcctggtat ctttatagtc ctgtcgggtt 4555
tcgccacctc tgacttgagc gtcgattttt gtgatgctcg tcaggggggc ggagcctatg 4615
gaaaaacgcc agcaacgcgg cctttttacg gttcctggcc ttttgctggc cttttgctca 4675
catgttcttt cctgcgttat cccctgattc tgtggataac cgtattaccg ccatgcat   4733
```

<210> 2
<211> 239
<212> PRT
<213> Artificial Sequence
<223> Description of Artificial Sequence: pEGFP-N1 cloning vector (Clonetech)

<400> 2

```
Met Val Ser Lys Gly Glu Glu Leu Phe Thr Gly Val Val Pro Ile Leu
 1               5                  10                  15

Val Glu Leu Asp Gly Asp Val Asn Gly His Lys Phe Ser Val Ser Gly
            20                  25                  30

Glu Gly Glu Gly Asp Ala Thr Tyr Gly Lys Leu Thr Leu Lys Phe Ile
        35                  40                  45

Cys Thr Thr Gly Lys Leu Pro Val Pro Trp Pro Thr Leu Val Thr Thr
    50                  55                  60

Leu Thr Tyr Gly Val Gln Cys Phe Ser Arg Tyr Pro Asp His Met Lys
65                  70                  75                  80

Gln His Asp Phe Phe Lys Ser Ala Met Pro Glu Gly Tyr Val Gln Glu
                85                  90                  95

Arg Thr Ile Phe Phe Lys Asp Asp Gly Asn Tyr Lys Thr Arg Ala Glu
            100                 105                 110

Val Lys Phe Glu Gly Asp Thr Leu Val Asn Arg Ile Glu Leu Lys Gly
        115                 120                 125

Ile Asp Phe Lys Glu Asp Gly Asn Ile Leu Gly His Lys Leu Glu Tyr
    130                 135                 140

Asn Tyr Asn Ser His Asn Val Tyr Ile Met Ala Asp Lys Gln Lys Asn
145                 150                 155                 160

Gly Ile Lys Val Asn Phe Lys Ile Arg His Asn Ile Glu Asp Gly Ser
                165                 170                 175

Val Gln Leu Ala Asp His Tyr Gln Gln Asn Thr Pro Ile Gly Asp Gly
            180                 185                 190

Pro Val Leu Leu Pro Asp Asn His Tyr Leu Ser Thr Gln Ser Ala Leu
        195                 200                 205

Ser Lys Asp Pro Asn Glu Lys Arg Asp His Met Val Leu Leu Glu Phe
    210                 215                 220

Val Thr Ala Ala Gly Ile Thr Leu Gly Met Asp Glu Leu Tyr Lys
225                 230                 235
```

<210> 3
<211> 3023
<212> DNA
<213> Homo sapiens

<220>
<221> CDS
<222> (391)..(1737)

<400> 3

```
ggggtaagga gttcaaggca gcgcccacac ccgggggctc tccgcaaccc gaccgcctgt 60

ccgctccccc acttcccgcc ctccctccca cctactcatt cacccaccca cccacccaga 120

gccgggacgg cagcccaggc gcccgggccc cgccgtctcc tcgccgcgat cctggacttc 180

ctcttgctgc aggacccggc ttccacgtgt gtcccggagc cggcgtctca gcacacgctc 240

cgctccgggc ctgggtgcct acagcagcca gagcagcagg gagtccggga cccgggcggc 300

atctgggcca agttaggcgc cgccgaggcc agcgctgaac gtctccaggg ccggaggagc 360

cgcggggcgt ccgggtctga gccgcagcaa atg ggc tcc gac gtg cgg gac ctg  414
                                Met Gly Ser Asp Val Arg Asp Leu
                                1               5

aac gcg ctg ctg ccc gcc gtc ccc tcc ctg ggt ggc ggc ggc ggc tgt  462
Asn Ala Leu Leu Pro Ala Val Pro Ser Leu Gly Gly Gly Gly Gly Cys
    10                  15                  20

gcc ctg cct gtg agc ggc gcg gcg cag tgg gcg ccg gtg ctg gac ttt  510
Ala Leu Pro Val Ser Gly Ala Ala Gln Trp Ala Pro Val Leu Asp Phe
25                  30                  35                  40
```

```
gcg ccc ccg ggc gct tcg gct tac ggg tcg ttg ggc ggc ccc gcg ccg    558
Ala Pro Pro Gly Ala Ser Ala Tyr Gly Ser Leu Gly Gly Pro Ala Pro
                45                  50                  55

cca ccg gct ccg ccg cca ccc ccg ccg ccg ccg cct cac tcc ttc atc    606
Pro Pro Ala Pro Pro Pro Pro Pro Pro Pro Pro Pro His Ser Phe Ile
            60                  65                  70

aaa cag gag ccg agc tgg ggc ggc gcg gag ccg cac gag gag cag tgc    654
Lys Gln Glu Pro Ser Trp Gly Gly Ala Glu Pro His Glu Glu Gln Cys
        75                  80                  85

ctg agc gcc ttc act gtc cac ttt tcc ggc cag ttc act ggc aca gcc    702
Leu Ser Ala Phe Thr Val His Phe Ser Gly Gln Phe Thr Gly Thr Ala
    90                  95                  100

gga gcc tgt cgc tac ggg ccc ttc ggt cct cct ccg ccc agc cag gcg    750
Gly Ala Cys Arg Tyr Gly Pro Phe Gly Pro Pro Pro Pro Ser Gln Ala
105                 110                 115                 120

tca tcc ggc cag gcc agg atg ttt cct aac gcg ccc tac ctg ccc agc    798
Ser Ser Gly Gln Ala Arg Met Phe Pro Asn Ala Pro Tyr Leu Pro Ser
                125                 130                 135

tgc ctc gag agc cag ccc gct att cgc aat cag ggt tac agc acg gtc    846
Cys Leu Glu Ser Gln Pro Ala Ile Arg Asn Gln Gly Tyr Ser Thr Val
            140                 145                 150

acc ttc gac ggg acg ccc agc tac ggt cac acg ccc tcg cac cat gcg    894
Thr Phe Asp Gly Thr Pro Ser Tyr Gly His Thr Pro Ser His His Ala
        155                 160                 165

gcg cag ttc ccc aac cac tca ttc aag cat gag gat ccc atg ggc cag    942
Ala Gln Phe Pro Asn His Ser Phe Lys His Glu Asp Pro Met Gly Gln
    170                 175                 180

cag ggc tcg ctg ggt gag cag cag tac tcg gtg ccg ccc ccg gtc tat    990
Gln Gly Ser Leu Gly Glu Gln Gln Tyr Ser Val Pro Pro Pro Val Tyr
185                 190                 195                 200

ggc tgc cac acc ccc acc gac agc tgc acc ggc agc cag gct ttg ctg    1038
Gly Cys His Thr Pro Thr Asp Ser Cys Thr Gly Ser Gln Ala Leu Leu
                205                 210                 215

ctg agg acg ccc tac agc agt gac aat tta tac caa atg aca tcc cag    1086
Leu Arg Thr Pro Tyr Ser Ser Asp Asn Leu Tyr Gln Met Thr Ser Gln
            220                 225                 230

ctt gaa tgc atg acc tgg aat cag atg aac tta gga gcc acc tta aag    1134
Leu Glu Cys Met Thr Trp Asn Gln Met Asn Leu Gly Ala Thr Leu Lys
        235                 240                 245

gga gtt gct gct ggg agc tcc agc tca gtg aaa tgg aca gaa ggg cag    1182
Gly Val Ala Ala Gly Ser Ser Ser Ser Val Lys Trp Thr Glu Gly Gln
    250                 255                 260

agc aac cac agc aca ggg tac gag agc gat aac cac aca acg ccc atc    1230
Ser Asn His Ser Thr Gly Tyr Glu Ser Asp Asn His Thr Thr Pro Ile
265                 270                 275                 280

ctc tgc gga gcc caa tac aga ata cac acg cac ggt gtc ttc aga ggc    1278
Leu Cys Gly Ala Gln Tyr Arg Ile His Thr His Gly Val Phe Arg Gly
```

```
                285                 290                 295

att cag gat gtg cga cgt gtg cct gga gta gcc ccg act ctt gta cgg   1326
Ile Gln Asp Val Arg Arg Val Pro Gly Val Ala Pro Thr Leu Val Arg
            300                 305                 310

tcg gca tct gag acc agt gag aaa cgc ccc ttc atg tgt gct tac cca   1374
Ser Ala Ser Glu Thr Ser Glu Lys Arg Pro Phe Met Cys Ala Tyr Pro
        315                 320                 325

ggc tgc aat aag aga tat ttt aag ctg tcc cac tta cag atg cac agc   1422
Gly Cys Asn Lys Arg Tyr Phe Lys Leu Ser His Leu Gln Met His Ser
    330                 335                 340

agg aag cac act ggt gag aaa cca tac cag tgt gac ttc aag gac tgt   1470
Arg Lys His Thr Gly Glu Lys Pro Tyr Gln Cys Asp Phe Lys Asp Cys
345                 350                 355                 360

gaa cga agg ttt tct cgt tca gac cag ctc aaa aga cac caa agg aga   1518
Glu Arg Arg Phe Ser Arg Ser Asp Gln Leu Lys Arg His Gln Arg Arg
                365                 370                 375

cat aca ggt gtg aaa cca ttc cag tgt aaa act tgt cag cga aag ttc   1566
His Thr Gly Val Lys Pro Phe Gln Cys Lys Thr Cys Gln Arg Lys Phe
            380                 385                 390

tcc cgg tcc gac cac ctg aag acc cac acc agg act cat aca ggt aaa   1614
Ser Arg Ser Asp His Leu Lys Thr His Thr Arg Thr His Thr Gly Lys
        395                 400                 405

aca agt gaa aag ccc ttc agc tgt cgg tgg cca agt tgt cag aaa aag   1662
Thr Ser Glu Lys Pro Phe Ser Cys Arg Trp Pro Ser Cys Gln Lys Lys
    410                 415                 420

ttt gcc cgg tca gat gaa tta gtc cgc cat cac aac atg cat cag aga   1710
Phe Ala Arg Ser Asp Glu Leu Val Arg His His Asn Met His Gln Arg
425                 430                 435                 440

aac atg acc aaa ctc cag ctg gcg ctt tgaggggtct ccctcgggga         1757
Asn Met Thr Lys Leu Gln Leu Ala Leu
                445

ccgttcagtg tcccaggcag cacagtgtgt gaactgcttt caagtctgac tctccactcc 1817

tcctcactaa aaaggaaact tcagttgatc ttcttcatcc aacttccaag acaagatacc 1877

ggtgcttctg gaaactacca ggtgtgcctg gaagagttgg tctctgccct gcctactttt 1937

agttgactca caggccctgg agaagcagct aacaatgtct ggttagttaa aagcccattg 1997

ccatttggtg tggattttct actgtaagaa gagccatagc tgatcatgtc cccctgaccc 2057

ttcccttctt tttttatgct cgttttcgct ggggatggaa ttattgtacc attttctatc 2117

atggaatatt tataggccag ggcatgtgta tgtgtctgct aatgtaaact ttgtcatggt 2177

ttccatttac taacagcaac agcaagaaat aaatcagaga gcaaggcatc gggggtgaat 2237

cttgtctaac attcccgagg tcagccaggc tgctaacctg gaaagcagga tgtagttctg 2297

ccaggcaact tttaaagctc atgcatttca agcagctgaa gaaaaaatca gaactaacca 2357

gtacctctgt atagaaatct aaaagaattt taccattcag ttaattcaat gtgaacactg 2417
```

gcacactgct cttaagaaac tatgaagatc tgagattttt ttgtgtatgt ttttgactct 2477

tttgagtggt aatcatatgt gtctttatag atgtacatac ctccttgcac aaatggaggg 2537

gaattcattt tcatcactgg gagtgtcctt agtgtataaa aaccatgctg gtatatggct 2597

tcaagttgta aaaatgaaag tgactttaaa agaaaatagg ggatggtcca ggatctccac 2657

tgataagact gtttttaagt aacttaagga cctttgggtc tacaagtata tgtgaaaaaa 2717

atgagactta ctgggtgagg aaatccattg tttaaagatg gtcgtgtgtg tgtgtgtgtg 2777

tgtgtgtgtg tgtgtgttgt gttgtgtttt gtttttttaag ggagggaatt tattatttac 2837

cgttgcttga aattactgtg taaatatatg tctgataatg atttgctctt tgacaactaa 2897

aattaggact gtataagtac tagatgcatc actgggtgtt gatcttacaa gatattgatg 2957

ataacactta aaattgtaac ctgcattttt cactttgctc tcaattaaag tctattcaaa 3017

aggaaa 3023

<210> 4
<211> 449
<212> PRT
<213> Homo sapiens

<400> 4

```
Met Gly Ser Asp Val Arg Asp Leu Asn Ala Leu Leu Pro Ala Val Pro
 1               5                  10                  15

Ser Leu Gly Gly Gly Gly Gly Cys Ala Leu Pro Val Ser Gly Ala Ala
            20                  25                  30

Gln Trp Ala Pro Val Leu Asp Phe Ala Pro Pro Gly Ala Ser Ala Tyr
        35                  40                  45

Gly Ser Leu Gly Gly Pro Ala Pro Pro Pro Ala Pro Pro Pro Pro Pro
    50                  55                  60

Pro Pro Pro Pro His Ser Phe Ile Lys Gln Glu Pro Ser Trp Gly Gly
65                  70                  75                  80

Ala Glu Pro His Glu Glu Gln Cys Leu Ser Ala Phe Thr Val His Phe
                85                  90                  95

Ser Gly Gln Phe Thr Gly Thr Ala Gly Ala Cys Arg Tyr Gly Pro Phe
            100                 105                 110

Gly Pro Pro Pro Pro Ser Gln Ala Ser Ser Gly Gln Ala Arg Met Phe
        115                 120                 125

Pro Asn Ala Pro Tyr Leu Pro Ser Cys Leu Glu Ser Gln Pro Ala Ile
    130                 135                 140

Arg Asn Gln Gly Tyr Ser Thr Val Thr Phe Asp Gly Thr Pro Ser Tyr
145                 150                 155                 160

Gly His Thr Pro Ser His His Ala Ala Gln Phe Pro Asn His Ser Phe
                165                 170                 175
```

```
Lys His Glu Asp Pro Met Gly Gln Gln Gly Ser Leu Gly Glu Gln Gln
                180                 185                 190

Tyr Ser Val Pro Pro Pro Val Tyr Gly Cys His Thr Pro Thr Asp Ser
        195                 200                 205

Cys Thr Gly Ser Gln Ala Leu Leu Leu Arg Thr Pro Tyr Ser Ser Asp
    210                 215                 220

Asn Leu Tyr Gln Met Thr Ser Gln Leu Glu Cys Met Thr Trp Asn Gln
225                 230                 235                 240

Met Asn Leu Gly Ala Thr Leu Lys Gly Val Ala Ala Gly Ser Ser Ser
                245                 250                 255

Ser Val Lys Trp Thr Glu Gly Gln Ser Asn His Ser Thr Gly Tyr Glu
            260                 265                 270

Ser Asp Asn His Thr Thr Pro Ile Leu Cys Gly Ala Gln Tyr Arg Ile
        275                 280                 285

His Thr His Gly Val Phe Arg Gly Ile Gln Asp Val Arg Arg Val Pro
    290                 295                 300

Gly Val Ala Pro Thr Leu Val Arg Ser Ala Ser Glu Thr Ser Glu Lys
305                 310                 315                 320

Arg Pro Phe Met Cys Ala Tyr Pro Gly Cys Asn Lys Arg Tyr Phe Lys
                325                 330                 335

Leu Ser His Leu Gln Met His Ser Arg Lys His Thr Gly Glu Lys Pro
            340                 345                 350

Tyr Gln Cys Asp Phe Lys Asp Cys Glu Arg Arg Phe Ser Arg Ser Asp
        355                 360                 365

Gln Leu Lys Arg His Gln Arg Arg His Thr Gly Val Lys Pro Phe Gln
    370                 375                 380

Cys Lys Thr Cys Gln Arg Lys Phe Ser Arg Ser Asp His Leu Lys Thr
385                 390                 395                 400

His Thr Arg Thr His Thr Gly Lys Thr Ser Glu Lys Pro Phe Ser Cys
                405                 410                 415

Arg Trp Pro Ser Cys Gln Lys Lys Phe Ala Arg Ser Asp Glu Leu Val
            420                 425                 430

Arg His His Asn Met His Gln Arg Asn Met Thr Lys Leu Gln Leu Ala
        435                 440                 445

Leu
```

<210> 5
<211> 458
<212> DNA
<213> Human papillomavirus type 16

<220>

<221> CDS
<222> (3) .. (455)

<400> 5

```
ca atg ttt cag gac cca cag gag cga ccc gga aag tta cca cag tta      47
   Met Phe Gln Asp Pro Gln Glu Arg Pro Gly Lys Leu Pro Gln Leu
   1               5                   10                  15

tgc aca gag ctg caa aca act ata cat gat ata ata tta gaa tgt gtg     95
Cys Thr Glu Leu Gln Thr Thr Ile His Asp Ile Ile Leu Glu Cys Val
                20                  25                  30

tac tgc aag caa cag tta ctg cga cgt gag gta tat gac ttt gct ttt     143
Tyr Cys Lys Gln Gln Leu Leu Arg Arg Glu Val Tyr Asp Phe Ala Phe
            35                  40                  45

cgg gat tta tgc ata gta tat aga gat ggg aat cca tat gct gta tgt     191
Arg Asp Leu Cys Ile Val Tyr Arg Asp Gly Asn Pro Tyr Ala Val Cys
        50                  55                  60

gat aaa tgt tta aag ttt tat tct aaa att agt gag tat aga cat tat     239
Asp Lys Cys Leu Lys Phe Tyr Ser Lys Ile Ser Glu Tyr Arg His Tyr
    65                  70                  75

tgt tat agt gtg tat gga aca aca tta gaa cag caa tac aac aaa ccg     287
Cys Tyr Ser Val Tyr Gly Thr Thr Leu Glu Gln Gln Tyr Asn Lys Pro
80                  85                  90                  95

ttg tgt gat ttg tta att agg tgt att aac tgt caa aag cca ctg tgt     335
Leu Cys Asp Leu Leu Ile Arg Cys Ile Asn Cys Gln Lys Pro Leu Cys
                100                 105                 110

cct gaa gaa aag caa aga cat ctg gac aaa aag caa aga ttc cat aat     383
Pro Glu Glu Lys Gln Arg His Leu Asp Lys Lys Gln Arg Phe His Asn
            115                 120                 125

ata agg ggt cgg tgg acc ggt cga tgt atg tct tgt tgc aga tca tca     431
Ile Arg Gly Arg Trp Thr Gly Arg Cys Met Ser Cys Cys Arg Ser Ser
        130                 135                 140

aga aca cgt aga gaa acc cag ctg taa                                 458
Arg Thr Arg Arg Glu Thr Gln Leu
    145                 150
```

<210> 6
<211> 151
<212> PRT
<213> Human papillomavirus type 16

<400> 6

```
Met Phe Gln Asp Pro Gln Glu Arg Pro Gly Lys Leu Pro Gln Leu Cys
 1               5                  10                  15

Thr Glu Leu Gln Thr Thr Ile His Asp Ile Ile Leu Glu Cys Val Tyr
            20                  25                  30

Cys Lys Gln Gln Leu Leu Arg Arg Glu Val Tyr Asp Phe Ala Phe Arg
        35                  40                  45

Asp Leu Cys Ile Val Tyr Arg Asp Gly Asn Pro Tyr Ala Val Cys Asp
    50                  55                  60

Lys Cys Leu Lys Phe Tyr Ser Lys Ile Ser Glu Tyr Arg His Tyr Cys

65                  70                  75                  80

Tyr Ser Val Tyr Gly Thr Thr Leu Glu Gln Gln Tyr Asn Lys Pro Leu
                85                  90                  95

Cys Asp Leu Leu Ile Arg Cys Ile Asn Cys Gln Lys Pro Leu Cys Pro
            100                 105                 110

Glu Glu Lys Gln Arg His Leu Asp Lys Lys Gln Arg Phe His Asn Ile
        115                 120                 125

Arg Gly Arg Trp Thr Gly Arg Cys Met Ser Cys Cys Arg Ser Ser Arg
    130                 135                 140

Thr Arg Arg Glu Thr Gln Leu
145                 150
```

<210> 7
<211> 540
<212> DNA
<213> Artificial Sequence

<220>
<221> CDS
<222> (82)..(375)

<220>
<223> Description of Artificial Sequence: fragment of human papilloma virus type 16 E7 gene

<400> 7

```
taatataagg ggtcggtgga ccggtcgatg tatgtcttgt tgcagatcat caagaacacg 60

tagagaaacc cagctgtaat c atg cat gga gat aca cct aca ttg cat gaa   111
                        Met His Gly Asp Thr Pro Thr Leu His Glu
                         1               5                  10

tat atg tta gat ttg caa cca gag aca act gat ctc tac tgt tat gag   159
Tyr Met Leu Asp Leu Gln Pro Glu Thr Thr Asp Leu Tyr Cys Tyr Glu
                15                  20                  25

caa tta aat gac agc tca gag gag gag gat gaa ata gat ggt cca gct   207
Gln Leu Asn Asp Ser Ser Glu Glu Glu Asp Glu Ile Asp Gly Pro Ala
            30                  35                  40

gga caa gca gaa ccg gac aga gcc cat tac aat att gta acc ttt tgt   255
Gly Gln Ala Glu Pro Asp Arg Ala His Tyr Asn Ile Val Thr Phe Cys
        45                  50                  55

tgc aag tgt gac tct acg ctt cgg ttg tgc gta caa agc aca cac gta   303
Cys Lys Cys Asp Ser Thr Leu Arg Leu Cys Val Gln Ser Thr His Val
     60                  65                  70

gac att cgt act ttg gaa gac ctg tta atg ggc aca cta gga att gtg   351
Asp Ile Arg Thr Leu Glu Asp Leu Leu Met Gly Thr Leu Gly Ile Val
 75                  80                  85                  90

tgc ccc atc tgt tct cag aaa cca taatctacca tggctgatcc tgcaggtacc  405
Cys Pro Ile Cys Ser Gln Lys Pro
                95

aatggggaag agggtacggg atgtaatgga tggttttatg tagaggctgt agtggaaaaa 465

aaaacagggg atgctatatc agatgacgag aacgaaaatg acagtgatac aggtgaagat 525

ttggtagatt ttata                                                  540
```

<210> 8
<211> 98
<212> PRT
<213> Artificial Sequence
<223> Description of Artificial Sequence: fragment of human papilloma virus type 16 E7 gene

<400> 8

```
Met His Gly Asp Thr Pro Thr Leu His Glu Tyr Met Leu Asp Leu Gln
 1               5                  10                  15

Pro Glu Thr Thr Asp Leu Tyr Cys Tyr Glu Gln Leu Asn Asp Ser Ser
            20                  25                  30

Glu Glu Glu Asp Glu Ile Asp Gly Pro Ala Gly Gln Ala Glu Pro Asp
        35                  40                  45

Arg Ala His Tyr Asn Ile Val Thr Phe Cys Cys Lys Cys Asp Ser Thr
    50                  55                  60

Leu Arg Leu Cys Val Gln Ser Thr His Val Asp Ile Arg Thr Leu Glu
65                  70                  75                  80

Asp Leu Leu Met Gly Thr Leu Gly Ile Val Cys Pro Ile Cys Ser Gln
                85                  90                  95

Lys Pro
```

<210> 9
<211> 2627
<212> DNA
<213> Human immunodeficiency virus

<400> 9

```
gaattcagag cagaagacag tggcaatgag agtgaagggg atcaggagga attatcagca 60
ctggtgggga tggggcacga tgctccttgg gttattaatg atctgtagtg ctacagaaaa 120
attgtgggtc acagtctatt atggggtacc tgtgtggaaa gaagcaacca ccactctatt 180
ttgtgcatca gatgctaaag catatgatac agaggtacat aatgtttggg ccacacatgc 240
ctgtgtaccc acagacccca acccacaaga agtaaaattg gtaaatgtga cagaaaattt 300
taacatgtgg aaaaataacg tggtagaaca gatgcatgag gatataatca gtttatggga 360
tcaaagccta aagccatgtg taaaattaac cccactctgt gttactttaa attgcactga 420
tttgaggaat actactaata ccaataatag tactgctaat aacaatagta atagcgaggg 480
aacaataaag ggaggagaaa tgaaaaactg ctctttcaat atcaccacaa gcataagaga 540
taagatgcag aaagaatatg cacttcttta taaacttgat atagtatcaa tagataatga 600
tagtaccagc tataggttga taagttgtaa tacctcagtc attacacaag cttgtccaaa 660
gatatccttt gagccaattc ccatacacta ttgtgccccg gctggttttg cgattctaaa 720
gtgtaacgta aaaaagttca gtggaaaagg atcatgtaaa aatgtcagca cagtacaatg 780
tacacatgga attaggccag tagtatcagt tcaactgctg ttaaatggca gtctagcaga 840
agaagaggta gtaattagat ctgagaattt cactgataat gctaaaacca tcatagtaca 900
tctgaatgaa tctgtacaaa ttaattgtac aagacccaac tacaataaaa gaaaaaggat 960
acatatagga ccagggagag cattttatac aacaaaaaat ataaaaggaa ctataagaca 1020
agcacattgt aacattagta gagcaaaatg gaatgacact ttaagacaga tagttagcaa 1080
attaaaagaa caatttaaga ataaaacaat agtctttact caatcctcag gaggggaccc 1140
```

```
agaaattgta atgcacagtt ttaattgtgg aggggaattt ttctactgta atacatcacc 1200
actgtttaat agtacttgga atggtaataa tacttggaat aatactacgg ggtcaaataa 1260
caatatcaca cttcaatgca aaataaaaca aattataaac atgtggcaga aagtaggaaa 1320
agcaatgtat gcccctccca ttgaaggaca aattagatgt tcatcaaata ttacagggct 1380
actattaaca agagatggtg gtaaggacac ggacacgaac gacaccgaga tcttcagacc 1440
tggaggagga gatatgaggg acaattggag aagtgaatta tataaatata aagtagtaac 1500
aattgaacca ttaggagtag cacccaccaa ggcaaagaga agagtggtgc agagagaaaa 1560
aagagcagcg ataggagctc tgttccttgg gttcttagga gcggcaggaa gcactatggg 1620
cgcagcgtca gtgacgctga cggtacaggc cagactatta ttgtctggta tagtgcaaca 1680
gcagaacaat ttgctgaggg ccattgagtc gcaacagcat atgttgcaac tcacagtctg 1740
gggcatcaag cagctccagg caagagtcct ggctgtggaa agatacctaa aggatcaaca 1800
gctcctgggg ttttggggtt gctctggaaa actcatttgc accactactg tgccttggaa 1860
tgctagttgg agtaataaat ctctggatga tatttggaat aacatgacct ggatgcagtg 1920
ggaaagagaa attgacaatt acacaagctt aatatactca ttactagaaa aatcgcaaac 1980
ccaacaagaa aagaatgaac aagaattatt ggaattggat aaatgggcaa gtttgtggaa 2040
ttggtttgac ataacaaatt ggctgtggta tataaaaata ttcataatga tagtaggagg 2100
ctggtaggtt taagaatagt ttttactgta ctttctatag tgaatagagt taggcaggga 2160
tactcaccat tgtcgttgca gacccgcccc ccagttccga ggggacccga caggcccgaa 2220
ggaatcgaag aagaaggtgg agagagagac agagacacat ccggtcgatt agtgcatgga 2280
ttcttagcaa ttatctgggt cgacctgcgg agcctgttcc tcttcagcta ccaccacttg 2340
agagacttac tcttgattgc agcgaggagt gtggaacttc tgggacgcag gggtgggaa 2400
gtcctcaaat attggtggaa tctcctacag tattggagtc aggaactaaa gaatagtgct 2460
gttagcttgc ttaatgccac agctatagca gtagctgagg ggacagatag ggttatagaa 2520
gtactgcaaa gagctggtag agctattctc cacataccta caagaataag acagggcttg 2580
gaaagggctt tgctataaga tgggtggcaa gtggtcaaag cggccgc              2627
```

<210> 10
<211> 1564
<212> DNA
<213> Human immunodeficiency virus

<400> 10

gaattccatg ggtgcgagag cgtcggtatt aagcgggga gaattagatc gatgggaaaa 60
aattcggtta aggccagggg gaaagaaaaa atataaatta aaacatgtag tatgggcaag 120
cagggagcta gaacgattcg cagtcaatcc tggcctgtta gaaacatcag aaggctgtag 180
acaaatactg ggacagctac aaccatccct tcagacagga tcagaagaac gtaaatcatt 240
atataataca gtagcaaccc tctattgtgt gcatcaaaag atagagataa aagacaccaa 300
ggaagcttta gagaaaatag aggaagagca aaacaaaagt aagaaaaaag cacagcaagc 360
agcagctgac acaggaaaca gaggaaacag cagccaagtc agccaaaatt accccatagt 420
gcagaacatc caggggcaaa tggtacatca ggccatatca cctagaactt taaatgcatg 480
ggtaaaagta gtagaagaga aggctttcag cccagaagta atacccatgt tttcagcatt 540
atcagaagga gccaccccac aagatttaaa caccatgcta aacacagtgg ggggacatca 600
agcagccatg caaatgttaa aagagaccat caatgaggaa gctgcagaat gggatagatt 660
gcatccagtg catgcagggc ctattgcacc aggccagatg agagaaccaa ggggaagtga 720
catagcagga actactagta cccttcagga acaaatagga tggatgacaa ataatccacc 780
tatcccagta ggagaaatct ataaaagatg gataatcctg ggattaaata aaatagtaag 840
gatgtatagc ccttccagca tcctggacat aagacaagga ccaaaggaac cctttagaga 900
ctatgtagac cggttctata aaactctaag agccgagcaa gcttcacagg aggtaaaaaa 960
ttggatgaca gaaaccttgt tggtccaaaa tgcgaaccca gattgtaaga ctattttaaa 1020
agcattggga ccagcagcta cactagaaga aatgatgaca gcatgtcagg gagtgggagg 1080
acctggtcat aaagcaggag ttttggcgga agcgatgagc caagtaacaa attcagctac 1140
cataatggtg cagagaggca attttaggaa tcaaagaaag attatcaagt gcttcaattg 1200
tggcaaagaa gggcacatag ccaaaaattg cagggcccct aggaaaaggg gctgttggaa 1260
atgtggaaag gaaggacacc aaatgaaaga ttgtactgag ggacaggcta atttttttagg 1320
gaagatctgg ccttcctgca agggaaggcc agggaatttt cctcagagca gaacagagcc 1380
aacagcccca ccagaagaga gcttcaggtt tggggaagag acaacaactc cctaccagaa 1440
gcaggagaag aagcaggaga caatagacaa ggacctgtat cctttagctt ccctcaaatc 1500
actctttggc aacgacccat tgtcacaata agcggccgct cgagtctaga gggcccgttt 1560
aaac 1564

<210> 11
<211> 318
<212> DNA
<213> Human immunodeficiency virus

<400> 11

gaattcatgg agccagtaga tcctaaacta gagccctgga agcatccagg aagtcagcct 60
aagactgctt gtaacaattg ctattgtaaa aagtgttgct ttcattgcca agtttgtttc 120
acaaaaaaag gcttaggcat ctcctatggc aggaagaagc ggagacagcg acgaagatct 180
cctcaagaca gtgagactca tcaagtttct ctatcaaagc aacccgcctc ccagccccga 240
ggggacccga caggcccgaa ggaatcgaag aagaaggttg agagagagac agagacagat 300
ccggtcgatt aggtcgac 318

<210> 12
<211> 318
<212> DNA
<213> Human immunodeficiency virus

<400> 12

gaattcatgg agccagtaga tcctaaacta gagccctgga agcatccagg aagtcagcct 60
aagactgctt gtaacaattg ctattcgaaa aagtgttgct ttcattgcca agtttgtttc 120
acaaaaaaag gcttaggcat ctcctatggc aggaagacgc ggagacagct gcgaagatct 180
cctcaagaca gtgagactca tcaagtttct ctatcaaagc aacccgcctc ccagccccga 240
ggggacccga caggcccgaa ggaatcgaag aagaaggtgg agagagagac agagacagat 300
ccggtcgatt aggtcgac 318

<210> 13
<211> 742
<212> DNA
<213> Human immunodeficiency virus

<400> 13

```
cctagaagaa taagacaggg ctttgaagcc gtgttgctat aaaatggggg gcaaatggtc 60
aaaatccagt atagttgggt ggcctgcagt aagagacaaa ataagaggaa ctgatccagc 120
agcaaaggga gtaggagcag cgtctcaaga cttagataaa tatggggcac ttacaagcag 180
caacacaccc gccaataatg ctgattgtgc ctggctggaa gcgcaagagg aggaaggaga 240
agtaggcttt ccagtcagac ctcaggtacc tttaagacca atgacttata agggagcatt 300
cgatctcggc ttctttttaa aagaaaaggg gggactggaa ggggtaattt actccaagaa 360
aaggcaagag atccttgatt tgtgggtcta tcacacacaa ggcttcttcc ctgattggca 420
aaactacaca ccaggaccag ggtcagata tccattgacc tttggatggt gcttcaagct 480
agtgccagtt gacccaaggg aagtggaaga ggccaatgaa ggagaggaca actgcttact 540
gcatcctatg agcctgcatg gaatggagga tgcacacgga gaagtattaa ggtggaagta 600
tgacagtaaa ctagcacgca gacacatggc ccgcgagcta catccggagt attacaaaga 660
ctgctgacac agaagggact ttccgctggg actttccact ggggcgttcc aggaggagtg 720
gtctgggcgg gactgggagt gg                                          742
```

<210> 14
<211> 394
<212> DNA
<213> Human immunodeficiency virus

<400> 14

```
cccgggcatc tcctatggca ggaagaagcg gagacagcga cgaagagctc ctcaaggcag 60
tcagactcat caagtttctc tatcaaagca acccacctcc caatcccgag gggacccgac 120
aggcccgaag gaatagaaga agaaggtgga gagagagaca gagacagatc cattcgatta 180
gtgaacggat ccttagcact tatctgggac gatctgcgga gcctgtgcct cttcagctac 240
caccgcttga gagacttact cttgattgta acgaggattg tggaacttct gggacgcagg 300
gggtgggaag ccctcaaata ttggtggaat ctcctacaat attggagtca ggagctaaag 360
aatagtgctg ttagcttgct caatgccatc taga                              394
```

<210> 15
<211> 1524
<212> DNA
<213> Homo sapiens

<220>
<221> CDS
<222> (54) .. (407)

<400> 15

```
agcagacaga ggactctcat taaggaaggt gtcctgtgcc ctgaccctac aag atg    56
                                                           Met
                                                             1

cca aga gaa gat gct cac ttc atc tat ggt tac ccc aag aag ggg cac  104
Pro Arg Glu Asp Ala His Phe Ile Tyr Gly Tyr Pro Lys Lys Gly His
                5                  10                  15

ggc cac tct tac acc acg gct gaa gag gcc gct ggg atc ggc atc ctg  152
Gly His Ser Tyr Thr Thr Ala Glu Glu Ala Ala Gly Ile Gly Ile Leu
            20                  25                  30

aca gtg atc ctg gga gtc tta ctg ctc atc ggc tgt tgg tat tgt aga  200
Thr Val Ile Leu Gly Val Leu Leu Leu Ile Gly Cys Trp Tyr Cys Arg
        35                  40                  45

aga cga aat gga tac aga gcc ttg atg gat aaa agt ctt cat gtt ggc  248
Arg Arg Asn Gly Tyr Arg Ala Leu Met Asp Lys Ser Leu His Val Gly
 50                  55                  60                  65

act caa tgt gcc tta aca aga aga tgc cca caa gaa ggg ttt gat cat  296
Thr Gln Cys Ala Leu Thr Arg Arg Cys Pro Gln Glu Gly Phe Asp His
                 70                  75                  80

cgg gac agc aaa gtg tct ctt caa gag aaa aac tgt gaa cct gtg gtt  344
Arg Asp Ser Lys Val Ser Leu Gln Glu Lys Asn Cys Glu Pro Val Val
                85                  90                  95

ccc aat gct cca cct gct tat gag aaa ctc tct gca gaa cag tca cca  392
Pro Asn Ala Pro Pro Ala Tyr Glu Lys Leu Ser Ala Glu Gln Ser Pro
            100                 105                 110

cca cct tat tca cct taagagccag cgagacacct gagacatgct gaaattattt  447
Pro Pro Tyr Ser Pro
    115

ctctcacact tttgcttgaa tttaatacag acatctaatg ttctcctttg gaatggtgta 507

ggaaaaatgc aagccatctc taataataag tcagtgttaa aattttagta ggtccgctag 567

cagtactaat catgtgagga aatgatgaga aatattaaat tgggaaaact ccatcaataa 627

atgttgcaat gcatgatact atctgtgcca gaggtaatgt tagtaaatcc atggtgttat 687

tttctgagag acagaattca agtgggtatt ctggggccat ccaatttctc tttacttgaa 747

atttggctaa taacaaacta gtcaggtttt cgaaccttga ccgacatgaa ctgtacacag 807

aattgttcca gtactatgga gtgctcacaa aggatacttt tacaggttaa gacaaagggt 867

tgactggcct atttatctga tcaagaacat gtcagcaatg tctctttgtg ctctaaaatt 927

ctattatact acaataatat attgtaaaga tcctatagct cttttttttt gagatggagt 987
```

```
ttcgcttttg ttgcccaggc tggagtgcaa tggcgcgatc ttggctcacc ataacctccg 1047
cctcccaggt tcaagcaatt ctcctgcctt agcctcctga gtagctggga ttacaggcgt 1107
gcgccactat gcctgactaa ttttgtagtt ttagtagaga cggggtttct ccatgttggt 1167
caggctggtc tcaaactcct gacctcaggt gatctgcccg cctcagcctc ccaaagtgct 1227
ggaattacag gcgtgagcca ccacgcctgg ctggatccta tatcttaggt aagacatata 1287
acgcagtcta attacatttc acttcaaggc tcaatgctat tctaactaat gacaagtatt 1347
ttctactaaa ccagaaattg gtagaaggat ttaaataagt aaaagctact atgtactgcc 1407
ttagtgctga tgcctgtgta ctgccttaaa tgtacctatg gcaatttagc tctcttgggt 1467
tcccaaatcc ctctcacaag aatgtgcaga agaaatcata aaggatcaga gattctg    1524
```

<210> 16
<211> 118
<212> PRT
<213> Homo sapiens

<400> 16

```
Met Pro Arg Glu Asp Ala His Phe Ile Tyr Gly Tyr Pro Lys Lys Gly
1               5                   10                  15

His Gly His Ser Tyr Thr Thr Ala Glu Glu Ala Ala Gly Ile Gly Ile
            20                  25                  30

Leu Thr Val Ile Leu Gly Val Leu Leu Leu Ile Gly Cys Trp Tyr Cys
        35                  40                  45

Arg Arg Arg Asn Gly Tyr Arg Ala Leu Met Asp Lys Ser Leu His Val
    50                  55                  60

Gly Thr Gln Cys Ala Leu Thr Arg Arg Cys Pro Gln Glu Gly Phe Asp
65                  70                  75                  80

His Arg Asp Ser Lys Val Ser Leu Gln Glu Lys Asn Cys Glu Pro Val
                85                  90                  95

Val Pro Asn Ala Pro Pro Ala Tyr Glu Lys Leu Ser Ala Glu Gln Ser
            100                 105                 110

Pro Pro Pro Tyr Ser Pro
        115
```

<210> 17
<211> 1722
<212> DNA
<213> Homo sapiens

<220>
<221> CDS
<222> (188)..(1114)

<400> 17

cgtagagttc ggccgaagga acctgaccca ggctctgtga ggaggcaagg ttttcagggg 60

```
acaggccaac ccagaggaca ggattccctg gaggccacag aggagcacca aggagaagat 120

ctgcctgtgg gtcttcattg cccagctcct gcccacactc ctgcctgctg ccctgacgag 180

agtcatc atg tct ctt gag cag agg agt ctg cac tgc aag cct gag gaa    229
        Met Ser Leu Glu Gln Arg Ser Leu His Cys Lys Pro Glu Glu
        1               5                   10

gcc ctt gag gcc caa caa gag gcc ctg ggc ctg gtg tgt gtg cag gct    277
Ala Leu Glu Ala Gln Gln Glu Ala Leu Gly Leu Val Cys Val Gln Ala
15                  20                  25                  30

gcc gcc tcc tcc tcc tct cct ctg gtc ctg ggc acc ctg gag gag gtg    325
Ala Ala Ser Ser Ser Ser Pro Leu Val Leu Gly Thr Leu Glu Glu Val
                35                  40                  45

ccc act gct ggg tca aca gat cct ccc cag agt cct cag gga gcc tcc    373
Pro Thr Ala Gly Ser Thr Asp Pro Pro Gln Ser Pro Gln Gly Ala Ser
            50                  55                  60

gcc ttt ccc act acc atc aac ttc act cga cag agg caa ccc agt gag    421
Ala Phe Pro Thr Thr Ile Asn Phe Thr Arg Gln Arg Gln Pro Ser Glu
        65                  70                  75

ggt tcc agc agc cgt gaa gag gag ggg cca agc acc tct tgt atc ctg    469
Gly Ser Ser Ser Arg Glu Glu Glu Gly Pro Ser Thr Ser Cys Ile Leu
    80                  85                  90

gag tcc ttg ttc cga gca gta atc act aag aag gtg gct gat ttg gtt    517
Glu Ser Leu Phe Arg Ala Val Ile Thr Lys Lys Val Ala Asp Leu Val
95                  100                 105                 110

ggt ttt ctg ctc ctc aaa tat cga gcc agg gag cca gtc aca aag gca    565
Gly Phe Leu Leu Leu Lys Tyr Arg Ala Arg Glu Pro Val Thr Lys Ala
                115                 120                 125

gaa atg ctg gag agt gtc atc aaa aat tac aag cac tgt ttt cct gag    613
Glu Met Leu Glu Ser Val Ile Lys Asn Tyr Lys His Cys Phe Pro Glu
            130                 135                 140

atc ttc ggc aaa gcc tct gag tcc ttg cag ctg gtc ttt ggc att gac    661
Ile Phe Gly Lys Ala Ser Glu Ser Leu Gln Leu Val Phe Gly Ile Asp
        145                 150                 155

gtg aag gaa gca gac ccc acc ggc cac tcc tat gtc ctt gtc acc tgc    709
Val Lys Glu Ala Asp Pro Thr Gly His Ser Tyr Val Leu Val Thr Cys
    160                 165                 170

cta ggt ctc tcc tat gat ggc ctg ctg ggt gat aat cag atc atg ccc    757
Leu Gly Leu Ser Tyr Asp Gly Leu Leu Gly Asp Asn Gln Ile Met Pro
175                 180                 185                 190

aag aca ggc ttc ctg ata att gtc ctg gtc atg att gca atg gag ggc    805
Lys Thr Gly Phe Leu Ile Ile Val Leu Val Met Ile Ala Met Glu Gly
                195                 200                 205

ggc cat gct cct gag gag gaa atc tgg gag gag ctg agt gtg atg gag    853
Gly His Ala Pro Glu Glu Glu Ile Trp Glu Glu Leu Ser Val Met Glu
            210                 215                 220

gtg tat gat ggg agg gag cac agt gcc tat ggg gag ccc agg aag ctg    901
Val Tyr Asp Gly Arg Glu His Ser Ala Tyr Gly Glu Pro Arg Lys Leu
```

```
        225                 230                 235

ctc acc caa gat ttg gtg cag gaa aag tac ctg gag tac cgg cag gtg    949
Leu Thr Gln Asp Leu Val Gln Glu Lys Tyr Leu Glu Tyr Arg Gln Val
    240                 245                 250

ccg gac agt gat ccc gca cgc tat gag ttc ctg tgg ggt cca agg gcc    997
Pro Asp Ser Asp Pro Ala Arg Tyr Glu Phe Leu Trp Gly Pro Arg Ala
255                 260                 265                 270

ctt gct gaa acc agc tat gtg aaa gtc ctt gag tat gtg atc aag gtc   1045
Leu Ala Glu Thr Ser Tyr Val Lys Val Leu Glu Tyr Val Ile Lys Val
                275                 280                 285

agt gca aga gtt cgc ttt ttc ttc cca tcc ctg cgt gaa gca gct ttg   1093
Ser Ala Arg Val Arg Phe Phe Phe Pro Ser Leu Arg Glu Ala Ala Leu
            290                 295                 300

aga gag gag gaa gag gga gtc tgagcatgag ttgcagccag ggccagtggg      1144
Arg Glu Glu Glu Glu Gly Val
        305

agggggactg ggccagtgca ccttccaggg ccgcgtccag cagcttcccc tgcctcgtgt 1204

gacatgaggc ccattcttca ctctgaagag agcggtcagt gttctcagta gtaggtttct 1264

gttctattgg gtgacttgga gatttatctt tgttctcttt tggaattgtt caaatgtttt 1324

tttttaaggg atggttgaat gaacttcagc atccaagttt atgaatgaca gcagtcacac 1384

agttctgtgt atatagttta agggtaagag tcttgtgttt tattcagatt gggaaatcca 1444

ttctattttg tgaattggga taataacagc agtggaataa gtacttagaa atgtgaaaaa 1504

tgagcagtaa aatagatgag ataaagaact aaagaaatta agagatagtc aattcttgct 1564

ttatacctca gtctattctg taaaattttt aaagatatat gcatacctgg atttccttgg 1624

cttctttgag aatgtaagag aaattaaatc tgaataaaga attcttcctg ttaaaaaaaa 1684

aaaaaaaaaa aaaaaaaaaa aaaaaaaaaa aaaaaaa                          1722
```

<210> 18
<211> 309
<212> PRT
<213> Homo sapiens

<400> 18

Met Ser Leu Glu Gln Arg Ser Leu His Cys Lys Pro Glu Glu Ala Leu
1 5 10 15

Glu Ala Gln Gln Glu Ala Leu Gly Leu Val Cys Val Gln Ala Ala Ala
20 25 30

Ser Ser Ser Ser Pro Leu Val Leu Gly Thr Leu Glu Glu Val Pro Thr
35 40 45

Ala Gly Ser Thr Asp Pro Pro Gln Ser Pro Gln Gly Ala Ser Ala Phe
50 55 60

Pro Thr Thr Ile Asn Phe Thr Arg Gln Arg Gln Pro Ser Glu Gly Ser
65 70 75 80

Ser Ser Arg Glu Glu Glu Gly Pro Ser Thr Ser Cys Ile Leu Glu Ser
85 90 95

Leu Phe Arg Ala Val Ile Thr Lys Lys Val Ala Asp Leu Val Gly Phe
100 105 110

Leu Leu Leu Lys Tyr Arg Ala Arg Glu Pro Val Thr Lys Ala Glu Met
115 120 125

Leu Glu Ser Val Ile Lys Asn Tyr Lys His Cys Phe Pro Glu Ile Phe
130 135 140

Gly Lys Ala Ser Glu Ser Leu Gln Leu Val Phe Gly Ile Asp Val Lys
145 150 155 160

Glu Ala Asp Pro Thr Gly His Ser Tyr Val Leu Val Thr Cys Leu Gly
165 170 175

Leu Ser Tyr Asp Gly Leu Leu Gly Asp Asn Gln Ile Met Pro Lys Thr
180 185 190

Gly Phe Leu Ile Ile Val Leu Val Met Ile Ala Met Glu Gly Gly His
195 200 205

Ala Pro Glu Glu Glu Ile Trp Glu Glu Leu Ser Val Met Glu Val Tyr
210 215 220

Asp Gly Arg Glu His Ser Ala Tyr Gly Glu Pro Arg Lys Leu Leu Thr
225 230 235 240

Gln Asp Leu Val Gln Glu Lys Tyr Leu Glu Tyr Arg Gln Val Pro Asp
245 250 255

Ser Asp Pro Ala Arg Tyr Glu Phe Leu Trp Gly Pro Arg Ala Leu Ala
260 265 270

Glu Thr Ser Tyr Val Lys Val Leu Glu Tyr Val Ile Lys Val Ser Ala
275 280 285

Arg Val Arg Phe Phe Phe Pro Ser Leu Arg Glu Ala Ala Leu Arg Glu
290 295 300

Glu Glu Glu Gly Val
305

<210> 19
<211> 1753
<212> DNA
<213> Homo sapiens

<220>
<221> CDS
<222> (210)..(1151)

<400> 19

gagattctcg ccctgagcaa cgagcgacgg cctgacgtcg gcggagggaa gccggcccag 60

gctcggtgag gaggcaaggt tctgagggga caggctgacc tggaggacca gaggcccccg 120

gaggagcact gaaggagaag atctgccagt gggtctccat tgcccagctc ctgcccacac 180

```
tcccgcctgt tgccctgacc agagtcatc atg cct ctt gag cag agg agt cag   233
                                Met Pro Leu Glu Gln Arg Ser Gln
                                 1               5

cac tgc aag cct gaa gaa ggc ctt gag gcc cga gga gag gcc ctg ggc   281
His Cys Lys Pro Glu Glu Gly Leu Glu Ala Arg Gly Glu Ala Leu Gly
    10                  15                  20

ctg gtg ggt gcg cag gct cct gct act gag gag cag gag gct gcc tcc   329
Leu Val Gly Ala Gln Ala Pro Ala Thr Glu Glu Gln Glu Ala Ala Ser
25                  30                  35                  40

tcc tct tct act cta gtt gaa gtc acc ctg ggg gag gtg cct gct gcc   377
Ser Ser Ser Thr Leu Val Glu Val Thr Leu Gly Glu Val Pro Ala Ala
                45                  50                  55

gag tca cca gat cct ccc cag agt cct cag gga gcc tcc agc ctc ccc   425
Glu Ser Pro Asp Pro Pro Gln Ser Pro Gln Gly Ala Ser Ser Leu Pro
            60                  65                  70

act acc atg aac tac cct ctc tgg agc caa tcc tat gag gac tcc agc   473
Thr Thr Met Asn Tyr Pro Leu Trp Ser Gln Ser Tyr Glu Asp Ser Ser
        75                  80                  85

aac caa gaa gag gag ggg cca agc acc ttc cct gac ctg gag tcc gag   521
Asn Gln Glu Glu Glu Gly Pro Ser Thr Phe Pro Asp Leu Glu Ser Glu
    90                  95                  100

ttc caa gca gca ctc agt agg aag gtg gcc gag ttg gtt cat ttt ctg   569
Phe Gln Ala Ala Leu Ser Arg Lys Val Ala Glu Leu Val His Phe Leu
105                 110                 115                 120

ctc ctc aag tat cga gcc agg gag ccg gtc aca aag gca gaa atg ctg   617
Leu Leu Lys Tyr Arg Ala Arg Glu Pro Val Thr Lys Ala Glu Met Leu
                125                 130                 135

ggg agt gtc gtc gga aat tgg cag tat ttc ttt cct gtg atc ttc agc   665
Gly Ser Val Val Gly Asn Trp Gln Tyr Phe Phe Pro Val Ile Phe Ser
            140                 145                 150

aaa gct tcc agt tcc ttg cag ctg gtc ttt ggc atc gag ctg atg gaa   713
Lys Ala Ser Ser Ser Leu Gln Leu Val Phe Gly Ile Glu Leu Met Glu
        155                 160                 165

gtg gac ccc atc ggc cac ttg tac atc ttt gcc acc tgc ctg ggc ctc   761
Val Asp Pro Ile Gly His Leu Tyr Ile Phe Ala Thr Cys Leu Gly Leu
    170                 175                 180

tcc tac gat ggc ctg ctg ggt gac aat cag atc atg ccc aag gca ggc   809
Ser Tyr Asp Gly Leu Leu Gly Asp Asn Gln Ile Met Pro Lys Ala Gly
185                 190                 195                 200

ctc ctg ata atc gtc ctg gcc ata atc gca aga gag ggc gac tgt gcc   857
Leu Leu Ile Ile Val Leu Ala Ile Ile Ala Arg Glu Gly Asp Cys Ala
                205                 210                 215

cct gag gag aaa atc tgg gag gag ctg agt gtg tta gag gtg ttt gag   905
Pro Glu Glu Lys Ile Trp Glu Glu Leu Ser Val Leu Glu Val Phe Glu
            220                 225                 230

ggg agg gaa gac agt atc ttg ggg gat ccc aag aag ctg ctc acc caa   953
Gly Arg Glu Asp Ser Ile Leu Gly Asp Pro Lys Lys Leu Leu Thr Gln
```

```
        235                 240                 245

cat ttc gtg cag gaa aac tac ctg gag tac cgg cag gtc ccc ggc agt    1001
His Phe Val Gln Glu Asn Tyr Leu Glu Tyr Arg Gln Val Pro Gly Ser
    250                 255                 260

gat cct gca tgt tat gaa ttc ctg tgg ggt cca agg gcc ctc gtt gaa    1049
Asp Pro Ala Cys Tyr Glu Phe Leu Trp Gly Pro Arg Ala Leu Val Glu
265                 270                 275                 280

acc agc tat gtg aaa gtc ctg cac cat atg gta aag atc agt gga gga    1097
Thr Ser Tyr Val Lys Val Leu His His Met Val Lys Ile Ser Gly Gly
                285                 290                 295

cct cac att tcc tac cca ccc ctg cat gag tgg gtt ttg aga gag ggg    1145
Pro His Ile Ser Tyr Pro Pro Leu His Glu Trp Val Leu Arg Glu Gly
            300                 305                 310

gaa gag tgagtctgag cacgagttgc agccagggcc agtgggaggg ggtctgggcc     1201
Glu Glu

agtgcacctt ccggggccgc atcccttagt ttccactgcc tcctgtgacg tgaggcccat 1261

tcttcactct ttgaagcgag cagtcagcat tcttagtagt gggtttctgt tctgttggat 1321

gactttgaga ttattctttg tttcctgttg gagttgttca aatgttcctt ttaacggatg 1381

gttgaatgag cgtcagcatc caggtttatg aatgacagta gtcacacata gtgctgttta 1441

tatagtttag gagtaagagt cttgtttttt actcaaattg ggaaatccat tccattttgt 1501

gaattgtgac ataataatag cagtggtaaa agtatttgct taaaattgtg agcgaattag 1561

caataacata catgagataa ctcaagaaat caaaagatag ttgattcttg ccttgtacct 1621

caatctattc tgtaaaatta aacaaatatg caaaccagga tttccttgac ttctttgaga 1681

atgcaagcga aattaaatct gaataaataa ttcttcctct ccaaaaaaaa aaaaaaaaaa 1741

aaaaaaaaaa aa                                                      1753
```

<210> 20
<211> 314
<212> PRT
<213> Homo sapiens

<400> 20

```
Met Pro Leu Glu Gln Arg Ser Gln His Cys Lys Pro Glu Glu Gly Leu
 1               5                  10                  15

Glu Ala Arg Gly Glu Ala Leu Gly Leu Val Gly Ala Gln Ala Pro Ala
            20                  25                  30

Thr Glu Glu Gln Glu Ala Ala Ser Ser Ser Ser Thr Leu Val Glu Val
        35                  40                  45

Thr Leu Gly Glu Val Pro Ala Ala Glu Ser Pro Asp Pro Pro Gln Ser
    50                  55                  60

Pro Gln Gly Ala Ser Ser Leu Pro Thr Thr Met Asn Tyr Pro Leu Trp
65                  70                  75                  80

Ser Gln Ser Tyr Glu Asp Ser Ser Asn Gln Glu Glu Glu Gly Pro Ser
                85                  90                  95

Thr Phe Pro Asp Leu Glu Ser Glu Phe Gln Ala Ala Leu Ser Arg Lys
            100                 105                 110

Val Ala Glu Leu Val His Phe Leu Leu Leu Lys Tyr Arg Ala Arg Glu
        115                 120                 125

Pro Val Thr Lys Ala Glu Met Leu Gly Ser Val Val Gly Asn Trp Gln
    130                 135                 140

Tyr Phe Phe Pro Val Ile Phe Ser Lys Ala Ser Ser Ser Leu Gln Leu
145                 150                 155                 160

Val Phe Gly Ile Glu Leu Met Glu Val Asp Pro Ile Gly His Leu Tyr
                165                 170                 175

Ile Phe Ala Thr Cys Leu Gly Leu Ser Tyr Asp Gly Leu Leu Gly Asp
            180                 185                 190

Asn Gln Ile Met Pro Lys Ala Gly Leu Leu Ile Ile Val Leu Ala Ile
        195                 200                 205

Ile Ala Arg Glu Gly Asp Cys Ala Pro Glu Glu Lys Ile Trp Glu Glu
    210                 215                 220

Leu Ser Val Leu Glu Val Phe Glu Gly Arg Glu Asp Ser Ile Leu Gly
225                 230                 235                 240

Asp Pro Lys Lys Leu Leu Thr Gln His Phe Val Gln Glu Asn Tyr Leu
                245                 250                 255

Glu Tyr Arg Gln Val Pro Gly Ser Asp Pro Ala Cys Tyr Glu Phe Leu
            260                 265                 270

Trp Gly Pro Arg Ala Leu Val Glu Thr Ser Tyr Val Lys Val Leu His
        275                 280                 285

His Met Val Lys Ile Ser Gly Gly Pro His Ile Ser Tyr Pro Pro Leu
    290                 295                 300

His Glu Trp Val Leu Arg Glu Gly Glu Glu
305                 310
```

<210> 21
<211> 756
<212> DNA
<213> Homo sapiens

<220>
<221> CDS
<222> (87) .. (422)

<400> 21

```
gctggaggat gtggctgcag agcctgctgc tcttgggcac tgtggcctgc agcatctctg 60

cacccgcccg ctcgcccagc cccagc acg cag ccc tgg gag cat gtg aat gcc  113
                             Thr Gln Pro Trp Glu His Val Asn Ala
                             1               5

atc cag gag gcc cgg cgt ctc ctg aac ctg agt aga gac act gct gct   161
Ile Gln Glu Ala Arg Arg Leu Leu Asn Leu Ser Arg Asp Thr Ala Ala
10                  15                  20                  25

gag atg aat gaa aca gta gaa gtc atc tca gaa atg ttt gac ctc cag   209
Glu Met Asn Glu Thr Val Glu Val Ile Ser Glu Met Phe Asp Leu Gln
                30                  35                  40

gag ccg acc tgc cta cag acc cgc ctg gag ctg tac aag cag ggc ctg   257
Glu Pro Thr Cys Leu Gln Thr Arg Leu Glu Leu Tyr Lys Gln Gly Leu
            45                  50                  55

cgg ggc agc ctc acc aag ctc aag ggc ccc ttg acc atg atg gcc agc   305
Arg Gly Ser Leu Thr Lys Leu Lys Gly Pro Leu Thr Met Met Ala Ser
        60                  65                  70

cac tac aag cag cac tgc cct cca acc ccg gaa act tcc tgt gca acc   353
His Tyr Lys Gln His Cys Pro Pro Thr Pro Glu Thr Ser Cys Ala Thr
    75                  80                  85

cag act atc acc ttt gaa agt ttc aaa gag aac ctg aag gac ttt ctg   401
Gln Thr Ile Thr Phe Glu Ser Phe Lys Glu Asn Leu Lys Asp Phe Leu
90                  95                  100                 105

ctt gtc atc ccc ttt gac tgc tgggagccag tccaggagtg agaccggcca      452
Leu Val Ile Pro Phe Asp Cys
                110

gatgaggctg gccaagccgg ggagctgctc tctcatgaaa caagagctag aaactcagga 512

tggtcatctt ggagggacca aggggtgggc cacagccatg gtgggagtgg cctggacctg 572

ccctgggcca cactgaccct gatacaggca tggcagaaga atgggaatat tttatactga 632

cagaaatcag taatatttat atatttatat ttttaaaata tttatttatt tatttattta 692

agttcatatt ccatatttat tcaagatgtt ttaccgtaat aattattatt aaaaatatgc 752

ttct                                                              756
```

<210> 22
<211> 112

<212> PRT
<213> Homo sapiens

<400> 22

```
Thr Gln Pro Trp Glu His Val Asn Ala Ile Gln Glu Ala Arg Arg Leu
1               5                   10                  15

Leu Asn Leu Ser Arg Asp Thr Ala Ala Glu Met Asn Glu Thr Val Glu
            20                  25                  30

Val Ile Ser Glu Met Phe Asp Leu Gln Glu Pro Thr Cys Leu Gln Thr
        35                  40                  45

Arg Leu Glu Leu Tyr Lys Gln Gly Leu Arg Gly Ser Leu Thr Lys Leu
    50                  55                  60

Lys Gly Pro Leu Thr Met Met Ala Ser His Tyr Lys Gln His Cys Pro
65                  70                  75                  80

Pro Thr Pro Glu Thr Ser Cys Ala Thr Gln Thr Ile Thr Phe Glu Ser


                85                  90                  95

Phe Lys Glu Asn Leu Lys Asp Phe Leu Leu Val Ile Pro Phe Asp Cys
            100                 105                 110
```

<210> 23
<211> 825
<212> DNA
<213> Homo sapiens

<220>
<221> CDS
<222> (48) .. (515)

<400> 23

```
atcactctct ttaatcacta ctcacattaa cctcaactcc tgccaca atg tac agg    56
                                                    Met Tyr Arg
                                                     1

atg caa ctc ctg tct tgc att gca cta att ctt gca ctt gtc aca aac   104
Met Gln Leu Leu Ser Cys Ile Ala Leu Ile Leu Ala Leu Val Thr Asn
    5                   10                  15

agt gca cct act tca agt tcg aca aag aaa aca aag aaa aca cag cta   152
Ser Ala Pro Thr Ser Ser Ser Thr Lys Lys Thr Lys Lys Thr Gln Leu
 20                 25                  30                  35

caa ctg gag cat tta ctg ctg gat tta cag atg att ttg aat gga att   200
Gln Leu Glu His Leu Leu Leu Asp Leu Gln Met Ile Leu Asn Gly Ile
                40                  45                  50

aat aat tac aag aat ccc aaa ctc acc agg atg ctc aca ttt aag ttt   248
Asn Asn Tyr Lys Asn Pro Lys Leu Thr Arg Met Leu Thr Phe Lys Phe
            55                  60                  65

tac atg ccc aag aag gcc aca gaa ctg aaa cag ctt cag tgt cta gaa   296
Tyr Met Pro Lys Lys Ala Thr Glu Leu Lys Gln Leu Gln Cys Leu Glu
        70                  75                  80

gaa gaa ctc aaa cct ctg gag gaa gtg ctg aat tta gct caa agc aaa   344
Glu Glu Leu Lys Pro Leu Glu Glu Val Leu Asn Leu Ala Gln Ser Lys
    85                  90                  95

aac ttt cac tta aga ccc agg gac tta atc agc aat atc aac gta ata   392
Asn Phe His Leu Arg Pro Arg Asp Leu Ile Ser Asn Ile Asn Val Ile
100                 105                 110                 115

gtt ctg gaa cta aag gga tct gaa aca aca ttc atg tgt gaa tat gca   440
Val Leu Glu Leu Lys Gly Ser Glu Thr Thr Phe Met Cys Glu Tyr Ala
                120                 125                 130

gat gag aca gca acc att gta gaa ttt ctg aac aga tgg att acc ttt   488
Asp Glu Thr Ala Thr Ile Val Glu Phe Leu Asn Arg Trp Ile Thr Phe
            135                 140                 145

tgt caa agc atc atc tca aca cta act tgataattaa gtgcttccca         535
Cys Gln Ser Ile Ile Ser Thr Leu Thr
        150                 155

cttaaaacat atcaggcctt ctatttattt atttaaatat ttaaatttta tatttattgt 595

tgaatgtatg gttgctacct attgtaacta ttattcttaa tcttaaaact ataaatatgg 655

atcttttatg attctttttg taagccctag gggctctaaa atggtttacc ttatttatcc 715

caaaaatatt tattattatg ttgaatgtta aatatagtat ctatgtagat tggttagtaa 775

aactatttaa taaatttgat aaatataaaa aaaaaaaaca aaaaaaaaaa            825
```

<210> 24
<211> 156
<212> PRT
<213> Homo sapiens

<400> 24

```
Met Tyr Arg Met Gln Leu Leu Ser Cys Ile Ala Leu Ile Leu Ala Leu
1               5                   10                  15

Val Thr Asn Ser Ala Pro Thr Ser Ser Ser Thr Lys Lys Thr Lys Lys
            20                  25                  30

Thr Gln Leu Gln Leu Glu His Leu Leu Leu Asp Leu Gln Met Ile Leu
        35                  40                  45

Asn Gly Ile Asn Asn Tyr Lys Asn Pro Lys Leu Thr Arg Met Leu Thr
    50                  55                  60

Phe Lys Phe Tyr Met Pro Lys Lys Ala Thr Glu Leu Lys Gln Leu Gln
65                  70                  75                  80

Cys Leu Glu Glu Glu Leu Lys Pro Leu Glu Glu Val Leu Asn Leu Ala
                85                  90                  95

Gln Ser Lys Asn Phe His Leu Arg Pro Arg Asp Leu Ile Ser Asn Ile
            100                 105                 110

Asn Val Ile Val Leu Glu Leu Lys Gly Ser Glu Thr Thr Phe Met Cys
        115                 120                 125

Glu Tyr Ala Asp Glu Thr Ala Thr Ile Val Glu Phe Leu Asn Arg Trp
    130                 135                 140

Ile Thr Phe Cys Gln Ser Ile Ile Ser Thr Leu Thr
145                 150                 155
```

<210> 25
<211> 975
<212> DNA
<213> Homo sapiens

<220>
<221> CDS
<222> (1) .. (972)

<400> 25

```
atg ttc ccc agc cct gct ctc acg ccc acg ccc ttc tca gtc aaa gac      48
Met Phe Pro Ser Pro Ala Leu Thr Pro Thr Pro Phe Ser Val Lys Asp
1               5                   10                  15

atc cta aac ctg gag cag cag cag cgc agc ctg gct gcc gcc gga gag      96
Ile Leu Asn Leu Glu Gln Gln Gln Arg Ser Leu Ala Ala Ala Gly Glu
```

```
                20                      25                      30

ctc tct gcc cgc ctg gag gcg acc ctg gcg ccc tcc tcc tgc atg ctg     144
Leu Ser Ala Arg Leu Glu Ala Thr Leu Ala Pro Ser Ser Cys Met Leu
        35                  40                  45

gcc gcc ttc aag cca gag gcc tac gct ggg ccc gag gcg gct gcg ccg     192
Ala Ala Phe Lys Pro Glu Ala Tyr Ala Gly Pro Glu Ala Ala Ala Pro
    50                  55                  60

ggc ctc cca gag ctg cgc gca gag ctg ggc cgc gcg cct tca ccg gcc     240
Gly Leu Pro Glu Leu Arg Ala Glu Leu Gly Arg Ala Pro Ser Pro Ala
65                  70                  75                      80

aag tgt gcg tct gcc ttt ccc gcc gcc ccc gcc ttc tat cca cgt gcc     288
Lys Cys Ala Ser Ala Phe Pro Ala Ala Pro Ala Phe Tyr Pro Arg Ala
                85                  90                  95

tac agc gac ccc gac cca gcc aag gac cct aga gcc gaa aag aaa gag     336
Tyr Ser Asp Pro Asp Pro Ala Lys Asp Pro Arg Ala Glu Lys Lys Glu
            100                 105                 110

ctg tgc gcg ctg cag aag gcg gtg gag ctg gag aag aca gag gcg gac     384
Leu Cys Ala Leu Gln Lys Ala Val Glu Leu Glu Lys Thr Glu Ala Asp
        115                 120                 125

aac gcg gag cgg ccc cgg gcg cga cgg cgg agg aag ccg cgc gtg ctc     432
Asn Ala Glu Arg Pro Arg Ala Arg Arg Arg Arg Lys Pro Arg Val Leu
    130                 135                 140

ttc tcg cag gcg cag gtc tat gaa ctg gag cgg cgc ttc aag caa cag     480
Phe Ser Gln Ala Gln Val Tyr Glu Leu Glu Arg Arg Phe Lys Gln Gln
145                 150                 155                 160

cgg tac ctg tcg gcc ccc gaa cgc gac cag ctg gcc agc gtg ctg aaa     528
Arg Tyr Leu Ser Ala Pro Glu Arg Asp Gln Leu Ala Ser Val Leu Lys
                165                 170                 175

ctc acg tcc acg cag gtc aag atc tgg ttc cag aac cgg cgc tac aag     576
Leu Thr Ser Thr Gln Val Lys Ile Trp Phe Gln Asn Arg Arg Tyr Lys
            180                 185                 190

tgc aag cgg cag cgg cag gac cag act ctg gag ctg gtg ggg ctg ccc     624
Cys Lys Arg Gln Arg Gln Asp Gln Thr Leu Glu Leu Val Gly Leu Pro
        195                 200                 205

ccg ccg ccg ccg ccg cct gcc cgc agg atc gcg gtg cca gtg ctg gtg     672
Pro Pro Pro Pro Pro Pro Ala Arg Arg Ile Ala Val Pro Val Leu Val
    210                 215                 220

cgc gat ggc aag cca tgc cta ggg gac tcg gcg ccc tac gcg cct gcc     720
Arg Asp Gly Lys Pro Cys Leu Gly Asp Ser Ala Pro Tyr Ala Pro Ala
225                 230                 235                     240

tac ggc gtg ggc ctc aat ccc tac ggt tat aac gcc tac ccc gcc tat     768
Tyr Gly Val Gly Leu Asn Pro Tyr Gly Tyr Asn Ala Tyr Pro Ala Tyr
                245                 250                 255

ccg ggt tac ggc ggc gcg gcc tgc agc cct ggc tac agc tgc act gcc     816
Pro Gly Tyr Gly Gly Ala Ala Cys Ser Pro Gly Tyr Ser Cys Thr Ala
            260                 265                 270

gct tac ccc gcc ggg cct tcc cca gcg cag ccg gcc act gcc gcc gcc     864
```

```
Ala Tyr Pro Ala Gly Pro Ser Pro Ala Gln Pro Ala Thr Ala Ala Ala
        275                     280                     285

aac aac aac ttc gtg aac ttc ggc gtc ggg gac ttg aat gcg gtt cag      912
Asn Asn Asn Phe Val Asn Phe Gly Val Gly Asp Leu Asn Ala Val Gln
    290                     295                     300

agc ccc ggg att ccg cag agc aac tcg gga gtg tcc acg ctg cat ggt      960
Ser Pro Gly Ile Pro Gln Ser Asn Ser Gly Val Ser Thr Leu His Gly
305                     310                     315                 320

atc cga gcc tgg tag                                                  975
Ile Arg Ala Trp
```

<210> 26
<211> 324
<212> PRT
<213> Homo sapiens

<400> 26

```
Met Phe Pro Ser Pro Ala Leu Thr Pro Thr Pro Phe Ser Val Lys Asp
 1               5                  10                  15

Ile Leu Asn Leu Glu Gln Gln Gln Arg Ser Leu Ala Ala Ala Gly Glu
            20                  25                  30

Leu Ser Ala Arg Leu Glu Ala Thr Leu Ala Pro Ser Ser Cys Met Leu
        35                  40                  45

Ala Ala Phe Lys Pro Glu Ala Tyr Ala Gly Pro Glu Ala Ala Ala Pro
    50                  55                  60

Gly Leu Pro Glu Leu Arg Ala Glu Leu Gly Arg Ala Pro Ser Pro Ala
65                  70                  75                  80

Lys Cys Ala Ser Ala Phe Pro Ala Ala Pro Ala Phe Tyr Pro Arg Ala
                85                  90                  95

Tyr Ser Asp Pro Asp Pro Ala Lys Asp Pro Arg Ala Glu Lys Lys Glu
            100                 105                 110

Leu Cys Ala Leu Gln Lys Ala Val Glu Leu Glu Lys Thr Glu Ala Asp
        115                 120                 125

Asn Ala Glu Arg Pro Arg Ala Arg Arg Arg Arg Lys Pro Arg Val Leu
    130                 135                 140

Phe Ser Gln Ala Gln Val Tyr Glu Leu Glu Arg Arg Phe Lys Gln Gln
145                 150                 155                 160

Arg Tyr Leu Ser Ala Pro Glu Arg Asp Gln Leu Ala Ser Val Leu Lys
                165                 170                 175

Leu Thr Ser Thr Gln Val Lys Ile Trp Phe Gln Asn Arg Arg Tyr Lys
            180                 185                 190

Cys Lys Arg Gln Arg Gln Asp Gln Thr Leu Glu Leu Val Gly Leu Pro
        195                 200                 205

Pro Pro Pro Pro Pro Pro Ala Arg Arg Ile Ala Val Pro Val Leu Val
    210                 215                 220
```

```
Arg Asp Gly Lys Pro Cys Leu Gly Asp Ser Ala Pro Tyr Ala Pro Ala
225                 230                 235                 240

Tyr Gly Val Gly Leu Asn Pro Tyr Gly Tyr Asn Ala Tyr Pro Ala Tyr
                245                 250                 255

Pro Gly Tyr Gly Gly Ala Ala Cys Ser Pro Gly Tyr Ser Cys Thr Ala
            260                 265                 270

Ala Tyr Pro Ala Gly Pro Ser Pro Ala Gln Pro Ala Thr Ala Ala Ala
        275                 280                 285

Asn Asn Asn Phe Val Asn Phe Gly Val Gly Asp Leu Asn Ala Val Gln
    290                 295                 300

Ser Pro Gly Ile Pro Gln Ser Asn Ser Gly Val Ser Thr Leu His Gly
305                 310                 315                 320

Ile Arg Ala Trp
```

<210> 27
<211> 980
<212> DNA
<213> Homo sapiens

<220>
<221> CDS
<222> (221) .. (727)

<400> 27

```
cagccagtgc aactcagccg gctgcctctt cgacggcttt gactgccagc gtgcggaagg 60

ccagtgcaac cccctgtacg accagtactg caaggaccac ttcagcgacg ggcactgcga 120

ccagggctgc aacagcgcgg agtgcgagtg ggacgggctg gactgtgcgg agcatgtacc 180

cgagaggctg gcggccggca cgctggtggt ggtggtgctg atg ccg ccg gag cag   235
                                          Met Pro Pro Glu Gln
                                           1               5

ctg cgc aac agc tcc ttc cac ttc ctg cgg gag ctc agc cgc gtg ctg   283
Leu Arg Asn Ser Ser Phe His Phe Leu Arg Glu Leu Ser Arg Val Leu
                10                  15                  20

cac acc aac gtg gtc ttc aag cgt gac gca cac ggc cag cag atg atc   331
His Thr Asn Val Val Phe Lys Arg Asp Ala His Gly Gln Gln Met Ile
            25                  30                  35

ttc ccc tac tac ggc cgc gag gag gag ctg cgc aag cac ccc atc aag   379
Phe Pro Tyr Tyr Gly Arg Glu Glu Glu Leu Arg Lys His Pro Ile Lys
        40                  45                  50

cgt gcc gcc gag ggc tgg gcc gca cct gac gcc ctg ctg ggc cag gtg   427
Arg Ala Ala Glu Gly Trp Ala Ala Pro Asp Ala Leu Leu Gly Gln Val
    55                  60                  65

aag gcc tcg ctg ctc cct ggt ggc agc gag ggt ggg cgg cgg cgg agg   475
Lys Ala Ser Leu Leu Pro Gly Gly Ser Glu Gly Gly Arg Arg Arg Arg
 70                  75                  80                  85
```

```
gag ctg gac ccc atg gac gtc cgc ggc tcc atc gtc tac ctg gag att    523
Glu Leu Asp Pro Met Asp Val Arg Gly Ser Ile Val Tyr Leu Glu Ile
                90                  95                  100

gac aac cgg cag tgt gtg cag gcc tcc tcg cag tgc ttc cag agt gcc    571
Asp Asn Arg Gln Cys Val Gln Ala Ser Ser Gln Cys Phe Gln Ser Ala
            105                 110                 115

acc gac gtg gcc gca ttc ctg gga gcg ctc gcc tcg ctg ggc agc ctc    619
Thr Asp Val Ala Ala Phe Leu Gly Ala Leu Ala Ser Leu Gly Ser Leu
        120                 125                 130

aac atc ccc tac aag atc gag gcc gtg cag act cag tgc gag tgc agc    667
Asn Ile Pro Tyr Lys Ile Glu Ala Val Gln Thr Gln Cys Glu Cys Ser
    135                 140                 145

gat ttg aag ttg act aat cct cct tcc tta aag gag aaa aaa gta aaa    715
Asp Leu Lys Leu Thr Asn Pro Pro Ser Leu Lys Glu Lys Lys Val Lys
150                 155                 160                 165

gcc gtc tcc aga tagagtcggc tggtgcagga gagaatttag cgatagtttg        767
Ala Val Ser Arg

caattctgat taatcgcgta gaaaatgacc ttattttgga gggcgggatg gaggagagtg 827

ggtgaggagg cgcccggacg cggagccagt ccgccgcccc ccggccacca gcctgctgcg 887

tagccgctgc ctgatgtccg ggcacctgcc cctggccccc gtgcccgcag gtgagaccgt 947

ggagccgccc ccgccggcgc agctgcactt cat                                980
```

<210> 28
<211> 169
<212> PRT
<213> Homo sapiens

<400> 28

```
Met Pro Pro Glu Gln Leu Arg Asn Ser Ser Phe His Phe Leu Arg Glu
 1               5                  10                  15

Leu Ser Arg Val Leu His Thr Asn Val Val Phe Lys Arg Asp Ala His
            20                  25                  30

Gly Gln Gln Met Ile Phe Pro Tyr Tyr Gly Arg Glu Glu Glu Leu Arg
        35                  40                  45

Lys His Pro Ile Lys Arg Ala Ala Glu Gly Trp Ala Ala Pro Asp Ala
    50                  55                  60

Leu Leu Gly Gln Val Lys Ala Ser Leu Leu Pro Gly Gly Ser Glu Gly
65                  70                  75                  80

Gly Arg Arg Arg Arg Glu Leu Asp Pro Met Asp Val Arg Gly Ser Ile
                85                  90                  95

Val Tyr Leu Glu Ile Asp Asn Arg Gln Cys Val Gln Ala Ser Ser Gln
            100                 105                 110

Cys Phe Gln Ser Ala Thr Asp Val Ala Ala Phe Leu Gly Ala Leu Ala
        115                 120                 125

Ser Leu Gly Ser Leu Asn Ile Pro Tyr Lys Ile Glu Ala Val Gln Thr
        130                 135                 140

Gln Cys Glu Cys Ser Asp Leu Lys Leu Thr Asn Pro Pro Ser Leu Lys
145                 150                 155                 160

Glu Lys Lys Val Lys Ala Val Ser Arg
                165
```

<210> 29
<211> 2826
<212> DNA
<213> Homo sapiens

<220>
<221> CDS
<222> (184) .. (618)

<400> 29

```
gagagggccc ggactagggg cggcgggcac cgcaggagct ccgcgcggct gcagcgcggg 60

cgggagcggg gacgcgatgt cgccgccgcc gcctccttgc gggccggggc tgcgcctccg 120

gggctgagcc gccgccagag ccgacagccg agcagccgct gggcgctccc gcggcgcagg 180

agg atg ggc tgc ggc ggg agc cgg gcg gat gcc atc gag ccc cgc tac    228
    Met Gly Cys Gly Gly Ser Arg Ala Asp Ala Ile Glu Pro Arg Tyr
     1               5                  10                  15

tac gag agc tgg acc cgg gag aca gaa tcc acc tgg ctc acc tac acc    276
Tyr Glu Ser Trp Thr Arg Glu Thr Glu Ser Thr Trp Leu Thr Tyr Thr
                20                  25                  30

gac tcg gac gcg ccg ccc agc gcc gcc gcc ccg gac agc ggc ccc gaa    324
Asp Ser Asp Ala Pro Pro Ser Ala Ala Ala Pro Asp Ser Gly Pro Glu
            35                  40                  45

gcg ggc ggc ctg cac tcg ggc atg ctg gaa gat gga ctg ccc tcc aat    372
Ala Gly Gly Leu His Ser Gly Met Leu Glu Asp Gly Leu Pro Ser Asn
        50                  55                  60

ggt gtg ccc cga tct aca gcc cca ggt gga ata ccc aac cca gag aag    420
Gly Val Pro Arg Ser Thr Ala Pro Gly Gly Ile Pro Asn Pro Glu Lys
    65                  70                  75

aag acg aac tgt gag acc cag tgc cca aat ccc cag agc ctc agc tca    468
Lys Thr Asn Cys Glu Thr Gln Cys Pro Asn Pro Gln Ser Leu Ser Ser
80                  85                  90                  95

ggc cct ctg acc cag aaa cag aat ggc ctt cag acc aca gag gct aaa    516
Gly Pro Leu Thr Gln Lys Gln Asn Gly Leu Gln Thr Thr Glu Ala Lys
                100                 105                 110

aga gat gct aag aga atg cct gca aaa gaa gtc acc att aat gta aca    564
Arg Asp Ala Lys Arg Met Pro Ala Lys Glu Val Thr Ile Asn Val Thr
            115                 120                 125

gat agc atc caa cag atg gac aga agt cga aga atc aca aag aac tgt    612
Asp Ser Ile Gln Gln Met Asp Arg Ser Arg Arg Ile Thr Lys Asn Cys
        130                 135                 140

gtc aac tagcagagag tccaagcaga agggcagatg gacttcttca gtgtccttca     668
```

```
Val Asn
    145

cggcactgga tcccatcaaa gaaccttgaa gaagtggctg cccccttgctg gacctgaatt 728

ctactgagtc cctggcaaga ccgtcttacc tggcagcaaa ctgctgcctg atttgttggg 788

accttctgag ccttctactt atcatgtaaa tgtattggca cagtgcttac atatgttaat 848

aaactgcaaa tgtgcagttc agtttgtctc tttgcaactc ctgtaatacg gtctggtgta 908

aaagtagtga gttaaagcta caggtcagtt tatgaaacag aaaagtagga atgcattttc 968

tgggtgaaag agtcacacct tagtgctata actctcctgc ccatgatagt gtattctgtt 1028

tcaggcaagc ttattctttc cttccttcat tttaaatatt gtcattacaa atcttaccag 1088

gttcacttaa aagctggctt tcatccaact ctaaacccac atattgaaaa aatcaaggta 1148

caggaaaact ccttgttatc cttgtttcct tagcttggta tgagacagat cggatccagt 1208

ttcccatgca ccaacccact gcccatggca tgtctttggg aggtgtctgt gaagcagtca 1268

tacctgctcc tcatctgcct ggaaagtcct cctattccag tgtccatgtt ggcctccagt 1328

ccttaatgtc accatgcttg tggccaatgc atccaaataa ggataccccct cagggctcag 1388

ctagacattg caattttgca tagctttcca gttccctttg cttgtcttct tgactgtctt 1448

ccctctctac cggggtcact tgcaattgtt aatcaaagat tgaacactgc gtaggagagg 1508

gagatgatcc agagacatgt ggcagcaggc atggcttccc cttggcctct ctgtacactg 1568

ccccaggact gtcattttgg catctgcaaa ggaatcactt tagaaagcca gcacctggtt 1628

gatgtgtatt catactgaca ttagattgat gtgcactgca ttagaaatga ggtagctgac 1688

acagaaaaag gatgttttga taggaataat tttctagtat gtcttgaaac atgttcatct 1748

ggaagtattt tcctccaaag taatgtagca tgatttttca aggattgtta acatgcctgg 1808

gattgggaaa gataggacta aagttgtgcc aaactatatc aataaattcc atgtttagca 1868

gaaataggca gcctattggt gttatgttta tgtaacatag tccagagaac tgacatgcag 1928

gtcaaaagtc agatacgcaa cctccttatc tgctaactct gttattcttc aaacacaagt 1988

gggtagtgtc atttttcctt ccttccttcc attggcagat tgtatattta ttcacaaaac 2048

attaaatgtc catcctgtgc caggtactat gcagatgttg agggatttgg ggtctggtta 2108

gtcgtgacta tctatcctga atctaacagt gacttcataa ctaggagact gaattagacc 2168

cttaaggtat agtgtgtgtt gcaaatcact ctgcaatgga aacttttata ttcagggtag 2228

gtttgtgtct taaactaggt gttctaatca atgtacaaga ctttaccata cacgcaacta 2288

tagtttttct aaaccttcat cattttgtga ttctttgaga aagggctttt aggaacttta 2348

tgttctaaaa aatgttttta acaataataa gataaaagaa aaacctgtga ttcatatgtc 2408

cccactggca ttactcagca ggagcccccca gctgccaaag gttggcagtg atcctgcaag 2468
```

```
ttcaagggct ctttctccct ggggatgtgc tttgtggctt ctctttacag ctttgtttct 2528
gcatcagttc actgctgcat gttgtttgga atttatcacc ttaagaaagt gtctctgttt 2588
tatatagaaa cactttctca cttacagggg agaaggaaat gcagggcaca tgatctggcc 2648
ctccccagaa caatctggat ttcacggaga cagcaaccag aagttaaacc atgtgactaa 2708
aaatgcatct ggctactttt tcatgtatgt atgagacaga aactaatcct tactatccta 2768
ttaggatacc acttttcatt gcaaagtttg tgtcaataaa gtcattaatt ttaaacat   2826
```

<210> 30
<211> 145
<212> PRT
<213> Homo sapiens

<400> 30.

```
Met Gly Cys Gly Gly Ser Arg Ala Asp Ala Ile Glu Pro Arg Tyr Tyr
1               5                   10                  15

Glu Ser Trp Thr Arg Glu Thr Glu Ser Thr Trp Leu Thr Tyr Thr Asp
            20                  25                  30

Ser Asp Ala Pro Pro Ser Ala Ala Ala Pro Asp Ser Gly Pro Glu Ala
        35                  40                  45

Gly Gly Leu His Ser Gly Met Leu Glu Asp Gly Leu Pro Ser Asn Gly
    50                  55                  60

Val Pro Arg Ser Thr Ala Pro Gly Gly Ile Pro Asn Pro Glu Lys Lys
65                  70                  75                  80

Thr Asn Cys Glu Thr Gln Cys Pro Asn Pro Gln Ser Leu Ser Ser Gly
                85                  90                  95

Pro Leu Thr Gln Lys Gln Asn Gly Leu Gln Thr Thr Glu Ala Lys Arg
            100                 105                 110

Asp Ala Lys Arg Met Pro Ala Lys Glu Val Thr Ile Asn Val Thr Asp
        115                 120                 125

Ser Ile Gln Gln Met Asp Arg Ser Arg Arg Ile Thr Lys Asn Cys Val
    130                 135                 140

Asn
145
```

<210> 31
<211> 1027
<212> DNA
<213> Influenza virus

<400> 31

```
agcgaaagca ggtagatatt gaaagatgag tcttctaacc gaggtcgaaa cgtacgttct 60
ctctatcatc ccgtcaggcc ccctcaaagc cgagatcgca cagagacttg aagatgtctt 120
tgcagggaag aacaccgatc ttgaggttct catggaatgg ctaaagacaa gaccaatcct 180
gtcacctctg actaagggga ttttaggatt tgtgttcacg ctcaccgtgc ccagtgagcg 240
aggactgcag cgtagacgct ttgtccaaaa tgcccttaat gggaacgggg atccaaataa 300
catggacaaa gcagttaaac tgtataggaa gctcaagagg gagataacat tccatggggc 360

caaagaaatc tcactcagtt attctgctgg tgcacttgcc agttgtatgg gcctcatata 420
caacaggatg gggctgtga ccactgaagt ggcatttggc ctggtatgtg caacctgtga 480
acagattgct gactcccagc atcggtctca taggcaaatg gtgacaacaa ccaacccact 540
aatcagacat gagaacagaa tggttttagc cagcactaca gctaaggcta tggagcaaat 600
ggctggatcg agtgagcaag cagcagaggc catggaggtt gctagtcagg ctaggcaaat 660
ggtgcaagcg atgagaacca ttgggactca tcctagctcc agtgctggtc tgaaaaatga 720
tcttcttgaa aatttgcagg cctatcagaa acgaatgggg gtgcagatgc aacggttcaa 780
gtgatcctct cgctattgcc gcaaatatca ttgggatctt gcacttgata ttgtggattc 840
ttgatcgtct ttttttcaaa tgcatttacc gtcgctttaa atacggactg aaaggagggc 900
cttctacgga aggagtgcca aagtctatga gggaagaata tcgaaaggaa cagcagagtg 960
ctgtggatgc tgacgatggt cattttgtca gcatagagct ggagtaaaaa actaccttgt 1020
ttctact                                               1027
```

<210> 32
<211> 9
<212> PRT
<213> Influenza virus

<400> 32

```
Gly Ile Leu Gly Phe Val Phe Thr Leu
 1               5
```

<210> 33
<211> 10
<212> PRT
<213> Influenza virus

<400> 33

```
Tyr Met Leu Asp Leu Gln Pro Glu Thr Thr
 1               5                  10
```

<210> 34
<211> 9
<212> PRT
<213> Homo sapiens

<400> 34

```
Ala Ala Gly Ile Gly Ile Leu Thr Val
 1               5
```